(19) Europäisches Patentamt
European Patent Office
Office européen des brevets

(11) Numéro de publication : **0 050 534**
**B1**

(12) # FASCICULE DE BREVET EUROPÉEN

(45) Date de publication du fascicule du brevet : **03.04.85**

(21) Numéro de dépôt : **81401360.3**

(22) Date de dépôt : **28.08.81**

(51) Int. Cl.[4] : **C 07 C121/75**, C 07 D233/74, C 07 D213/64, C 07 C 69/65, A 01 N 53/00, A 61 K 31/22, A 23 K 1/16

(54) **Esters d'acides cyclopropane carboxyliques apparentés à l'acide pyréthrique, leur procédé de préparation et leur application à la lutte contre les parasites.**

(30) Priorité : **01.10.80 FR 8021017**

(43) Date de publication de la demande : **28.04.82 Bulletin 82/17**

(45) Mention de la délivrance du brevet : **03.04.85 Bulletin 85/14**

(84) Etats contractants désignés : **AT BE CH DE FR GB IT LI LU NL SE**

(56) Documents cités :
**FR-A- 2 185 612**
**JOURNAL OF THE CHEMICAL SOCIETY, Perkin Transactions I, 1974, M. ELLIOTT et al.: "The pyrethrins and related compounds. Part XVIII Insecticidal 2,2-Dimethylcyclopropanecarboxylates with new unsaturated 3-substituents", pages 2470-2474**
**CHEMICAL ABSTRACTS, vol. 85, no. 17, 25 octobre 1976, page 150, résumé no. 117925b, COLUMBUS OHIO (US)**

(73) Titulaire : **ROUSSEL-UCLAF**
**35, boulevard des Invalides**
**F-75007 Paris (FR)**

(72) Inventeur : **Martel, Jacques**
**15, rue Douvillez**
**F-93140 Bondy (FR)**
Inventeur : **Tessier, Jean**
**30, route Jean Moulin**
**F-94300 Vincennes (FR)**
Inventeur : **Teche, André**
**15, rue Godot de Mauroy**
**F-75009 Paris (FR)**

(74) Mandataire : **Burlot, Pierre et al**
**ROUSSEL-UCLAF Boîte Postale No. 9 111, route de Noisy**
**F-93230 Romainville (FR)**

**EP 0 050 534 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

L'invention concerne de nouveaux esters d'acides cyclopropane carboxyliques apparentés à l'acide pyréthrique, leur procédé de préparation et leur application à la lutte contre les parasites.

On connaissait déjà comme pesticides, certains esters de l'acide 2,2-diméthyl cyclopropane carboxylique portant en 3 une chaîne :

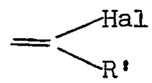

dans laquelle Hal représentait un atome de chlore ou de brome et R' un radical alcoxy carbonyle (voir à ce sujet, Journal of the Chemical Society Perkin I, 1974, p. 2471 et suivantes, Chemical Abstracts Vol. 85 n° 17, 25 octobre 76, page 150 résumé 117925b, FR-A-2185612. On vient de découvrir que certains esters de l'acide 1R cis 2,2-diméthyl cyclopropane carboxylique portant en 3 une chaîne :

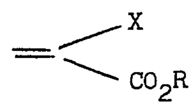

dans laquelle la géométrie de la double liaison et E, avaient des propriétés pesticides particulièrement intéressantes.

L'invention a pour objet sous toutes les formes isomères possibles, ou sous forme de mélanges d'isomères, les composés de formule I :

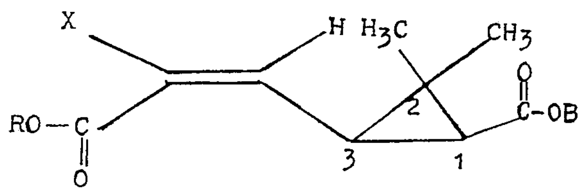 (I)

dans laquelle la structure de la copule acide cyclopropanique est 1R cis et la géométrie de la double liaison est E et dans laquelle R représente ou bien un radical alcoyle linéaire, ramifié ou cyclique, saturé ou insaturé, renfermant de 1 à 8 atomes de carbone, éventuellement substitué par un ou plusieurs groupements fonctionnels identiques ou différents, ou bien un groupement aryle renfermant de 6 à 14 atomes de carbone, éventuellement substitué par un ou plusieurs groupements fonctionnels identiques ou différents, ou bien un radical hétérocyclique éventuellement substitué par un ou plusieurs groupements fonctionnels identiques ou différents, X représente un atome d'halogène et B représente

soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxy et les atomes d'halogène,

soit un groupement

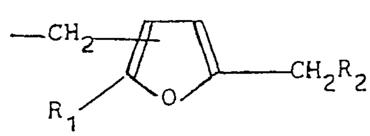

dans lequel le substituant $R_1$ représente un atome d'hydrogène ou un radical méthyle et le substituant $R_2$ un aryle monocyclique ou un groupement $-CH_2-C\equiv CH$ et notamment un groupement 5-benzyl 3-furyl méthyle,

soit un groupement

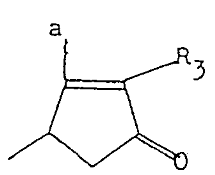

dans lequel a représente un atome d'hydrogène ou un radical méthyle et $R_3$ représente un radical organique aliphatique comportant de 2 à 6 atomes de carbones et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$,

soit un groupement

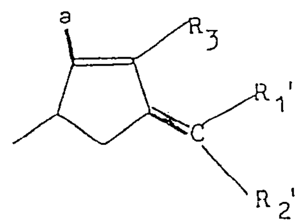

dans lequel a représente un atome d'hydrogène ou un radical méthyle, $R_3$ conserve la même signification que précédemment, $R_1'$ et $R_2'$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

soit un groupement

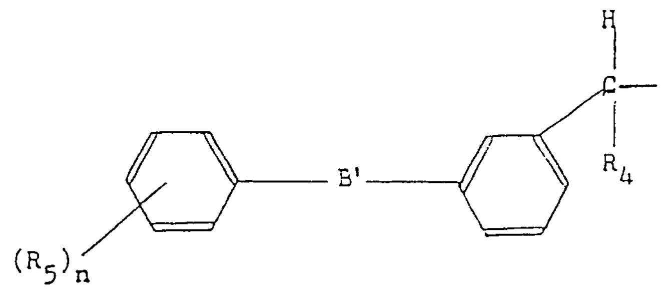

dans lequel B′ représente un atome d'oxygène ou de soufre, un groupement

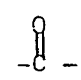

ou $-CH_2-$ ou un groupement sulfoxyde ou un groupement sulfone et $R_4$ représente un atome d'hydrogène, un radical $-C{\equiv}N$, un radical méthyle, un radical $-CONH_2$, un radical $-CSNH_2$ ou un radical $-C{\equiv}CH$, $R_5$ représente un atome d'halogène ou un radical méthyle et n représente un nombre égal à 0, 1 ou 2, et notamment le groupement 3-phénoxy benzyle, α-cyano 3-phénoxy benzyle, α-éthynyl 3-phénoxy benzyle, 3-benzoyl benzyle, 1-(3-phénoxy phényl) éthyle ou α-thioamido 3-phénoxy benzyle,

soit un groupement

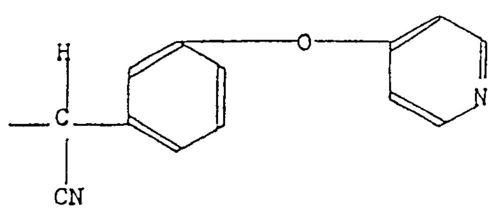

soit un groupement

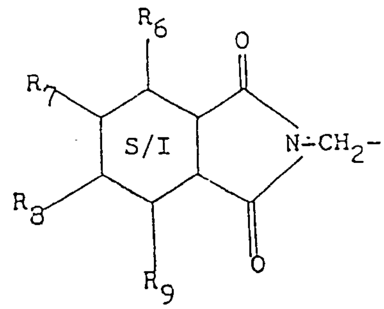

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

soit un groupement

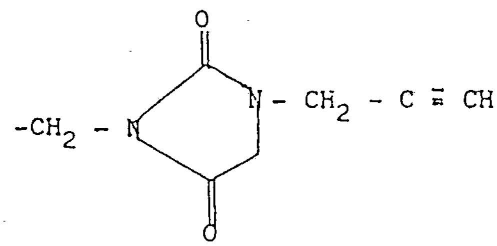

soit un groupement

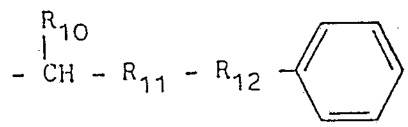

dans lequel $R_{10}$ représente un atome d'hydrogène ou un radical CN, $R_{12}$ représente un radical $-CH_2-$ ou un atome d'oxygène, $R_{11}$ représente un radical thiazolyle ou thiadiazolyle dont la liaison avec

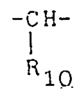

peut se trouver à l'une quelconque des positions disponibles, $R_{12}$ étant lié à $R_{11}$ par l'atome de carbone compris entre l'atome de soufre et un atome d'azote,

soit un groupement

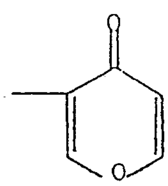

soit un groupement

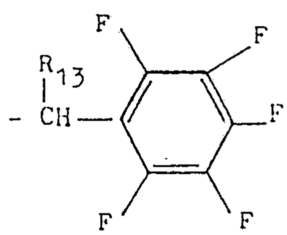

dans lequel $R_{13}$ représente un atome d'hydrogène ou un radical CN,

soit un groupement

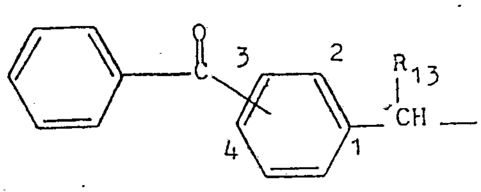

dans lequel $R_{13}$ est défini comme ci-dessus, et le radical benzoyle est en position 3 ou 4,

soit un groupement

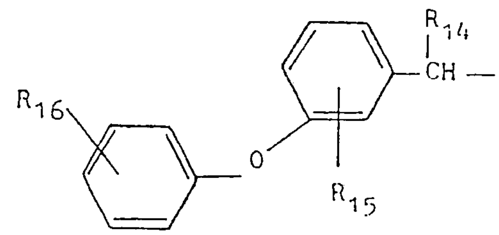

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

soit un groupement

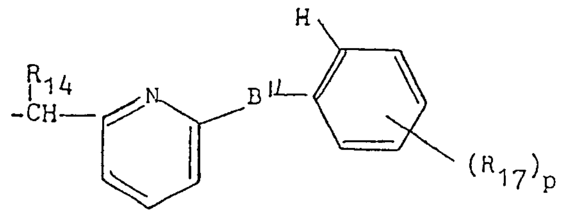

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoyl sulfonyl renfermant de 1 à 4 atomes de carbone, trifluorométhyl, 3,4-méthylène dioxy, chloro, fluoro ou bromo, p représente un nombre égal à 0, 1 ou 2 et B″ représente un atome d'oxygène ou un atome de soufre.

Les composés de formule (I) peuvent exister sous de nombreuses formes stéréoisomères : ils possèdent, en effet, deux carbones asymétriques en 1 et en 3 du cyclopropane ; ils peuvent, de plus, présenter un ou plusieurs centres d'asymétrie dans la partie B comme dans la partie R.

Lorsque R représente un radical alcoyle saturé, linéaire ou ramifié, il s'agit de préférence d'un radical méthyle, éthyle, n-propyle, isopropyle, n-butyle, sec-butyle, isobutyle, n-pentyle, n-hexyle, tert-butyle, tert-pentyle ou néopentyle.

Lorsque R représente un radical cyclique, il s'agit de préférence d'un radical cyclopropyle, cyclobutyle, cyclopentyle ou cyclohexyle, d'un radical alcoyle, linéaire ou ramifié, portant un radical cyclique ou d'un radical cyclopropyle, cyclobutyle, cyclopentyle ou cyclohexyle substitué par un ou plusieurs radicaux alcoyles dont la liaison avec le groupement —COO— est située sur l'un quelconque de ses sommets, par exemple, le radical 1-méthylcyclobutyle, 1-méthyl cyclopentyle, 1-méthylcyclohexyle ou 2,2,3,3-tétraméthylcyclopropyle.

Lorsque R représente un radical alcoyle insaturé, il s'agit d'un radical éthylénique, comme, par exemple, d'un radical vinyle ou 1,1-diméthylallyle ou d'un radical acétylénique, comme, par exemple, le radical éthynyle ou propynyle.

Lorsque R représente un radical alcoyle substitué par un ou plusieurs groupements fonctionnels, on entend de préférence par alcoyle un radical renfermant de 1 à 8 atomes de carbone comme, par exemple, le radical méthyle, éthyle, propyle, isopropyle, n-butyle, isobutyle ou tertbutyle.

Lorsque R représente un radical alcoyle substitué par un ou plusieurs groupements fonctionnels, on entend de préférence par groupement fonctionnel, un atome d'halogène, un groupement OH ou SH, un groupement OR′ ou SR′ dans lesquels R′ représente un radical alcoyle renfermant de 1 à 8 atomes de carbone,

un groupement

$-NO_2$, 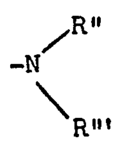

dans lequel R″ et R‴, identiques ou différents, représentent un atome d'hydrogène, ou un radical alcoyle renfermant de 1 à 8 atomes de carbone, un groupement —C≡N, $SO_3H$ ou $PO_4H_2$ ou un groupement $—COalc_1$, $SO_2alc_2$, ou $SO_3alc_3$ dans lesquels $alc_1$, $alc_2$ et $alc_3$ représentent des radicaux alcoyles renfermant de 1 à 18 atomes de carbone.

R peut représenter également un radical alcoyle substitué par un radical aryle comme, par exemple, le radical benzyle ou le radical phénéthyle, lui-même éventuellement substitué par un ou plusieurs groupements —OH, —Oalc ou alc renfermant de 1 à 8 atomes de carbone, par un ou plusieurs halogènes ou groupements $—CF_3$, $—OCF_3$, $—SCF_3$, ou par un groupement (G) :

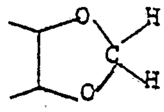

(G)

R peut représenter également un radical alcoyle substitué sur deux carbones adjacents par un groupement ($G_1$)

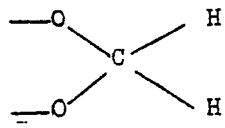

($G_1$)

substitué par un groupement.

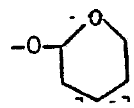

Lorsque R représente un radical alcoyle substitué par un ou plusieurs groupements fonctionnels, on peut citer comme valeurs préférées de R les radicaux :

$-(CH_2)_n-C\ Hal_3$ dans lequel n est un entier de 1 à 8 et Hal un atome d'halogène, par exemple le radical $-CH_2-CCl_3$, $-CH_2CF_3$,

$-CH_2-CH_2-CCl_3$ ou $-CH_2-CH_2-CF_3$,

$-(CH_2)_{n_1}-CH\ Hal_2$ dans lequel Hal est défini comme ci-dessus et $n_1$ est un nombre de 0 à 8, par exemple le radical $-CH_2-CHCl_2$,

$-CH_2-CHF_2$ ou $-CHF_2$,

$-(CH_2)_n-CH_2\ Hal$ dans lequel n et Hal sont définis comme ci-dessus, par exemple le radical $-CH_2-CH_2Cl$ ou $-CH_2-CH_2F$,

$-C-(CHal_3)_3$ dans lequel Hal est défini comme ci-dessus, par exemple le radical $-C-(CF_3)_3$ ou

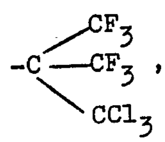 ,

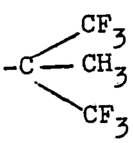 , 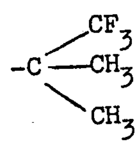 ou 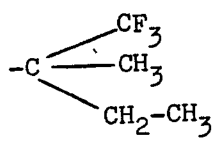

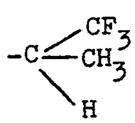 ou 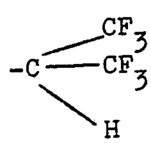

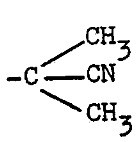 ou 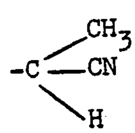

ou $-(CH_2)_n-CN$, dans lequel n est défini comme précédemment,

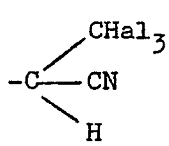 ,

dans lequel Hal est défini comme précédemment, par exemple le radical

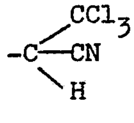 ,

—$(CH_2)_n$—OR′, dans lequel n est défini comme précédemment et R′ représente un atome d'hydrogène ou un radical alcoyle linéaire ou ramifié, comportant de 1 à 8 atomes de carbone, par exemple le radical —$CH_2$—$OCH_3$, —$CH_2$—$CH_2$—O—$CH_3$, —$CH_2$—$CH_2$—O—$CH_2$—$CH_3$ ou —$CH_2$—$CH_2$—OH,

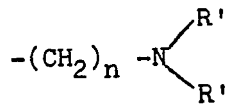

dans lequel n et R′ sont définis comme précédemment et les deux radicaux R′ peuvent être différents entre eux, par exemple le radical

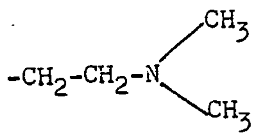

ou

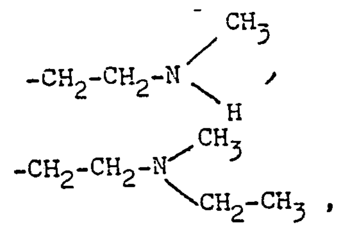

,

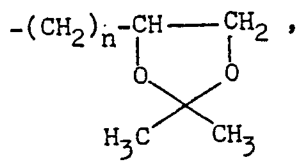

,

dans lequel n est défini comme précédemment, par exemple le radical

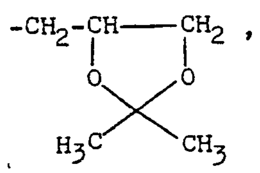

,

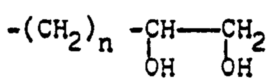

dans lequel n est défini comme précédemment, par exemple le radical

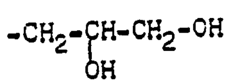

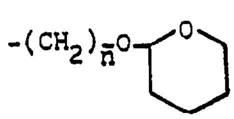

dans lequel n est défini comme précédemment, par exemple le radical

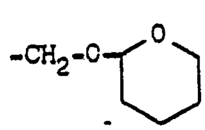

ou

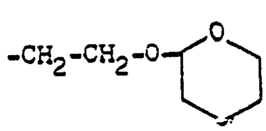

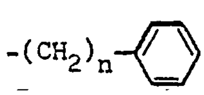

dans lequel n est défini comme précédemment, par exemple le radical benzyle ou phénéthyle,

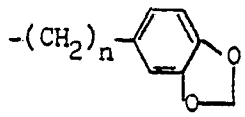

dans lequel n est défini comme précédemment, par exemple le radical

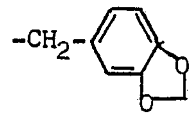

Lorsque R représente un radical aryle éventuellement substitué, il s'agit de préférence du radical phényle ou du radical phényle substitué par un ou plusieurs groupements OH, Oalc ou alc, renfermant de 1 à 8 atomes de carbone, ou par un halogène ou un groupement $—CF_3$, $—OCF_3$ ou $SCF_3$.

Lorsque R représente un radical hétérocyclique, il s'agit de préférence du radical pyridinyle, furanyle, thiophényle, oxazolyle ou thiazolyle.

Par halogène, on entend de préférence un atome de fluor, de chlore ou de brome.

Lorsque B représente un radical benzyle substitué par un ou plusieurs radicaux alcoyle, il s'agit de préférence du radical méthyle ou éthyle.

Lorsque B représente un radical benzyle substitué par un ou plusieurs radicaux alcényle, il s'agit de préférence de radicaux vinyle, allyle, 2-méthylallyle ou isobuténylе.

Lorsque B représente un radical benzyle substitué par un radical alcadiènyle, il s'agit de préférence d'un radical benzyle substitué par un radical alcadiènyle renfermant 4, 5 ou 6 atomes de carbone.

Lorsque B représente un radical benzyle substitué par un ou plusieurs alcényloxy, il s'agit de préférence de radicaux vinyloxy, allyloxy, 2-méthylallyloxy ou isobuténуloxy.

Lorsque B représente un radical benzyle substitué par un ou plusieurs atomes d'halogène, il s'agit de préférence d'atomes de chlore, de brome ou de fluor.

Lorsque B représente le radical :

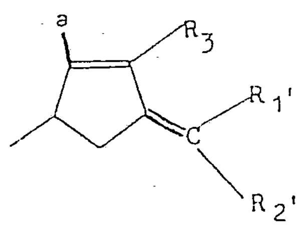

$R_3$ représente de préférence le radical $—CH_2—CH=CH_2$, $—CH_2—CH=CH—CH_3$, $—CH_2—CH=CH—CH=CH_2$ ou $—CH_2—CH=CH—CH_2—CH_3$.

Lorsque $R_1'$ et (ou) $R_2'$ représentent un atome d'halogène il s'agit de préférence d'un atome de chlore, de brome ou de fluor.

Lorsque $R_1'$ et (ou) $R_2'$ représentent un radical alcoyle, il s'agit de préférence du radical méthyle, éthyle, n-propyle ou n-butyle.

Lorsque $R_1'$ et (ou) $R_2'$ représentent un radical aryle, il s'agit de préférence du radical phényle.

Lorsque $R_1'$ et (ou) $R_2'$ représentent un radical alcoyloxy carbonyl, il s'agit de préférence du radical méthoxy carbonyl ou éthoxycarbonyl.

Lorsque dans le radical :

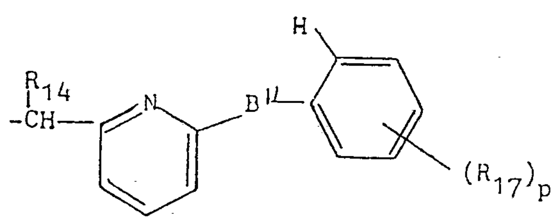

$R_{17}$ représente un radical alcoyle, alcoxy, alcoylthio ou alcoylsulfonyl, il s'agit de préférence du radical méthyle, éthyle, méthoxy, éthoxy, méthylthio, éthylthio, méthyl sulfonyl ou éthyl sulfonyl.

Parmi les composés de l'invention, on peut citer les composés de formule I pour lesquels X représente un atome de fluor ; on peut citer également les composés de formule I pour lesquels R représente un radical alcoyle, linéaire ramifié ou cyclique, renfermant de 1 à 8 atomes de carbone, et

notamment un radical éthyle, un radical tert-butyle ou encore un radical cyclopropyle ou cyclopropylméthyle. On peut citer également les composés de formule I pour lesquels R représente un radical alcoyle, linéaire ou ramifié, renfermant de 1 à 8 atomes de carbone, substitué par un ou plusieurs atomes d'halogène, par exemple, ceux pour lesquels R représente un radical alcoyle, linéaire renfermant de 1 à 8 atomes de carbone, substitué par un ou plusieurs atomes de fluor. On peut également citer les composés de formule I pour lesquels R représente un radical $(CH_2)_mO(CH_2)_n—CH_3$ dans lequel m représente un nombre entier pouvant varier de 1 à 8 et n représente un nombre entier pouvant varier de 0 à 8 et notamment, le radical $—CH_2O—CH_3$.

Parmi les composés de l'invention, on peut citer les composés répondant à la formule $I_A$ :

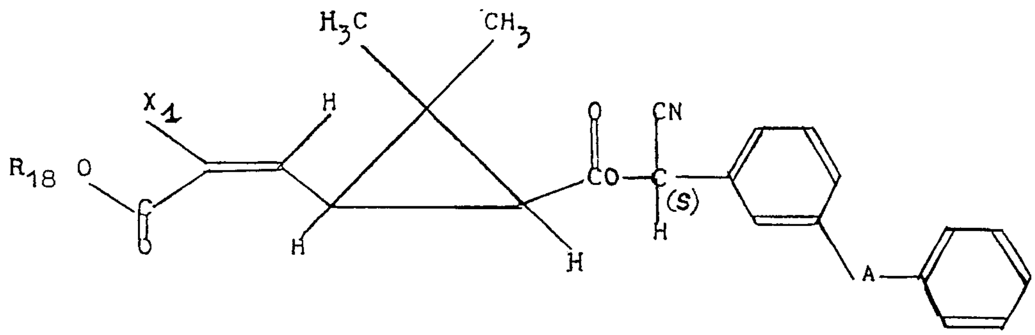

$(I_A)$

dans laquelle la structure de la copule acide cyclopropanique est 1R cis et la géométrie de la double liaison est E et dans laquelle A représente un atome d'oxygène, un groupement méthylène, un groupement carbonyle, un atome de soufre, un groupement sulfoxyde ou un groupement sulfone, $R_{18}$ représente un radical alcoyle linéaire ou ramifié renfermant de 1 à 8 atomes de carbone et $X_1$ représente un atome de fluor, de chlore ou de brome.

Dans les composés de formule $(I_A)$, $R_{18}$ peut représenter un radical alcoyle, linéaire ou ramifié, notamment un radical méthyle, éthyle, propyle, butyle, pentyle, hexyle, heptyle ou octyle, ces derniers radicaux pouvant être linéaires ou ramifiés.

L'invention a notamment pour objet les composés de formule $I_A$ pour lesquels A représente un atome d'oxygène.

Parmi ces composés préférés, on peut citer tout spécialement le (1R,cis)2,2-diméthyl 3(E)-2-fluoro 2-(éthoxy carbonyl) éthényl/cyclopropane-1-carboxylate de (S) α-cyano 3-phénoxy benzyle.

Parmi les composés préférés de l'invention, on peut citer également les composés de formule I pour lesquels B représente un radical :

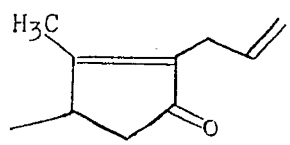

ceux pour lesquels B représente un radical :

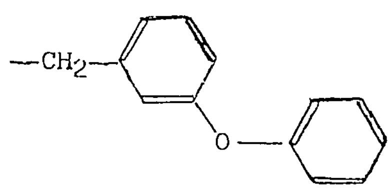

ceux pour lesquels B représente un radical :

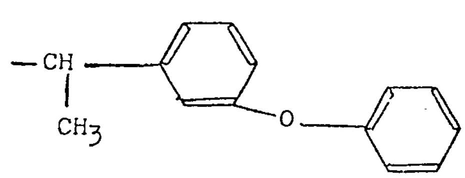

ceux pour lesquels B représente un radical :

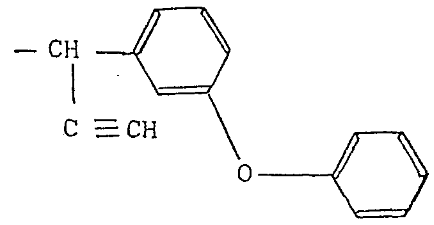

ainsi que ceux pour lesquels B représente un radical :

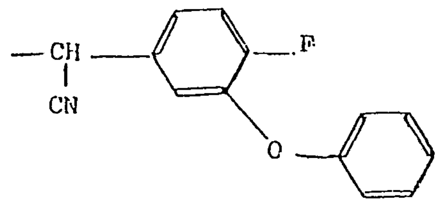

On peut encore citer comme composés préférés de l'invention, les composés pour lesquels B représente un radical [3-(propyn-2-yl)2,5-dioxo imidazolidinyl] méthyle ou encore un radical α-cyano 6-phénoxy 2-pyridyl méthyle.

L'invention a tout particulièrement pour objet les composés dont la préparation est donnée plus loin dans la partie expérimentale et notamment, parmi ceux-ci, les composés des exemples 1, 11 et 21.

Les composés de formule (I) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a donc pour objet l'application des composés de formule (I) à la lutte contre les parasites des végétaux et les parasites des locaux.

L'invention a notamment pour objet l'application des composés de formule $I_A$ à la lutte contre les parasites des végétaux et les parasites des locaux, les parasites des animaux à sang chaud.

Les produits de formule (I) peuvent donc être utilisés notamment pour lutter contre les insectes dans le domaine agricole, pour lutter, par exemple, contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les composés des exemples 1, 11 et 21 sont des produits tout à fait remarquables comme le montrent les résultats des tests ci-après. Ils présentent un excellent pouvoir létal et un très bon pouvoir de knock down.

Les produits de formule (I) sont de plus photostables et ne sont pas toxiques pour les mammifères.

L'ensemble de ces propriétés fait des produits de formule (I) des produits qui correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule I définis ci-dessus et notamment les produits de formule $I_A$.

L'invention a notamment pour objet les compositions insecticides renfermant comme principe actif au moins l'un des produits définis ci-dessus.

Parmi les compositions notamment insecticides préférées de l'invention, on peut citer tout spécialement les compositions renfermant le composé de l'exemple 1, le composé de l'exemple 11 et le composé de l'exemple 21.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces

compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensioactif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les compositions insecticides selon l'invention, comme les compositions acaricides et nématicides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrage foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo [2,2-1] 5-heptène-2,3-dicarboximide, ou le pipéronyl-bis-2-(2'-n-butoxy éthoxy) éthylacétal (ou tropital).

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des antioxydants.

L'invention a donc ainsi pour objet les compositions alimentaires définies ci-dessus.

Les composés de formule (I) présentent une excellente tolérance générale, et l'invention a donc également pour objet les produits de formule (I), à titre de médicaments, pour lutter notamment contre les affections créées par les tiques et les gales.

Les médicaments de l'invention peuvent être utilisés tant en médecine humaine qu'en médecine vétérinaire.

Les médicaments de l'invention sont notamment utilisés en médecine humaine pour lutter contre les poux à titre préventif ou curatif et pour lutter contre la gale. Ils peuvent également être utilisés comme anthelmintiques.

Les médicaments de l'invention peuvent être administrés par voie externe, par vaporisation, par shampooing, par bain ou badigeonnage.

Les médicaments de l'invention à usage vétérinaire peuvent être également administrés par badigeonnage de l'épine dorsale selon la méthode dite méthode « pour-on ». Ils peuvent être également administrés par voie digestive ou parentérale.

L'invention a donc pour objet les compositions pharmaceutiques renfermant comme principe actif au moins un des médicaments définis précédemment.

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitue par les esters d'alléthrolones, d'alcool 3,4,5,6-tétrahydrophtalimido méthylique, d'alcool 5-benzyl 3-furyl méthylique, d'alcools 3-phénoxy benzylique et d'alcools α-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl)cyclopropane-1-carboxyliques, dans lesquels « halo » représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les associations selon l'invention présentent notamment l'intérêt soit de permettre de combattre, par la polyvalence de leur action, une gamme de parasites, plus étendue, soit de manifester, dans certains cas, un effet de synergie.

L'invention a également pour objet un procédé de préparation des composés de formule I, caractérisé en ce que l'on fait réagir un acide de formule (II) :

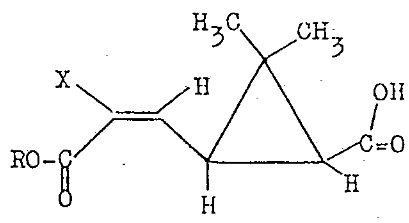

(II)

dans laquelle la structure de la copule cyclopropanique est 1R cis et la géométrie de la double liaison est E et dans laquelle X et R conservent la même signification que précédemment, ou un dérivé fonctionnel de cet acide avec un alcool de formule (III) :

B—OH (III)

B conservant la même signification que précédemment ou un dérivé fonctionnel de cet alcool, pour obtenir le composé de formule (I) correspondant, que l'on soumet, si désiré, à l'action d'un agent de clivage sélectif du groupement $CO_2R$ pour obtenir le composé de formule (IV) :

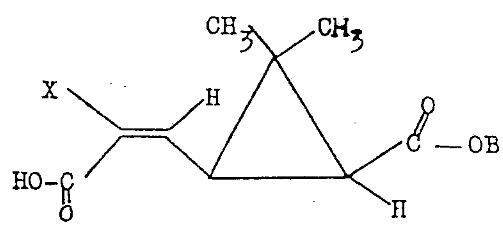

(IV)

dans laquelle la structure de la copule cyclopropanique est 1R cis et la géométrie de la double liaison est E et dans laquelle B conserve la même signification que précédemment, puis soumet, cet acide de formule (IV) ou un dérivé fonctionnel de cet acide, à l'action d'un alcool de formule R—OH dans laquelle R conserve sa signification précédente, pour obtenir le composé de formule (I) correspondant.

L'invention a notamment pour objet un procédé selon ce qui précède, pour préparer les composés de formule $I_A$, telle que définie précédemment, caractérisé en ce que l'on fait réagir un acide de formule ($II_A$) :

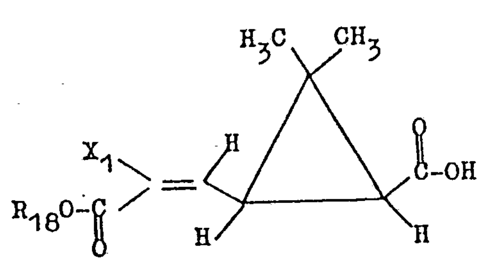

(IIA)

dans laquelle la structure de la copule cyclopropanique est 1R cis et la géométrie de la double liaison est E et dans laquelle $X_1$ et $R_{18}$ conservent la même signification que précédemment, le cycle cyclopropanique substitué pouvant être sous toutes ses formes stéréoisomères ou sous forme de mélange de stéréoisomères ou un dérivé fonctionnel de cet acide ($II_A$) avec un alcool de formule ($III_A$)

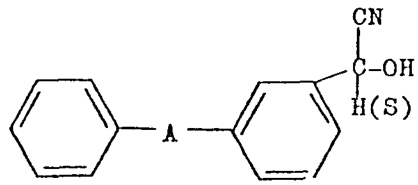

(IIIA)

dans laquelle A conserve la même signification que précédemment ou un dérivé fonctionnel de l'alcool de formule ($III_A$) et sépare pour obtenir le composé de formule $I_A$ correspondant.

L'estérification de l'acide (II) ou ($II_A$) avec l'alcool de formule (III) ou ($III_A$) peut être effectuée en présence d'une base tertiaire telle que la pyridine. Cette estérification peut être effectuée avantageusement en présence d'un mélange de pyridine, de dicyclohexyl carbodiimide, et de 4-diméthyl amino pyridine.

L'estérification peut également être réalisée en faisant réagir un chlorure de l'acide (II) ou ($II_A$) sur l'alcool de formule (III) ou ($III_A$) ou sur un dérivé métallique de cet alcool tel qu'un sel d'argent.

La réaction de Wittig utilisée généralement pour préparer les acides de formule (II) ou ($II_A$) fournit des acides dont la double liaison est E + Z.

Les isomères au niveau de la double liaison peuvent être séparés par des méthodes physiques, telle que la chromatographie, soit au niveau des acides, soit au niveau des esters. Des exemples de ces deux méthodes sont donnés plus loin dans la partie expérimentale.

L'agent de clivage du groupement $CO_2R$ est, de préférence, la chaleur utilisée avec un agent d'hydrolyse acide. Comme agent d'hydrolyse acide, on peut utiliser l'acide p-toluène sulfonique.

L'estérification du composé de formule (IV) est effectuée selon les méthodes classiques d'estérification, notamment celles décrites ci-dessus.

L'invention a également pour objet un procédé de préparation selon ce qui précède, caractérisé en ce que les acides de formule (II) ou ($II_A$) dans laquelle R ou $R_{18}$ représente un radical alcoyle renfermant de 1 à 5 atomes de carbone, sont préparés en faisant réagir selon la réaction de Wittig, en présence d'une base forte, un aldéhyde cis sous forme de lactone de formule (V) :

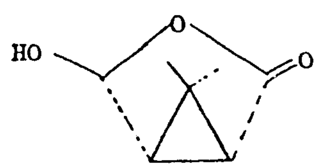

(V)

avec un phosphorane de formule (VII)

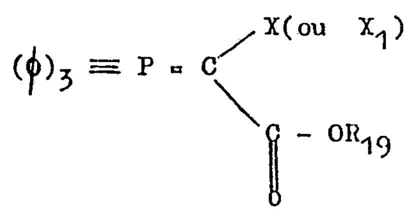

(VII)

formule dans laquelle $R_{19}$ représente un radical alcoyle comportant de 1 à 5 atomes de carbone et X et $X_1$ conservent leurs significations précédentes ou avec un phosphonate de formule (VIII)

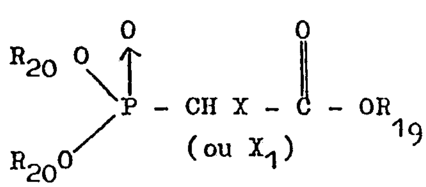

(VIII)

formule dans laquelle $R_{19}$, X et $X_1$ conservent les significations précitées et $R_{20}$ représente un radical alcoyle comportant de 1 à 6 atomes de carbone.

Les acides de formule (II) de stéréochimie déterminée peuvent également être préparés selon le schéma réactionnel suivant :

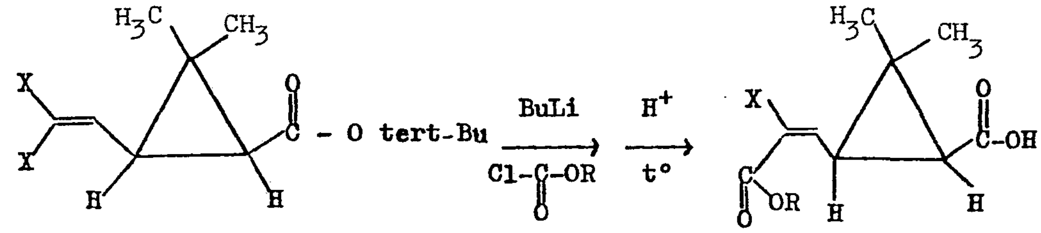


Dans les formules de ce schéma, X représente un atome de fluor, de chrome ou de brome, R représente un radical alcoyle comportant de 1 à 8 atomes de carbone et les composés utilisés possèdent une configuration stérique bien déterminée.

Les acides de formule II pour lesquels X représente un atome de brome peuvent également être préparés en soumettant un composé de formule (IX)

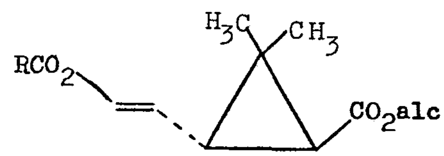

(IX)

dans laquelle la géométrie de la double liaison est E R conservant sa signification précédente et alc représentant un radical alcoyle, à l'action d'un agent de bromuration comme, par exemple, le bromure de pyridinium, puis à l'action d'un agent de débromhydratation, pour obtenir :

— si l'agent de débromhydratation est un agent basique agissant dans des conditions assez douces, le composé de formule (X)

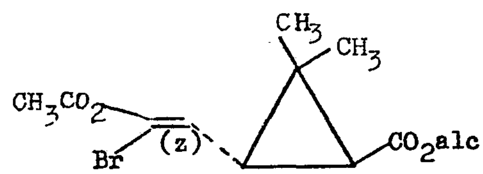

(X)

— si l'agent de débromhydratation est un agent basique agissant dans des conditions plus dures, l'isomère E recherché. La partie expérimentale donne un exemple d'une telle préparation où l'on emploie la triéthylamine pour avoir l'isomère Z et la soude pour avoir l'isomère E.

Les composés de formule V sont ensuite soumis à un agent de clivage de la fonction $CO_2$alc, pour obtenir l'acide correspondant de formule II.

Des exemples de préparations des acides de formule II sont donnés dans la partie expérimentale.

Les acides de formules II et $II_4$ ainsi que les acides de formule IV, obtenus lors de la mise en œuvre du procédé de l'invention, sont des produits nouveaux et sont eux-mêmes un des objet de l'invention.

L'invention a naturellement plus particulièrement pour objet ces produits de formule II, $II_A$ et IV, à titre de produits intermédiaires nécessaires à la préparation des composés de formule I et $I_A$.

Les exemples suivants illustrent l'invention sans toutefois la limiter.

## Exemple 1

(1R,cis) 2,2-diméthyl 3-(E) [2-fluoro 2-(éthoxycarbonyl)éthényl] cyclopropane-1-carboxylate de (S) α-cyano 3-phénoxy benzyle.

Dans une solution de 3,87 g d'acide (1R,cis) 2,2-diméthyl 3(Z + E) (2-fluoro 2-éthoxycarbonyl éthényl)-cyclopropane-1-carboxylique dans 30 $cm^3$ de chlorure de méthylène, on introduit 1,5 cm3 de pyridine, 3,7 g de dicyclohexyl carbodiimide, agite pendant 10 minutes et ajoute en une fois une solution de 4,05 g d'alcool (S) α-cyano 3-phénoxy benzylique dans 10 $cm^3$ de chlorure de méthylène, agite pendant une heure et 30 minute, ajoute 25 mg de 4-diméthyl amino pyridine, agite pendant une heure et trente minutes, refroidit à 0 °C, élimine par filtration l'insoluble formé, concentre le filtrat à sec par distillation sous pression réduite, chromatographie le résidu sur gel de silice en éluant par un mélange de cyclohexane et d'acétate d'éthyle (80/20) cristallise le résidu obtenu dans l'acétate d'éthyle, isole, par essorage le précipité formé, les liqueurs-mères sont chromatographiées sur gel de silice en éluant par un mélange de cyclohexane et d'acétate d'éthyle (9/1), et recueille en tout 4,28 g de (1R,cis) 2,2-diméthyl 3(E) [2-fluoro 2-(éthoxycarbonyl)éthényle] cyclopropane-1-carboxylate de (S) α-cyano 3-phénoxy benzyle et 2,8 g de dérivé 3(Z).

Analyse : $C_{25}H_{24}FNO_2$ (437,47)
Calculé : C % 68,64 H % 5,53 N % 3,20 F % 4,34
Trouvé : C % 68,8 H % 5,5 N % 3,1 F % 4,2

Spectre IR (chloroforme)
— absorption à 1 738 $cm^{-1}$ et 1 722 $cm^{-1}$ attribuée au carbonyle et à l'ester conjugué,
— absorption à 1 611 $cm^{-1}$ attribuée à la double liaison éthylénique,
— absorption à 1 589 $cm^{-1}$-1 489 $cm^{-1}$ attribuée aux noyaux aromatiques,
— absorption à 1 380 $cm^{-1}$ attribuée aux méthyles géminés.

Spectre RMN (Deutérochloroforme)
— pics à 1,2-1,27 ppm attribués aux hydrogènes des méthyles géminés,
— pics à 1,23-1,35-1,47 ppm, 4,15-4,26 ppm et 4,38-4,50 ppm attribués aux hydrogènes du radical éthyle du groupement éthoxy carbonyle,
— pics à 1,88-2,02 ppm attribués à l'hydrogène en position 1 du cyclopropyle,
— pics à 2,8-3,13 ppm attribués à l'hydrogène en position 3 du cyclopropyle,
— pics à 6,05-6,21 et 6,38-6,55 ppm attribués à l'hydrogène de la double liaison éthylénique,
— pic à 6,36 ppm attribué à l'hydrogène porté par le même carbone que le groupement C≡N,
— pics à 6,9-7,58 ppm attribués aux hydrogènes des noyaux aromatiques.

L'acide (1R,cis) 2,2-diméthyl 3-(E,Z) [2-fluoro 2-éthoxycarbonyl éthenyl] cyclopropane-1-carboxylique est obtenu de la manière suivante :

Dans une solution de 12,1 g de diéthyl phosphoro fluoro acétate d'éthyle, dans 120 $cm^3$ de diméthoxyéthane, on introduit à + 5 °C, 2 g de suspension d'hydrure de sodium à 60 % dans l'huile, agite pendant 30 minutes, introduit à 0 °C, 5,7 g de la lactone de l'acide (1R,cis) 2,2-diméthyl 3-dihydroxyméthyl)-cyclopropane-1-carboxylique, agite pendant 2 heures 30 minutes à 20 °C, verse le mélange réactionnel dans un mélange d'eau et de glace contenant du phosphate monosodique, extrait à l'éther éthylique, lave à l'eau les phases organiques réunies, les sèche, les concentre à sec par distillation sous pression réduite, chromatographie le résidu sur gel de silice en éluant avec un mélange de cyclohexane, d'acétate d'éthyle et d'acide acétique (50/50/1) et obtient 3,87 g d'un mélange d'acide (1R,cis) 2,2-diméthyl 3(E,Z) [2-fluoro 2-éthoxycarbonyléthényl] cyclopropane-1-carboxylique.

## Exemple 2

(1R,cis) 2,2-diméthyl 3-(Z) [2-fluoro 2-(éthoxycarbonyl)éthényl]-cyclopropane-1-carboxylate de (S) α-cyano 3-phénoxy benzyle.

A l'issue de la chromatographie de l'exemple 1 permettant d'obtenir le dérivé de structure 3(E) on a obtenu 2,8 g de résidu contenant le dérivé de structure 3(Z), empâte ce résidu dans l'acétate d'éthyle, élimine par filtration l'insoluble formé ; concentre le filtrat à sec par distillation sous pression réduite et obtient un résidu que l'on chromatographie sur gel de silice en éluant avec un mélange de cyclohexane et d'acétate d'éthyle (9/1) et obtient 1.0 g de (1R,cis) 2,2-diméthyl 3-(Z) [2-fluoro 2-(éthoxycarbonyl)éthényl] cyclopropane-1-carboxylate de (S) α-cyano 3-phénoxy benzyle.

Spectre IR (chloroforme)
— absorption à 1 730 $cm^{-1}$ attribuée au carbonyle et à l'ester conjugué,
— absorption à 1 620 $cm^{-1}$ attribuée à la double liaison éthylénique,
— absorption à 1 589-1 489 $cm^{-1}$ attribuée aux noyaux aromatiques,
— absorption à 1 380 $cm^{-1}$ attribuée aux méthyles géminés.

Spectre de RMN (deutérochloroforme)
— pics à 1,2-1,32-1,43 ppm et 4,12-4,23-4,35-4,47 ppm attribués aux hydrogènes du radical éthyle du groupement éthoxy carbonyle,
— pics à 1,23-1,27 ppm attribués aux hydrogènes des méthyles géminés,
— pics à 6,2-6,36 ppm et 6,73-6,9 ppm attribués à l'hydrogène de la double liaison éthylénique,
— pic à 6,47 ppm attribué à l'hydrogène porté par le même carbone que le groupement —CN,
— pics à 6,9-7,58 ppm attribués aux hydrogènes des noyaux aromatiques.

## Exemple 3

(1R,cis) 2,2-diméthyl 3(E) [2-chloro 2-(méthoxycarbonyl)éthényl] cyclopropane-1-carboxylate de (S) α-cyano 3-phénoxy benzyle.

Dans une solution de 3 g de chlorure de l'acide (1R,cis) 2,2-diméthyl 3(E) (2-chloro 2-méthoxy carbonyl éthényl)cyclopropane-1-carboxylique dans 15 $cm^3$ de benzène, on introduit 3 g d'alcool (S) α-cyano 3-phénoxy benzylique introduit à + 50 °C, 1,3 $cm^3$ de pyridine, agite pendant 15 minutes à + 5 °C puis pendant 16 heures à + 20 °C, verse le mélange réactionnel dans un mélange d'eau et d'acide chlorhydrique, extrait à l'éther éthylique, lave à l'eau, sèche, concentre la solution organique à sec par distillation sous pression réduite, chromatographie le résidu sur gel de silice en éluant succéssivement par un mélange de cyclohexane et d'acétate d'éthyle (8/2) puis de cyclohexane et d'acétate d'éthyle (9/1) et obtient 2,1 g de (1R,cis) 2,2-diméthyl 3(E) [2-chloro 2-(méthoxycarbonyl éthenyl] cyclopropane-1-carboxylate de (S) α-cyano 3-phénoxy benzyle, $(\alpha)_D = 50°,5$ (C = 0,8 %, benzène).

Analyse : $C_{24}H_{22}ClNO_5$ 439,9

| | | | | |
|---|---|---|---|---|
| Calculé : | C % 65,53 | H % 5,04 | Cl % 8,05 | N % 3,18 |
| Trouvé : | C % 65,5 | H % 5,2 | Cl % 8,0 | N % 3,0 |

Spectre IR (chloroforme)
— absorption à 1 738 $cm^{-1}$ et 1 719 $cm^{-1}$ attribuée au carbonyle de l'ester,
— absorption à 1 608 $cm^{-1}$ attribuée à —C=C—
— absorption à 1 589 $cm^{-1}$-1 489 $cm^{-1}$ attribuée aux noyaux aromatiques,
— absorption à 1 390 $cm^{-1}$ attribuée aux méthyles géminés.

Spectre de RMN (deutérochloroforme)
— pics à 1,24-1,25 ppm attribués aux hydrogènes des méthyles géminés,
— pics à 1,93-2,07 ppm attribués à l'hydrogène en position 1 du cyclopropyle,
— pics à 2,87-3,01-3,04-3,18 ppm attribués à l'hydrogène en position 3 du cyclopropyle,
— pic à 3,85 ppm attribué au méthyle du méthoxy carbonyle,
— pic à 6,35 ppm attribué à l'hydrogène porté par le même atome de carbone que le groupement —C≡N,
— pics à 6,91-7,5 ppm attribués aux hydrogènes des noyaux aromatiques.

Le chlorure de l'acide (1R,cis) 2,2-diméthyl 3(E) [2-chloro 2-(méthoxy carbonyl)éthényl] cyclopropane-1-carboxylique peut être obtenu de la façon suivante :

Stade A

Acide (1R,cis) 2,2-diméthyl 3(E) [2-chloro 2-méthoxycarbonyl)éthényl] cyclopropane-1-carboxylique.

Dans 50 $cm^3$ de tétrahydrofurane on introduit à 20 °C, 12,6 g de méthoxy carbonyl chloro méthylène triphényl phosphorane, 4,85 g de la lactone de l'acide (1R,cis) 2,2-diméthyl 3-(dihydroxyméthyl)cyclopropane-1-carboxylique en solution dans 40 $cm^3$ de tétrahydrofurane, agite pendant deux heures à 20 °C, porte le mélange réactionnel au reflux, l'y maintient pendant une heure, concentre à sec par distillation sous pression réduite, ajoute au résidu de l'éther éthylique, élimine par filtration l'insoluble formé (oxyde de triphényl phosphine), concentre le filtrat à sec par distillation sous pression réduite, chromatographie le résidu sur gel de silice en éluant par un mélange de benzène et d'acétate d'éthyle (8/2) contenant 1 % d'acide acétique et obtient 2,2 g d'acide (1R,cis) 2,2-diméthyl 3(E)-(2-chloro 2-méthoxy carbonyl éthényl)cyclopropane-1-carboxylique et 3 g d'acide (1R,cis) 2,2-diméthyl 3(Z) (2-chloro 2-méthoxy carbonyl éthényl)cyclopropane-1-carboxylique.

L'acide (1R,cis) 2,2-diméthyl 3(E) (2-chloro 2-méthoxy carbonyl éthényl)cyclopropane-1-carboxylique possède les caractéristiques suivantes :

Spectre IR (chloroforme)
— absorption à 3 500 $cm^{-1}$ attribuée à l'hydroxyle du carboxyle,
— absorption à 1 721 $cm^{-1}$, 1 713 $cm^{-1}$, 1 700 $cm^{-1}$ attribuée au carbonyle,
— absorption à 1 490 $cm^{-1}$-1 410 $cm^{-1}$ attribuée à —C=C—
— absorption à 1 393 $cm^{-1}$-1 380 $cm^{-1}$ attribuée aux hydrogènes des méthyles géminés.

Spectre de RMN (deutérochloroforme)
— pics à 1,3-1,32 ppm attribués aux méthyles géminés,
— pics à 1,87-2,02 ppm attribués à l'hydrogène en position 1 du cyclopropyle,
— pics à 2,82, 2,97 ppm-2,98, 3,13 ppm attribués à l'hydrogène en position 3 du cyclopropyle,
— pic à 3,82 ppm attribué aux hydrogènes du méthyle du méthoxy carbonyle,
— pics à 6,72-6,78 ppm attribués à l'hydrogène de la double liaison éthylénique de la chaîne latérale en position 3 du cyclopropyle,
— pic à 11 ppm attribué à l'hydrogène du carboxyle.

L'acide (1R,cis) 2,2-diméthyl 3(Z) (2-chloro 2-méthoxy carbonyl éthényl)cyclopropane-1-carboxylique présente les caractéristiques suivantes :

Spectre IR : (chloroforme)
— absorption à 3 500 $cm^{-1}$ attribuée à l'hydroxyle du carboxyle (mono + dimère),
— absorption à 1 725 $cm^{-1}$ attribuée à 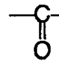 ester,

— absorption à 1 700 $cm^{-1}$ attribuée à l'hydrogène du carboxyle (monomère)
— absorption à 1 623 $cm^{-1}$ attribuée à —C=C—
— absorption à 1 393 $cm^{-1}$-1 381 $cm^{-1}$ attribuée aux hydrogènes des méthyles géminés.

Spectre de RMN (deutérochloroforme)
— pics à 1,32-1,35 ppm attribués aux hydrogènes des méthyles géminés,
— pics à 1,95 à 2,5 ppm attribués aux hydrogènes en position 1 et 3 du cyclopropyle,
— pic à 3,83 ppm attribué aux hydrogènes du méthyle du méthoxyle,
— pics à 7,28-7,45 ppm attribués à l'hydrogène de la double liaison de la chaîne latérale en position 3 du cyclopropyle,
— pic à 10,75 ppm attribué à l'hydrogène du carboxyle.

Stade B

Chlorure de l'acide (1R,cis) 2,2-diméthyl 3(E) [2-chloro 2-(méthoxy carbonyl)éthényl] cyclopropane-1-carboxylique.

On mélange 2,9 g d'acide (1R,cis) 2,2-diméthyl 3(E) [2-chloro 2-(méthoxy carbonyl)éthényl] cyclopropane-1-carboxylique, 20 $cm^3$ d'isoprène et 10 $cm^3$ de chlorure de thionyle, agite à 20 °C pendant 3 heures, élimine l'isoprène et le chlorure de thionyle par distillation sous pression réduite et obtient 6 g de chlorure brut de l'acide (1R,cis) 2,2-diméthyl 3(E)-[2-chloro 2-(méthoxy carbonyl)éthényl] cyclopropane-1-carboxylique.

Exemple 4

(1R,cis) 2,2-diméthyl 3(Z) [2-bromo 2-(propoxycarbonyl)éthényl] cyclopropane-1-carboxylate de (S) α-cyano 3-phénoxy benzyle.

Dans une solution de 2,9 g d'acide (1R,cis) 2,2-diméthyl 3(Z)-[2-bromo 2-(propyloxycarbonyl)éthényl] cyclopropane-1-carboxylique dans 40 $cm^3$ de chlorure de méthylène, on ajoute 0,9 $cm^3$ de pyridine, 2,1 g de dicyclohexyl carbodiimide, agite pendant 15 minutes, introduit 2,5 g de (S) α-cyano 3-phénoxy benzyle en solution dans 5 $cm^3$ de chlorure de méthylène, ajoute 25 mg de 4-diméthyl-aminopyridine, agite pendant 2 heures, élimine par filtration l'insoluble formé, concentre à sec le filtrat par distillation sous pression réduite, chromatographie le résidu sur gel de silice en éluant par un mélange de cyclohexane et d'acétate d'éthyle (9/1), obtient 4,26 g de (1R,cis) 3(Z)-[2-bromo 2-(propoxycarbonyl)éthényl] cyclopropane-1-carboxylate de (S) α-cyano 3-phénoxy benzyle, F = 64 °C.

Analyse : $C_{26}$ $H_{26}$ $BrNO_5$ 512,41

| | | | | |
|---|---|---|---|---|
| Calculé : | C % 60,95 | H % 5,11 | N % 2,73 | Br % 15,6 |
| Trouvé : | C % 61,1 | H % 5,3 | N % 2,7 | Br % 15,5 |

Spectre IR (chloroforme)
— absorption à 1 743-1 718 $cm^{-1}$ attribuée au carbonyle de l'ester et de l'ester conjugué,
— absorption à 1 615 $cm^{-1}$ attribuée à —C=C—,
— absorption à 1 588-1 488 $cm^{-1}$ attribuée aux noyaux aromatiques,
— absorption à 1 390-1 380 $cm^{-1}$ attribuée aux méthyles géminés.

Spectre de RMN (deutérochloroforme)
— pics à 0,88-1,0-1,12 ppm attribués aux hydrogènes du méthyle du propyle,
— pics à 1,27-1,32 ppm attribués aux hydrogènes des méthyles géminés,
— pics à 4,08-4,2-4,32 ppm attribués aux hydrogènes en position 1 du propyle,
— pic à 6,4 ppm attribué à l'hydrogène porté par le même carbone que le groupement —C≡N
— pics à 7,57-7,67 ppm attribués à l'hydrogène de la double liaison éthylénique,
— pics à 6,9-7,58 ppm attribués aux hydrogènes des noyaux aromatiques.

L'acide (1R,cis) 2,2-diméthyl 3(Z)-[2-bromo 2-(propoxy carbonyl)éthényl] cyclopropane-1-carboxylique est préparé de la façon suivante :

Stade A

(1R,cis) 2,2-diméthyl 3(Z)-[2-bromo 2-(propyloxy carbonyl)éthényl] cyclopropane-1-carboxylate de terbutyle.

Dans une solution de 28,3 g d'ester terbutylique de l'acide (1R,cis) 2,2-diméthyl 3-(2,2-dibromovinyl) cyclopropane-1-carboxylique dans un mélange de 120 $cm^3$ de tétrahydrofurane et de 120 $cm^3$ d'éther éthylique, on introduit à − 115 °C en 25 minutes environ 50 $cm^3$ de solution de butyl lithium dans l'hexane, titrant à 1,6 N, agite pendant 15 minutes à − 115 °C, ajoute progressivement 10 $cm^3$ de chloroformiate de n-propyle, agite pendant 20 minutes à − 115 °C, puis pendant une heure à − 65 °C, verse le mélange réactionnel dans une solution aqueuse de phosphate monosodique, extrait à l'éther, lave à l'eau les solutions éthérées réunies, les sèche, les concentre à sec par distillation sous pression réduite, chromatographie sur gel de silice en éluant avec un mélange de cyclohexane et d'acétate d'éthyle (95/5) et obtient 3,52 g de (1R,cis) 2,2-diméthyl 3(Z)-[2-bromo 2-(propyloxy carbonyl)éthényl] cyclopropane-1-carboxylate de terbutyle, 3,16 g de (1R,cis) 2,2-diméthyl 3(E)-[(2-bromo 2-(propyloxy carbonyl)éthényl] cyclopropane-1-carboxylate de terbutyle.

Le (1R,cis) 2,2-diméthyl 3(Z)-[2-bromo 2-(propyloxy carbonyl)éthényl] cyclopropane-1-carboxylate de terbutyle présente les caractéristiques suivantes :

Spectre IR (chloroforme)
— absorption à 1 715 $cm^{-1}$ attribuée à C=O,
— absorption à 1 614 $cm^{-1}$ attribuée à C=C.

Spectre RMN (deutérochloroforme)
— pics à 0,9-0,97-1,05 ppm attribués aux hydrogènes du méthyle terminal du propyle,
— pics à 1,3-1,33 ppm attribués aux hydrogènes des méthyles géminés,
— pic à 1,52 attribué aux hydrogènes du radical terbutyle,
— pics à 1,88-1,97 et 2,05-2,15-2,24 ppm attribués aux hydrogènes en position 1 et 3 du cyclopropyle,
— pics à 4,1-4,18-4,26 ppm attribués aux hydrogènes du méthylène en position 1 du propyle,
— pics à 7,6-7,75 ppm attribués à l'hydrogène de la double liaison éthylénique.

Stade B

Acide (1R,cis) 2,2-diméthyl 3(Z)-[2-bromo 2-(propyloxy carbonyl)éthényl] cyclopropane-1-carboxylique.

Dans une solution de 3,45 g d'ester terbutylique de l'acide (1R,cis) 2,2-diméthyl 3(Z) [2-bromo 2-(propyloxy carbonyl)éthényl] cyclopropane-1-carboxylique dans 35 $cm^3$ de toluène, on introduit 0,35 g d'acide paratoluène sulfonique monohydraté, place le ballon contenant le mélange réactionnel dans un bain d'huile à 120 °C, pendant 10 minutes, ramène rapidement la température du mélange réactionnel à 20 °C, ajoute de l'éther, lave à l'eau les extraits organiques réunis, sèche, concentre à sec par distillation sous pression réduite et obtient 2,9 g d'acide (1R,cis) 2,2-diméthyl 3(Z)-[2-bromo 2-(propyloxy carbonyl)éthényl] cyclopropane-1-carboxylique.

Exemple 5

(1R,cis) 2,2-diméthyl 3(E)-[2-bromo 2-(propoxy carbonyl)éthényl] cyclopropane-1-carboxylate de (S) α-cyano 3-phénoxy benzyle.

Dans une solution de 2,6 g d'acide (1R,cis) 2,2-diméthyl 3(E)-[2-bromo 2-(propyloxy carbonyl)-éthényl] cyclopropane-1-carboxylique dans 40 $cm^3$ de chlorure de méthylène on introduit 0,9 $cm^3$ de pyridine, 2 g de dicyclohexyl carbodiimide, agite pendant 10 minutes, ajoute 2 g d'alcool (S) α-cyano 3-phénoxy benzylique, agite pendant 10 minutes, ajoute 25 mg de 4-diméthyl-amino-pyridine, agite pendant une heure et trente minutes, élimine par filtration l'insoluble formé, concentre le filtrat à sec par distillation sous pression réduite, chromatographie le résidu sur gel de silice en éluant avec un mélange de cyclohexane et d'acétate d'éthyle (95/5), obtient 0,743 g d'ester attendu et 3,64 g d'un mélange que l'on dissout à chaud dans 4 volumes d'éther isopropylique, agite à 20 °C, isole par essorage le précipité formé, le sèche, le joint aux 0,743 g de produit obtenu précédemment et recueille 2,32 g de (1R,cis) 2,2-diméthyl 3(E)[2-bromo 2-(propoxy carbonyl)-éthényl] cyclopropane-1-carboxylate de (S) α-cyano 3-phénoxy. benzyle, F = 68 °C.

Analyse : $C_{26}$ $H_{23}$ $BrNO_5$ (512,41)

| | | | | |
|---|---|---|---|---|
| Calculé : | C % 60,95 | H % 5,11 | N % 2,73 | Br % 15,6 |
| Trouvé : | C % 61 | H % 5,1 | N % 2,5 | Br % 15,5 |

Spectre IR (chloroforme)
— absorption à 1 737 $cm^{-1}$ attribuée au carbonyle de l'ester,
— absorption à 1 705 $cm^{-1}$ attribuée au carbonyle de l'ester conjugué,
— absorption à 1 605-1 610 $cm^{-1}$ attribuée à —C=C—,
— absorption à 1 585-1 485 $cm^{-1}$ attribuée aux noyaux aromatiques.

Spectre de RMN (deutérochloroforme)
— pics à 0,88-1,0-1,12 ppm attribués aux hydrogènes du méthyle du propoxyle,
— pics à 1,22-1,23 ppm attribués aux hydrogènes des méthyles géminés,
— pics à 1,92-2,06 ppm attribués à l'hydrogène en position 1 du cyclopropyle,
— pics à 4,08-4,18-4,28 ppm attribués aux hydrogènes du méthylène en position 1 du propyle,
— pics à 6,38 ppm attribué à l'hydrogène porté par le même carbone que le —C≡N,
— pics de 6,9 à 7,51 ppm attribués à l'hydrogène de la double liaison éthylénique,
— pics à 6,92-7,6 ppm attribués aux hydrogènes des noyaux aromatiques.

L'acide (1R,cis) 2,2-diméthyl 3(E)-[2-bromo 2-(propoxy carbonyl)éthényl] cyclopropane-1-carboxylique peut être obtenu de la façon suivante :

A une solution de 3,1 g d'ester terbutylique de l'acide (1R,cis) 2,2-diméthyl 3(E)-[2-bromo 2-(propoxy carbonyl)éthényl] cyclopropane-1-carboxylique obtenu en même temps que l'isomère 3(Z) au stade A de la préparation de l'acide 3(Z) correspondant faisant suite à l'exemple 4 dans 31 $cm^3$ de toluène, on introduit 0,31 g d'acide paratoluène sulfonique monohydraté, place le ballon contenant le mélange réactionnel dans un bain d'huile à 120 °C, pendant 15 minutes, amène rapidement la température du mélange réactionnel à 20 °C, extrait à l'éther, lave les extraits organiques à l'eau, les sèche, les concentre à sec par distillation sous pression réduite et obtient 2,6 g d'acide (1R,cis) 2,2-diméthyl 3(E)-[2-bromo 2-(propoxy carbonyl)éthényl] cyclopropane-1-carboxylique.

## Exemple 6

Acide (1R,cis) 2,2-diméthyl 3-(E)-[2-fluoro-2(éthoxy carbonyl)éthényl] cyclopropane-1-carboxylique.

On prépare l'acide (1R,cis) 2,2-diméthyl 3-(E,Z)[2-fluoro 2-éthoxy carbonyl éthényl] cyclopropane-1-carboxylique, à partir de 12,2 g de la lactone de l'acide (1R,cis) 2,2-diméthyl 3-(dihydroxy méthyl) cyclopropane carboxylique en opérant comme à l'exemple 1, en remplaçant la chromatographie indiquée à l'exemple 1, en remplaçant la chromatographie indiquée à l'exemple 1 par une chromatographie sur gel de silice en éluant par un mélange de cyclohexane et d'acétate d'éthyle (75/25) puis avec le mélange en proportion (50/50) et obtient 14,5 g d'acide (1R,cis) 2,2-diméthyl 3-(E) (2-fluoro 2-éthoxy carbonyl éthényl) cyclopropane-1-carboxylique $[\alpha_D]$ = — 42,5° (c = 1 %, chloroforme).

Spectre de RMN (deutérochloroforme)
— pic à 1,28 ppm attribué aux hydrogènes des méthyles géminés,
— pics à 1,23-1,35-1,47 ppm et 4,13-4,25-4,37-4,48 ppm attribués aux hydrogènes de l'éthyle de l'éthoxy carbonyle,
— pics à 1,82-1,97 ppm attribués à l'hydrogène en position 1 du cyclopropyle,
— pics à 2,75-2,9-3,05 ppm attribués à l'hydrogène en position 3 du cyclopropyle,
— pics à 6,12-6,28-6,47-6,63 ppm attribués à l'hydrogène éthylénique (J ≃ 21 H Hz correspondant à un dérivé cis),
— pic à 11,28 ppm attribué à l'hydrogène du carboxyle.

## Exemple 7

Acide (1R,cis) 2,2-diméthyl 3-(Z)-[2-fluoro 2-(éthoxy carbonyl)éthényl] cyclopropane-1-carboxylique.

En poursuivant la chromatographie de l'exemple 6 on obtient 4,64 g d'acide (1R,cis 2,2-diméthyl 3(Z)-[2-fluoro 2-(éthoxycarbonyl)éthényl] cyclopropane-1-carboxylique.

Par estérification par l'alcool (S) α-cyano 3-phénoxy benzylique les acides « E » et « Z » conduisent aux esters des exemples 1 et 2.

## Exemple 8

(1R,cis) 2,2-diméthyl 3(Z)-[2-fluoro-2-éthoxy carbonyl)éthényl] cyclopropane-1-carboxylate de (S) α-cyano 3-phénoxy benzyle.

Dans une solution de 4,9 g d'acide (1R,cis) 2,2-diméthyl 3(Z)-[2-fluoro-2-éthoxy carbonyl éthényl] cyclopropane-1-carboxylique, dans 39 $cm^3$ de chlorure de méthylène, on ajoute 1,9 $cm^3$ de pyridine, 4,8 g de dicyclohexyl carbodiimide, agite, introduit une solution de 5,3 g d'alcool (S) α-cyano 3-phénoxy

benzylique dans 9,8 $cm^3$ de chlorure de méthylène, agite pendant 2 heures, ajoute 30 mg de 4-diméthyl amino pyridine, agite pendant 2 heures, refroidit à 0 °C, élimine par filtration l'insoluble formé, concentre à sec par distillation sous pression réduite, chromatographie le résidu sur gel de silice en éluant successivement par un mélange de cyclohexane et d'acétate d'éthyle (9/1) puis le chlorure de méthylène et d'éther du pétrole (eb 35-70 °C) (6/4) et obtient 6,73 g de (1R,cis) 2,2-diméthyl 3-(Z)-[2-fluoro-2(éthoxy carbonyle)éthényl] cyclopropane-1-carboxylate de (S) α-cyano 3-phénoxy benzyle présentant les mêmes caractéristiques physiques que le composé obtenu à l'exemple 2.

En opérant comme à l'exemple 1 à partir des alcools correspondants, on a préparé les produits suivants ; (exemples 9, 10, 11 et 12).

Exemple 9

(1R,cis ΔE) 2,2-diméthyl 3[(2-fluoro 2-éthoxy carbonyl)éthényl] cyclopropane carboxylate de R/3-éthynyl 3-phénoxy-phényl-méthyle. $R^t$ = 72 %.

$$\alpha_D = +40° \mp 1{,}5° \quad c = 1\ \%\ CHCl_3.$$

Exemple 10

(1R,cis, ΔE) 2,2-diméthyl 3 [2-fluoro 2-éthoxy carbonyl éthényl] cyclopropane carboxylate de (R) (3-phénoxy phényl) éthyle. Rendement 81 %.

$$\alpha_D = +94{,}5° \mp 2{,}5° \quad c = 0{,}5\ \%\ CHCl_3.$$

Exemple 11

(1R,cis ΔE) 2,2-diméthyl 3 [2-fluoro 2-éthoxy carbonyl)éthényl] cyclopropane-1-carboxylate de (S) α-cyano 3-phénoxy 4-fluoro-benzyle.

$$\alpha_D = +50° \pm 2{,}5° \quad (c = 0{,}5\ \%\ CHCl_3).$$

Exemple 12

(1R,cis ΔE) 2,2-diméthyl 3 [(2-fluoro 2-éthoxy carbonyl)éthényl] cyclopropane-1-carboxylate de 3-phénoxy benzyle.

| | |
|---|---|
| Spectre IR : | |
| C=O ester<br>C=O ester conjuguée | } 1 725 $cm^{-1}$ |
| C=C conjuguée | 1 655 $cm^{-1}$ |
| aromatiques | 1 588-1 489 $cm^{-1}$ |
| Gem. diméthyle | 1 390-1 380 $cm^{-1}$ |

Exemple 13

(1R,trans) 2,2-diméthyl 3 [(ΔE, 2-fluoro 3-oxo 3-éthoxy propényl] cyclopropane carboxylate de (S) α-cyano 3-phénoxy benzyle.

En opérant comme à l'exemple 1, à partir de l'acide (1R trans) 2,2-diméthyl 3 [ΔE 2-fluoro 3-oxo 3-éthoxy propényl] cyclopropane carboxylique et de l'alcool (S) α-cyano 3-phénoxybenzylique, on obtient le produit recherché.

$$\alpha_D = -33{,}5° \mp 2{,}5° \ (c = 0{,}5\ \%\ CHCl_3).$$

L'acide (1R,trans) 2,2-diméthyl 3 [ΔE 2-fluoro 3-oxo 3-éthoxy propényl] cyclopropane carboxylique a été préparé comme suit :

On ajoute à 2 ~ 10 °C en 30 minutes dans une suspension renfermant 60 $cm^3$ de 1,2-diméthoxyéthane et 2,6 g d'une suspension d'hydrure de sodium titrant 60 % dans l'huile, une solution renfermant 7,7 g de diéthyl-phospono-fluoro acétate d'éthyle préparé selon le procédé décrit dans Ann. Chem. (1964) 674,60 $cm^3$ de 1,2-diméthoxyéthane, et 4 g d'acide (1R, trans) 2,2-diméthyl 3-formyl cyclopropane carboxylique.

On maintient l'agitation pendant 15 minutes à 5 °C puis 3 heures à la température ambiante. On obtient une solution que l'on verse sur une solution aqueuse de phosphate monosodique à 5 °C et agite pendant 10 minutes. On extrait à l'acétate d'éthyle, lave à l'eau, sèche et concentre à sec à 40 °C sous pression réduite. On obtient 6,5 g d'une huile que l'on chromatographie sur silice en éluant par le mélange : hexane, acétate d'éthyle-acide acétique (70-30-1). On obtient ainsi 4 g de produit recherché.

Exemple 14

(1R,trans) 2,2-diméthyl 3 [(ΔE) 2-fluoro 3-oxo 3-méthoxy propényl] cyclopropane carboxylate de (S) α-cyano 3-phénoxy benzyl.

Stade A

(1R,trans) 2,2-diméthyl 3-[(ΔE) 2-fluoro 3-oxo 3-hydroxy propényl] cyclopropane carboxylate de (S) α-cyano 3-phénoxybenzyle.

On ajoute 50 mg d'acide paratoluensulfonique monohydraté dans une solution renfermant 1 g de (1R trans) 2,2-diméthyl 3 [(ΔE) 2-fluoro 3-oxo 3-éthoxy propényl] cyclopropane carboxylate de (S) α-cyano 3-phénoxybenzyle, 1 $cm^3$ d'eau et 4 $cm^3$ de dioxanne. On porte le mélange réactionnel au reflux pendant 8 heures et amène à sec à température ambiante sous pression réduite. On reprend le résidu avec du chlorure de méthylène, lave à l'eau et sèche. On amène à sec pour obtenir 1,1 g d'huile que l'on chromatographie sur silice en éluant par le mélange : hexane-acétate d'éthyle-acide acétique (60-40-1). On obtient ainsi 280 mg du produit recherché.

Stade B

(1R,trans) 2,2-diméthyl 3-[ΔE 2-fluoro 3-oxo 3-méthoxy propényl] cyclopropane carboxylate de (S) α-cyano 3-phénoxy benzyle.

A une température comprise entre + 5 °C et + 10 °C, on ajoute un léger excès de diazométhane dissous dans du chlorure de méthylène, dans 2 $cm^3$ d'une solution de chlorure de méthylène renfermant 860 mg de produit préparé au stade A. On maintient l'agitation 15 minutes à 5 °C puis 30 minutes à la température ambiante. On ajoute quelques gouttes d'acide acétique. On amène à sec. On obtient ainsi 950 mg d'une huile que l'on chromatographie sur silice en éluant par le mélange : hexane-acétate d'éthyle (85-15). On obtient 700 mg de produit recherché.

$$\alpha_D = -31° \mp 2{,}5° \quad c = 0{,}25\ \%\ CHCl_3$$

Exemple 15

(1R,trans) 2,2-diméthyl 3 [(ΔZ) 2-fluoro 3-oxo 3-éthoxy propényl] cyclopropane carboxylate de (S) α-cyano 3-phénoxy benzyle.

En opérant comme à l'exemple 1, à partir de l'acide (1R, trans) 2,2-diméthyl 3 [(ΔZ) 2-fluoro 3-oxo 3-éthoxy propényl] cyclopropane carboxylique et de l'alcool (S) α-cyano 3-phénoxybenzylique, on obtient le produit recherché.

$$\alpha_D = +15° \mp 2° \quad c = 0{,}5\ \%\ CHCl_3$$

L'acide (1R,trans) 2,2-diméthyl 3 [(ΔZ) 2-fluoro 3-oxo 3-éthoxy propényl] cyclopropane carboxylique a été préparé comme suit :

On porte au reflux pendant 1 heure, une solution renfermant 4 g d'acide (1R, trans) 2,2-diméthyl 3-formyl cyclopropane carboxylique et 7 g de sel de sodium de l'oxalofluoroacétate de diéthyle. On laisse revenir à la température ambiante, verse sur une solution saturée de phosphate monosodique à 0 °C, + 5 °C. On extrait à l'éther, lave à l'eau et sèche. On obtient 8,2 g d'un produit que l'on chromatographie sur silice en éluant par le mélange hexane-acétate d'éthyl-acide acétique (70-30-1). On isole ainsi 4,3 g du produit recherché.

Exemple 16

(1R,cis) 2,2-diméthyl 3 [(ΔZ) 2-fluoro 3-oxo 3-méthoxy propényl] cyclopropane carboxylate de (S) α-cyano 3-phénoxybenzyle.

Stade A

(1R,cis) 2,2-diméthyl 3-[(ΔZ) 2-fluoro 3-oxo 3-hydroxy propényl] cyclopropane carboxylate de (S) α-cyano 3-phénoxybenzyle.

En opérant comme à l'exemple 14, stade A, à partir du produit (1R,cis) 2,2 diméthyl 3 [(ΔZ) 2-fluoro 2-éthoxy carbonyl)éthényl] cyclopropane carboxylate de (S) α-cyano 3-phénoxy benzyle, on obtient le produit recherché.

Stade B

En opérant comme à l'exemple 14, stade B, à partir du produit préparé au stade A, on obtient le produit recherché.

$\alpha_D = - 2,5° \mp 2°$ c = 0,4 % $CHCl_3$

Exemple 17

(1R,cis) 2,2-diméthyl 3-[(ΔZ) 2-fluoro 3-oxo 3-terbutoxy propényl] cyclopropane carboxylate de (S) α-cyano 3-phénoxy benzyle.

On maintient sous agitation pendant 2 heures, 2,3 g de (1R,cis) 2,2 diméthyl-3 [2-fluoro 3-oxo 3-hydroxy (Z) propényl] cyclopropanecarboxylate de (S) α-cyano 3-phénoxy benzyle, 15 $cm^3$ d'acétate d'éthyle et 2,4 g de N-(1-méthyl éthyl) N'-(1-méthyl éthyl) carbamimidate de terbutyle. On filtre, amène le filtrat à sec et obtient 2,6 g de produit que l'on purifie par chromatographie sur silice, éluant : n-hexane-éther isopropylique (8-2) sous pression d'azote. On obtient 2,2 g de produit que l'on recristallise dans l'éther isopropylique. On obtient 1,4 g de produit recherché fondant à 103 °C.

$\alpha_D = + 2,5° \mp 3°$ c = 0,2 % $CHCl_3$

Le N-(1-méthyl éthyl) N'-(1-méthyl éthyl)carbamimidate de terbutyle a été préparé comme suit :
On agite 98,7 g de N,N' diisopropylcarbodiimide et 57,9 g d'alcool terbutylique en présence de 5 g de chlorure cuivreux pendant 4 jours et demi à température ambiante. On obtient 117,7 g de produit attendu après distillation du mélange réactionnel sous 9 mm de mercure (et 74 °C sous 9 mm Hg).

Exemple 18

(1R,cis) 2,2-diméthyl 3-[(ΔZ) 2-fluoro 3-oxo 3-(1,1,1,3,3,3-hexafluoro)isopropoxy n'-propényl] cyclopropane carboxylate de (S) α-cyano 3-phénoxy benzyle.

On introduit, à + 5 °C + 10 °C, en 10 minutes, une solution renfermant 0,6 g de dicyclohexyl carbodiimide, 21 mg de 4-diméthylamino pyridine et 5 $cm^3$ de chlorure de méthylène dans une solution renfermant 1,1 g de (1R, cis) 2,2-diméthyl 3-[2-fluoro 3-oxo 3-hydroxy (Z) propényl] cyclopropane carboxylate de (S) α-cyano 3-phénoxybenzyle, 5 $cm^3$ de chlorure de méthylène et 0,5 $cm^3$ de 1,1,1,3,3,3-hexafluoropropan 2-ol. On filtre, amène le filtrat à sec, chromatographie le résidu obtenu sur silice en éluant par le mélange hexane-acétate d'éthyle (9-1) et en opérant sous pression d'azote. On obtient ainsi 750 mg du produit recherché.

$\alpha_D = + 18,5° \mp 1°$ c = 1 % benzène

Exemple 19

(1R,cis) 2,2-diméthyl 3-[2-fluoro 3-oxo 3-méthoxy (E) propényl] cyclopropane carboxylate de (S) α-cyano 3-phénoxybenzyle.

Stade A

(1R,cis) 2,2 diméthyl 3-[2-fluoro 3-oxo 3-hydroxy (E) propényl] cyclopropane carboxylate de (S) α-cyano 3-phénoxy benzyle.

On porte au reflux pendant 24 heures une solution renfermant 2,5 g de (1R,cis) 2,2-diméthyl 3-[(E) 2-fluoro 2-éthoxy carbonyl éthényl] cyclopropane-1-carboxylate de (S) α-cyano 3-phénoxybenzyle, 10 $cm^3$ de dioxanne, 2,5 $cm^3$ d'eau et 1 g d'acide paratoluène sulfonique manohydraté. On laisse revenir à la température ambiante, dilue au chlorure de méthylène, et lave à l'eau. On sèche la phase organique, la filtre et concentre le filtrat sous pression réduite. On chromatographie le résidu obtenu en éluant par le mélange cyclohexane-acétate d'éthyle-acide acétique (60-40-1). On récupère ainsi le produit recherché (980 mg).

Stade B

(1R,cis)-2,2-diméthyl 3-[2-fluoro 3-oxo 3 [méthoxy (E) propényl] cyclopropanecarboxylate de (S) α-cyano 3-phénoxybenzyle.

En opérant comme à l'exemple 14, stade B à partir du (1R,cis) 2,2-diméthyl 3-[2-fluoro 3-oxo 3-hydroxy (E) propényl] cyclopropane carboxylate de (S) α-cyano 3-phénoxybenzyle, on obtient le produit recherché F = 70 °C.

$\alpha_D = + 52° \mp 1{,}5°$    c = 1 % $CHCl_3$.

En opérant comme à l'exemple précédant à partir des alcools correspondants, on a préparé les produits suivants.

Exemple 20

(1R,cis) 2,2 diméthyl 3-[2-fluoro, 3-oxo 3-n-propyloxy (E) propényl] cyclopropane carboxylate de (S) α-cyano-phénoxy benzyle.

$\alpha_D = + 38{,}5° \mp 2°$    c = 0,7 % $CHCl_3$

Exemple 21

(1R,cis) 2,2-diméthyl 3-[(ΔE) 2-fluoro 3-oxo 3-terbutoxy propényl] cyclopropane carboxylate de (S) α-cyano 3-phénoxy benzyle.

En opérant comme à l'exemple 17, à partir de (1R,cis) 2,2-diméthyl 3 [2-fluoro 3-oxo 3-hydroxy (E) propényl] cyclopropane carboxylate de (S) α-cyano 3-phénoxy benzyle et de N-(1-méthyl éthyl) N'-(1-méthyl éthyl) carbamimidate de terbutyle, on obtient le produit recherché (Rendement 61 %).

$\alpha_D = + 26{,}5°$    (c = 0,25 % $CHCl_3$).

Exemple 22

(1R,cis) 2,2-diméthyl 3 [(ΔE) 2-fluoro 3-oxo 3(1,1,1,3,3,3) hexafluoro isopropoxy n-propényl] cyclopropane carboxylate de (S) α-cyano 3-phénoxy benzyle.

En opérant comme à l'exemple 14, stade B, à partir de (1R,cis) 2,2-diméthyl 3-[2-fluoro 3-oxo 3-hydroxy (E) propényl] cyclopropane carboxylate de (S) α-cyano 3-phénoxy benzyle et de 1,1,1,3,3,3-hexafluoropropan 2-ol, on obtient le produit recherché.

$\alpha_D = + 21° \mp 2°$    c = 0,5 % $CHCl_3$.

Exemple 23

(1R,cis) 2,2-diméthyl 3-[2-fluoro 3-oxo 3-isopropyloxy (E) propényl] cyclopropane carboxylate de (S) α-cyano 3-phénoxy benzyle.

$\alpha_D = + 46° \mp 1°$    c = 1 % $CHCl_3$.

Exemple 24

(1R,cis) 2,2-diméthyl 3-[2-fluoro 3-oxo 3-cyclopropyloxy (E)-propényl] cyclopropane carboxylate de (S) α-cyano 3-phénobenzyle.

F = 50 °C

$\alpha_D = + 35° \pm 1°$    (c = 1,3 % $CHCl_3$)

Exemple 25

(1R,cis, ΔE) 2,2-diméthyl 3 [2-fluoro 3-oxo 3(β-méthoxy éthoxy) propényl] cyclopropane carboxylate de (S) α-cyano 3-phénoxy benzyle.

$\alpha_D = 47° \mp 2{,}5°$    c = 0,5 % $CHCl_3$

Exemple 26

(1R,trans) 2,2-diméthyl 3 [(ΔZ) 2-fluoro 3-oxo 3-hydroxy propényl] cyclopropane carboxylate de (S) α-cyano 3-phénoxybenzyle.

Stade A

(1R,trans) 2,2-diméthyl 3 [(ΔZ) 2-fluoro 3-oxo 3-hydroxy propényl] cyclopropane carboxylate de (S) α-cyano métaphénoxybenzyle.

En opérant comme à l'exemple 14, stade A, à partir de l'ester correspondant (ΔZ) on a obtenu le produit recherché.

Stade B

(1R,trans) 2,2-diméthyl 3-[(ΔZ) 2-fluoro 3-oxo 3-méthoxy propényl] cyclopropane carboxylate de (S) α-cyano 3-phénoxybenzyle.

En opérant comme à l'exemple 14, stade B, à partir du produit préparé au stade A, on a obtenu le produit recherché.

$$\alpha_D = + 15{,}5° \mp 2{,}5° \qquad c = 0{,}3\ \%\ CHCl_3$$

Exemple 27

(1R,cis) 2,2-diméthyl 3 [(ΔZ) 2-chloro 2-méthoxy carbonyl éthényl] cyclopropane carboxylate de (S) α-cyano 3-phénoxy benzyle.

En opérant comme à l'exemple 1, à partir de l'acide (1R,cis) 2,2-diméthyl 3(Z) [2-chloro 2-méthoxy carbonyl éthényl], cyclopropane carboxylique et de l'alcool (S) α-cyano 3-phénoxybenzyle, on obtient le produit recherché.

$$\alpha_D = 62{,}5° \mp 1{,}5° \qquad c = 1\ \%\ \text{benzène.}$$

Exemple 28

(1R,cis) 2,2-diméthyl 3 [(ΔE) 2-chloro 2-méthoxy carbonyl éthényl] cyclopropane carboxylate de (S) 2-méthyl 4-oxo 3-(2-propényl) 2-cyclopenten 1-yle.

Le produit a été préparé comme le produit précédent à partir du chlorure de l'acide (1R,cis) 2,2-diméthyl 3(Z) [2-chloro 2-méthoxy carbonyl éthényl] cyclopropane carboxylique et de la (4S)-hydroxy 3-méthyl 2-(2-propényl) 2-cyclopenten-1-one.

$$\alpha_D = - 15° \mp 4° \qquad c = 0{,}25\ \%\ \text{benzène.}$$

RMN $CDCl_3$ ppm
1,28 et 1,3 H des méthyle en 2
3,8 H de $-CO_2CH_3$
2,02 H de $H_3C$
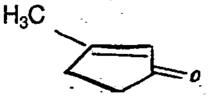

6,8-7 H éthylénique porté par le carbone en 1 du radical 2 méthoxy carbonyl éthényle.

Exemple 29

(1R,cis) 2,2-diméthyl 3-[(Z) 2-chloro 2-méthoxy carbonyl éthényl] cyclopropane carboxylate de (1S) 2-méthyl-4-oxo 3-(2-propényl)-2-cyclopenten-1-yle.

Le produit a été préparé à partir du chlorure de l'acide (1R,cis) 2,2-diméthyl 3-[(Z) 2-chloro 2-méthoxy carbonyl éthényl] cyclopropane carboxylique et de la (4S) hydroxy 3-méthyl 2-(2-propényl) 2-cyclopenten-1-one, on obtient le produit recherché.

$$\alpha_D = + 27° \mp 2{,}5° \qquad c = 0{,}5\ \%\ CHCl_3$$

Exemple 30

(1R,cis) 2,2-diméthyl 3 [(E) 3-oxo 2-chloro éthoxy propényl] cyclopropane carboxylate de (S) α-cyano 3-phénoxy benzyle.

En opérant comme à l'exemple 1 à partir de l'acide (1R,cis) 2,2-diméthyl 3 [(E) 3-oxo 2-chloro 3-éthoxy propényl] cyclopropane carboxylique et de l'alcool (S) α-cyano 3-phénoxy benzyle, on obtient le produit recherché.

$$\alpha_D = + 19° \mp 2° \quad (c = 1\ \%\ CHCl_3).$$

L'acide (1R,cis) 2,2-diméthyl 3 [3-oxo 2-chloro 3-éthoxy propényl] cyclopropane carboxylique (isomères E et Z) a été préparé comme suit :

Stade A

Ethoxy carbonyl chloro méthylène triphényl phosphorane.

On prépare une solution à 2 °C environ de 4 g de chlore dans 80 $cm^3$ de chloroforme. On ajoute 20 g d'éthoxy carbonyl méthylène triphényl phosphorane dans 40 $cm^3$ de chloroforme. On laisse remonter le mélange réactionnel à la température ambiante, et amène à sec sous pression réduite. On obtient une huile que l'on dissout dans 70 $cm^3$ de chlorure de méthylène, lave avec une solution à 6,1 g de carbonate de sodium dans 40 $cm^3$ d'eau, puis à l'eau. On sèche et amène à sec. On obtient 18,9 g de produit recherché.

F = 116° ~ 118 °C

Stade B

Acide (1R,cis) 2,2-diméthyl 3 [3-oxo 2-chloro-3-éthoxy propényl] cyclopropane carboxylique (isomères E et Z).

On ajoute 6,9 g de la lactone de l'acide (1R, cis) 2,2-diméthyl 3-dihydroxy méthyl) cyclopropane-1-carboxylique dans 100 $cm^3$ de tétrahydrofuranne dans une solution renfermant 18,9 g du produit préparé au stade A dans 200 $cm^3$ de tétrahydrofuranne. On agite la solution obtenue pendant 6 heures et demi à la température ambiante, distille le solvant sous pression réduite. On obtient une huile que l'on reprend dans 50 $cm^3$ d'éther éthylique, agite à 0 °C, filtre, lave à l'éther le précipité obtenu et amène à sec le filtrat. On obtient 22,2 g de produit recherché, que l'on chromatographie sur silice en éluant par le mélange cyclohexane-acétate d'éthyle-acide acétique (75-25-1). On obtient ainsi :

A) d'une part 3,58 g du produit recherché, sous forme d'isomère E ; RMN $CDCl_3$ ppm

| | |
|---|---|
| 1,3 et 1,33 | H des méthyle en 2 du cyclopropane |
| 1,89-2,02 | H du carbone en 1 du cyclopropane |
| 2,85 à 3,05 | H du carbone en 3 du cyclopropane |
| 6,78-6,95 | H du carbone en 1 du radical propényl |

B) d'autre part, 2,34 g du produit Z correspondant.
RMN $CDCl_3$ ppm

| | |
|---|---|
| 1,33 et 1,36 | H des méthyle en 2 |
| 1,96-2,1 | H du carbone en 1 du cyclopropane |
| 2,23 à 2,53 | H du carbone en 3 du cyclopropane. |

Exemple 31

(1R,cis) 2,2-diméthyl 3[(Z) 2-chloro 3-oxo 3-éthoxy propényl] cyclopropane carboxylate de (S) α-cyano (3-phénoxy benzyle).

En opérant comme à l'exemple 1, à partir de l'acide (1R,cis) 1,1-diméthyl 3[(Z) 3-oxo-2-chloro 3-éthoxy propényl] cyclopropane carboxylique et de l'alcool (S) α-cyano 3-phénoxy benzylique on obtient le produit recherché.

$$\alpha_D = + 21°5 \mp 2°5 \quad (c = 0,3\ \%\ CHCl_3)$$

Exemple 32

(1R,cis) 2,2-diméthyl 3[(E) 3-oxo 2-chloro 3-propoxy propényl] cyclopropane carboxylate de (S) α-cyano (3-phénoxy-benzyle).

En opérant comme à l'exemple 1, à partir de l'acide (1R,cis) 2,2-diméthyl 3[(E)-oxo-2-chloro 3-propoxy propényl] cyclopropane carboxylique et de l'alcool (S) α-cyano 3-phénoxy benzylique, on obtient le produit recherché.

$\alpha_D = + 24°5 \mp 2°$    c = 0,4 % $CHCl_3$

L'acide (1R,cis) 2,2-diméthyl 3[(E) 3-oxo-2-chloro 3-propoxy propényl] cyclopropane carboxylique a été préparé comme suit :

Stade A

Propoxy carbonyl chloro méthylène triphényl phosphorane.

En opérant comme pour la préparation de l'acide (1R,cis) 2,2 diméthyl 3[(E) 3-oxo 2-chloro 3-éthoxy propényl] cyclopropane carboxylique, exemple 30, stade A, à partir du propoxy carbonyl méthylène triphényl phosphorane, on obtient le produit recherché.

Stade B

Acide (1R,cis) 2,2-diméthyl 3[(E) 3-oxo 2-chloro 3-propoxy propényl] cyclopropane carboxylique.

En opérant comme par la préparation de l'acide (1R,cis) 2,2 diméthyl 3[(E) 3-oxo 2-chloro 3-éthoxy propényl] cyclopropane carboxylique, exemple 30 (stade B) à partir du produit préparé au stade A, on obtient d'une part un produit recherché, d'autre part, l'isomère ΔZ correspondant.

Exemple 33

(1R,cis) 2,2-diméthyl 3[(Z) 3-oxo 2-chloro 3-propoxy propényl] cyclopropane carboxylate de (S) α-cyano] 3-phénoxy benzyle.

En opérant comme à l'exemple 1, à partir de l'acide (1R, cis) 2,2-diméthyl 3[(Z) 3-oxo 2-chloro 3-propoxy propényl] cyclopropane carboxylique et de l'alcool (S) α-cyano 3-phénoxy benzylique, on obtient le produit recherché.

$\alpha_D = + 22°5 \mp 2°$    c = 0,7 % $CHCl_3$

Exemple 34

(1R,cis) 2,2-diméthyl 3[(E) 3-oxo 3-terbutoxy 2-chloro propényl] cyclopropane carboxylate de (S) α-cyano 3-phénoxy benzyle.

En opérant comme à l'exemple 1, à partir de l'acide (1R,cis) 2,2-diméthyl 3[(E) 3-oxo 3-terbutoxy 2-chloropropényl] cyclopropane carboxylique et de l'alcool (S) α-cyano 3-phénoxy benzylique, on obtient le produit recherché.

$\alpha_D = + 30°5 \pm 2°$ (c = 0,7 % $CHCl_3$)

L'acide (1R,cis) 2,2-diméthyl 3-oxo 3-terbutoxy 2-chloro propényl) cyclopropane carboxylique (isomère E et Z) a été préparé comme suit :

Stade A

Terbutoxy carbonyl chloro méthylène triphényl phosphorane.

En opérant comme précédemment (préparation donnée à la suite de l'exemple 30, stade A), on a obtenu à partir de terbutoxy carbonyl méthylène triphényl phosphorane le produit recherché.
F≃ 160°C

Stade B

Acide ΔE + Acide ΔZ

En opérant à partir du produit préparé au stade A, comme il a été indiqué à la préparation donnée à la suite de l'exemple 30, stade B, on a obtenu d'une part l'acide (1R,cis) 2,2-diméthyl 3[(E) 3-oxo 3-terbutoxy 2-chloro propényl] cyclopropane carboxylique.
F = 65°C
et, d'autre part l'isomère (Z) correspondant F < 50 °C

Exemple 35

(1R,cis) 2,2-diméthyl 3-[(Z) 2-bromo 3-oxo 3-méthoxy propényl] cyclopropane carboxylate de (S) α-cyano 3-phénoxybenzyle.

En opérant comme à l'exemple 1 à partir d'acide (1R, cis) 2,2-diméthyl 3-[2-bromo 3-oxo 3-méthoxy (Z) propényl] cyclopropane carboxylique, et de l'alcool (S) α-cyano 3-phénoxy benzylique, on obtient le produit recherché.

$$\alpha_D = + 29°,5 \mp 2°,5 \qquad (c = 0,5\ \%\ CHCl_3)$$

L'acide (1R,cis) 2,2 diméthyl 3-[2-bromo 3-oxo 3-méthoxy (Z) propényl] cyclopropane carboxylique a été préparé comme suit :

Stade A

(1R,cis) 2,2 diméthyl 3-[1,2-(dibromo RS) 3-oxo 3-méthoxy propyl] cyclopropane carboxylate de terbutyle.

On ajoute 13,3 g de tribomure de pyridinium dans une solution renfermant 8,07 g de (1R,cis) 2,2-diméthyl 3-[2-méthoxy carbonyl (E) éthényl] cyclopropane carboxylate de terbutyle, préparé à partir de l'acide correspondant décrit dans la demande européenne publiée : n° 0018 894, et 50 $cm^3$ de diméthylsulfoxyde. On maintient le mélange réactionnel sous agitation pendant 3 h 30 mn et le déverse dans l'eau glacée. On extrait au chlorure de méthylène. On réunit les phases organiques, les sèche et concentre à sec sous pression réduite. On obtient 14,3 g d'huile que l'on chromatographie sur silice en éluant par le mélange hexane-acétate d'éthyle (9-1). On obtient 4,3 g de produit recherché.

Stade B

(1R,cis) 2,2-diméthyl 3-[2-bromo 3-oxo 3-méthoxy (Z) propényl] cyclopropane carboxylate de terbutyle.

On ajoute 4 $cm^3$ de triéthylamine dans une solution renfermant 40 $cm^3$ de benzène, et 4,2 g de (1R,cis) 2,2 diméthyl 3-[1,2 (dibromo RS) 3-oxo 3-méthoxy propyl] cyclopropane carboxylate de terbutyle. On agite pendant 6 heures et demi le mélange réactionnel à 22° ~ 24 °C. On dilue à l'éther, lave avec une solution de phosphate monosodique puis à l'eau. On sèche, concentre sous pression réduite à 40 °C. On obtient ainsi 3,3 g du produit recherché.

Stade C

Acide (1R,cis) 2,2-diméthyl 3-[2-bromo 3-oxo 3-méthoxy (Z) propényl] cyclopropane carboxylique.

On porte au reflux un mélange de 3,3 g du produit préparé au stade B, 30 $cm^3$ de toluène et 0,33 g d'acide paratoluène sulfonique monohydrate. On maintient le reflux jusqu'à la fin du dégagement gazeux. On refroidit à 20 °C, dilue à l'éther éthylique, lave à l'eau. On sèche et concentre sous pression réduite à 40 °C. On obtient ainsi 2,9 g du produit recherché.

Exemple 36

(1R,cis) 2,2 diméthyl 3-[(E + Z) 3-oxo 3-méthoxy 2-bromopropényl] cyclopropane carboxylate de (S) α-cyano 3-phénoxy benzyle.

En opérant comme à l'exemple 1 à partir de l'acide (1R, cis) 2,2-diméthyl 3-[2-bromo 3-oxo 3-méthoxy (E) propényl] cyclopropane carboxylique et de l'alcool (S) α-cyano 3-phénoxy benzylique, on obtient le produit recherché.

$$\alpha_D = + 9°5 \mp 2°5 \qquad (c = 0,3\ \%\ CHCl_3)$$

L'acide (1R,cis) 2,2-diméthyl 3-[2-bromo 3-oxo 3-méthoxy (E) propényl] cyclopropane carboxylique a été préparé comme suit :

Stade A

(1R,cis) 2,2 diméthyl 3-[2-bromo 3-oxo 3-méthoxy-(E) propényl] cyclopropane carboxylate de terbutyle.

On ajoute 100 $cm^3$ d'une solution de soude à 50 % dans un mélange renfermant 120 $cm^3$ de chlorure de méthylène, 6,7 g de (1R,cis) 2,2 diméthyl 3-[1,2-(dibromo RS) 3-oxo 3-méthoxy propyl] cyclopropane de

terbutyle et 120 mg de cétavlon (bromure de triméthyl cétyl ammonium). On agite le mélange réactionnel pendant 4 h. On dilue par ajout de 100 $cm^3$ de chlorure de méthylène et décante la phase organique que l'on réextrait par 100 $cm^3$ de chlorure de méthylène. On lave les phases organiques à l'acide chlorhydrique N jusqu'à pH acide puis à l'eau jusqu'à pH 7. On réunit les phases organiques, les sèche et concentre sous pression réduite à 40 °C. On obtient 5,3 g d'un produit que l'on chromatographie en éluant par le mélange : hexane-éther isopropylique (8-2). On obtient 3,5 g du produit recherché.

Stade B

L'acide (1R,cis) 2,2 diméthyl 3-[2-bromo 3-oxo 3-méthoxy (E) propényl] cyclopropane carboxylique a été préparé comme suit :

On porte au reflux une solution de 3,4 g de (1R,cis) 2,2-diméthyl 3-[2-bromo 3-oxo 3-méthoxy (E) propényl] cyclopropane de terbutyle, 30 $cm^3$ de toluène, et 0,35 g d'acide paratoluène sulfonique monohydraté. On maintient le reflux jusqu'à la fin du dégagement gazeux. On refroidit à 0 °C, on filtre, lave le précipité obtenu avec du toluène froid et concentre le filtrat à 40 °C sous pression réduite. On obtient 2,8 g du produit recherché.

Exemple 37

(1R,cis) 2,2-diméthyl 3-[(E) 2-bromo 3-oxo 3-terbutoxy propényl] cyclopropane carboxylate de (S) α-cyano 3-phénoxy benzyle.

En opérant comme à l'exemple 1, à partir de l'acide (1R,cis) 2,2-diméthyl 3[(E) 2-bromo 3-oxo 3-terbutoxy propényl] cyclopropane carboxylique et de l'alcool (S) α-cyano 3-phénoxy benzylique, on obtient le produit recherché.

$\alpha_D = +16°5 \mp 2°$ (c = 0,7 % $CHCl_3$)

L'acide 1R,cis 2,2 diméthyl 3[(E) 2-bromo 3-oxo 3-terbutoxy propényl] cyclopropane carboxylique a été préparé comme suit :

Stade A

Terbutoxy carbonyl bromométhylène triphényl phosphorane.

En opérant comme pour la préparation de l'acide (1R,cis) 2,2 diméthyl 3[(E) 3-oxo 2-chloro 3-éthoxy propényl] cyclopropane carboxylique (donné à l'exemple 30, stade A), à partir du dérivé terbutoxy carbonyl méthylène triphényl phosphorane et de brome, on obtient le produit recherché fondant à 190 °C.

Stade B

Acide (1R,cis) 2,2-diméthyl 3[(E) 2-bromo 3-oxo 3-terbutoxy propényl] cyclopropane carboxylique.

En opérant comme pour la préparation de l'acide (1R,cis) 2,2-diméthyl 3[(E) 3-oxo 2-chloro 3-éthoxy propényl] cyclopropane carboxylique (donné à l'exemple 30, stade B), à partir du produit préparé au stade A, on obtient d'une part le produit recherché fondant à 76 °C et d'autre part, l'isomère Z correspondant F 50 °C

Exemple 38

(1R,cis) 2,2-diméthyl 3[(Z) 2-bromo 3-oxo 3-terbutoxy propényl] cyclopropane carboxylate de (S) α-cyano 3-phénoxy benzyle.

En opérant comme à l'exemple 1, à partir de l'acide (1R,cis) 2,2-diméthyl 3[(Z) 2-bromo 3-oxo 3-terbutoxy propényl] cyclopropane carboxylique et de l'alcool (S) α-cyano 3-phénoxy benzyle, on obtient le produit recherché.

$\alpha_D = +16°5 \mp 2°$ C = 0,5 % $CHCl_3$

Exemple 39

(1R,cis) 2,2 diméthyl 3[(E) 3-oxo 2-bromo 3-éthoxy propényl] cyclopropane carboxylate de (S) α-cyano 3-phénoxy benzyle.

En opérant comme à l'exemple 1, à partir de l'acide (1R,cis) 2,2-diméthyl 3[(E) 3-oxo 2-bromo 3-éthoxy propényl] cyclopropane carboxylique et de l'alcool (S) α-cyano 3-phénoxy benzylique, on obtient le produit recherché.

$$\alpha_D = -70^\circ5 \mp 2^\circ \qquad c = 0,7\ \%\ (CHCl_3)$$

L'acide (1R,cis) 2,2-diméthyl 3[(E) 2-bromo 3-oxo 3-éthoxy propényl] cyclopropane carboxylique a été préparé comme suit :

Stade A

Ethoxy carbonyl bromo méthylène triphényl phosphorane.

Le produit a été préparé selon le procédé indiqué pour l'acide (1R,cis) 2,2 diméthyl 3[(E) 3-oxo 2-chloro 3-éthoxy propényl] cyclopropane carboxylique, à l'exemple 30, stade A, à partir du dérivé éthoxy carbonyl méthylène triphényl phosphorane et de Brome. On a obtenu ainsi le produit recherché fondant à 150 °C.

Stade B

Acide (1R,cis) 2,2 diméthyl 3[(E) 2-bromo 3-oxo 3-éthoxy propényl] cyclopropane carboxylique.

Le produit a été préparé selon le procédé indiqué pour la préparation de l'acide chloré correspondant, à partir du produit préparé au stade A.

On a isolé ainsi d'une part, le produit recherché, d'autre part, le produit ΔZ correspondant.

Exemple 40

(1R,cis) 2,2 diméthyl 3-[(E) 2-fluoro 3-oxo 3-éthoxy propényl] cyclopropane carboxylate de (RS) α-cyano 6-phénoxy 2-pyridyl méthyle.

En opérant comme à l'exemple 1, à partir de l'acide et de l'alcool correspondants, on obtient le produit recherché.

$$\alpha_D = +35^\circ \mp 4^\circ \qquad (c = 0,3\ \%\ CHCl_3)$$

Exemple 41

(1R,cis) 2,2 diméthyl 3-[(E) 2-fluoro 3-oxo 3-éthoxy propényl] cyclopropane carboxylate de 3-(2-propynyl) 2,5-dioxoimidazolidinyl méthyle.

En opérant comme à l'exemple 1 à partir de l'acide et de l'alcool correspondants, on obtient le produit recherché.

$$\alpha_D = +12^\circ \mp 2^\circ \qquad c = 0,5\ \%\ CHCl_3$$

Exemple 42

(1R,cis) 2,2-diméthyl 3[(E) 2-fluoro 3-oxo 3-éthoxy propényl] cyclopropane carboxylate de (S) 2-méthyl 3-allyl 4-oxo 2-cyclopenten-1-yle.

En opérant comme à l'exemple 1 à partir de l'acide et de l'alcool correspondants, on obtient le produit recherché.

$$\alpha_D = +41^\circ5 \mp 2^\circ5 \qquad c = 0,5\ \%\ CHCl_3$$

Exemple 43

Préparation d'un concentré soluble

On effectue un mélange homogène de :

| | |
|---|---|
| Produit de l'exemple 1 | 0,25 g |
| Butoxyde de pipéronyle | 1,00 g |
| Tween 80 | 0,25 g |

| | |
|---|---|
| Topanol A | 0,1 g |
| Eau | 98,4 g |

Exemple 44

Préparation d'un concentré émulsifiable.

On mélange intimement :

| | |
|---|---|
| Produit de l'exemple 11 | 0,015 g |
| Butoxyde de pipéronyle | 0,5 g |
| Topanol A | 0,1 g |
| Tween 80 | 3,5 g |
| Xylène | 95,885 g |

Exemple 45

Préparation d'un concentré émulsifiable.

On effectue un mélange homogène de :

| | |
|---|---|
| Produit de l'exemple 21 | 1,5 g |
| Tween 80 | 20,00 g |
| Topanol A | 0,1 g |
| Xylène | 78,4 g |

Exemple 46

Préparation d'une composition fumigène.

On mélange d'une façon homogène :

| | |
|---|---|
| Produit de l'exemple 1 | 0,25 g |
| Poudre de tabu | 25,00 g |
| Poudre de feuille de cèdre | 40,00 g |
| Poudre de bois de pin | 33,75 g |
| Vert brillant | 0,5 g |
| p-nitrophénol | 0,5 g |

Exemple 47

Exemple de composition pharmaceutique.

On a préparé des solutions répondant à la formule suivante :

| | |
|---|---|
| Composé de l'exemple 11 | 5,00 g |
| Butoxyde de pipéronyle | 25,00 g |
| Polysorbate 80 | 10,00 g |
| Triton X 100 | 25,00 g |
| Acétate de Tocophérol | 1 g |
| Alcool éthylique q.s.p. | 100 ml |

On obtient ainsi une solution que l'on dilue dans 5 litres d'eau au moment de l'emploi.

Exemple 48

Exemple de composition pharmaceutique.

On a préparé une solution répondant à la formule suivante :

| | |
|---|---|
| Composé de l'exemple 11 | 0,5 g |
| Butoxyde de pipéronyle | 2,5 g |
| Polysorbate 80 | 10 g |
| Triton X 100 | 25 g |
| Acétate de Tocophérol | 1 g |
| Alcool éthylique q.s.p. | 100 $cm^3$ |

On obtient ainsi une solution que l'on dilue dans 5 litres d'eau au moment de l'emploi.

Exemple 49

Exemple de compositions pharmaceutiques.

On prépare des capsules contenant 1 g de produit de l'exemple 1.

Exemple 50

Exemple d'aliment composé pour animaux.

On utilise comme aliment équilibré de base un aliment comportant du maïs, de la luzerne déshydratée, de la paille de blé, du tourteau de palmiste mélassé, de l'urée, un condiment minéral vitaminé.

Cet aliment contient au minimum 11 % de matières protéiques brutes (dont 2,8 % apportés par l'urée), 2,5 % de matières grasses et au maximum 15 % de matières cellulosiques, 6 % de matières minérales et 13 % d'humidité.

L'aliment utilisé correspond à 82 unités fourragères pour 100 kilos et contient pour 100 kilos 910 000 U.I. de vitamine A, 91 000 U.I. de vitamines $D_3$, 150 mg de vitamine E, 150 mg de vitamine C.

On incorpore à cet aliment 0,3 kg de composé de l'exemple 3 pour 100 kg d'aliment au total.

Exemple 51

Exemple d'aliment composé pour animaux.

On utilise le même aliment équilibré, de base qu'à l'exemple 50. On incorpore à cet aliment, 0,04 kg de composé de l'exemple 1 pour 100 kg d'aliment au total.

Etude insecticide des composés de l'invention.

On utilise dans cette étude, le (1R,cis) 2,2-diméthyl 3(E) [2-chloro 2-méthoxy carbonyl éthényl] cyclopropane-1-carboxylate de (S) α-cyano 3-phénoxy benzyle (composé A), le (1R,cis) 2,2-diméthyl 3(E) (2-bromo 2-propoxy carbonyl éthényl) cyclopropane-1-carboxylate de (S) α-cyano 3-phénoxy benzyle (composé B), le (1R,cis) 2,2-diméthyl 3(Z) (2-bromo 2-propoxy carbonyl éthényl) cyclopropane-1-carboxylate de (S) α-cyano 3-phénoxy benzyle (composé C) et le (1R,cis) 2,2-diméthyl 3-[(E) 2-fluoro 2-éthoxy carbonyl éthényl] cyclopropane carboxylate de (S) α-cyano 3-phénoxy benzyle (composé D).

A. Etude de l'effet d'abattage sur mouche domestique.

Les insectes tests sont des mouches domestiques femelles âgées de 4 jours. On opère par pulvérisation directe à la concentration de 0,25 g/l en chambre de Kearns et March en utilisant comme solvant un mélange d'acétone (5 %) et d'Isopar L (solvant pétrolier) (quantité de solvant utilisée 2 ml en une seconde). On utilise 50 insectes par traitement. on effectue les contrôles toutes les minutes jusqu'à 10 minutes, puis à 15 minutes et l'on détermine le KT 50 par les méthodes habituelles.

Les résultats expérimentaux obtenus sont résumés dans le tableau suivant :

| Composés | $KT_{50}$ en mn |
|---|---|
| Composé (A) | 3,6 |
| Composé (B) | 4,5 |
| Composé (C) | 4,5 |
| Composé (D) | 2,1 |

B. Etude de l'effet létal des composés de l'invention sur divers insectes.

a) Etude de l'effet létal sur mouche domestique.

Les insectes tests sont des mouches domestiques femelles âgées de 4 à 5 jours. On opère par application topique de 1 μl de solution acétonique sur le thorax dorsal des insectes à l'aide du micro manipulateur d'Arnold. On utilise 50 individus par traitement. On effectue le contrôle de mortalité vingt-quatre heures après traitement.

Les résultats obtenus exprimés en DL 50 ou dose (en nanogrammes par individu nécessaire pour tuer 50 % des insectes, sont les suivants :

| Composés | DL en ng/ insecte |
|---|---|
| Composé (A) | 11,1 |
| Composé (B) | 1,0 |

b) Etude de l'effet létal sur blatte.

Les tests sont effectués par contact sur film de verre, par dépôt à la pipette, de solutions acétoniques de différentes concentrations sur fond de boîte de Petri en verre dont les bords ont été préalablement talqués afin d'éviter la fuite des insectes. On détermine la concentration létale 50 (CL 50).

Les résultats expérimentaux obtenus sont résumés dans le tableau suivant :

| Composés | CL 50 en mg/m2 |
|---|---|
| Composé (A) | 1,4 |
| Composé (B) | 0,40 |

c) Etude de l'effet létal sur larves de Spodoptera Littoralis.

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24 °C et 65 % d'humidité relative. Après traitement les individus sont placés sur un milieu nutritif artificiel (milieu de Poitout).

On effectue le contrôle des mortalités 48 heures après traitement.

Les résultats expérimentaux obtenus sont résumés dans le tableau suivant :

| Composés | DL 50 en ng par insecte |
|---|---|
| Composé (A) | 6,7 |
| Composé (B) | 3,2 |
| Composé (D) | 1,0 |

d) Etude de l'effet létal sur Epilachna varivestris.

Les essais sont effectués par application topique de manière analogue à celle utilisée pour les larves de Spodoptera. On utilise des larves de l'avant dernier stade larvaire et après traitement les larves sont alimentées par des plants de haricots. On effectue le contrôle de mortalité 72 heures après traitement.

Les résultats sont résumés dans le tableau suivant :

| Composés | DL 50 en ng/ insecte |
|---|---|
| Composé (A) | 17,7 |
| Composé (B) | 7,4 |
| Composé (D) | 0,85 |

e) Etude de l'effet létal sur Aphis Cracivora.

On utilise des adultes après 7 jours et l'on emploie 10 Aphis par concentration utilisée. On utilise une méthode de contact-injection. On effectue le traitement au pistolet de Fisher d'une feuille de fève que l'on dépose dans une boîte de Petri en matière plastique sur une rondelle de papier humidifiée. Le traitement est effectué à l'aide de 2 ml de solution acétonique de produit à tester (1 ml par face de feuille). L'infestation par insecte est effectuée après séchage de la feuille. On maintient les insectes en contact avec la feuille pendant une heure. On place les insectes sur des feuilles non traitées et contrôle la mortalité au bout de 24 heures.

Les résultats expérimentaux obtenus sont résumés dans le tableau suivant :

| Composé | DL 50 en ng/ insecte |
|---|---|
| Composé (B) | 6,4 |

f) Conclusion.

Dans les tests décrits aux paragraphes a, b, c, d et e, les composés A et B montrent également une activité insecticide intéressante.

g) Conclusion générale.

Les composés de l'invention sont doués d'une intéressante activité insecticide dans les tests précédemment décrits.

B. Etude acaricide des composés de l'invention.

On utilise des plants de haricot comportant 2 feuilles infestées de 25 femelles de Tétranychus Urticae par feuille et mis sous bonette aérée sous plafond lumineux en lumière constante. Les plants sont traités au pistolet Fisher : 4 ml de solution toxique par plant d'un mélange à volume égal d'eau et d'acétone. On laisse sécher pendant 12 heures puis on procède à l'infestation. Les contrôles de mortalité sont effectués 80 heures après. La dose utilisée dans chaque test est de 5 g de produit par hl.

Les composés A, B et C présentent, dans ce test, une bonne activité.

**Revendications** (pour les Etats contractants : BE, CH, DE, FR, GB, IT, LI, LU, NL, SE)

1. Sous toutes les formes isomères possibles, ou sous forme de mélange d'isomères, les composés de formule I

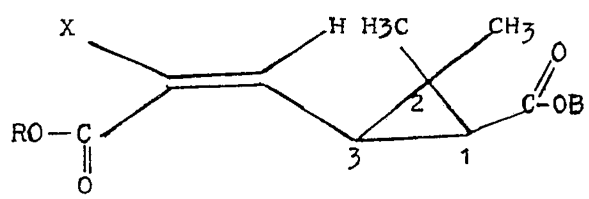

(I)

dans laquelle la structure de la copule acide cyclopropanique est 1R cis et la géométrie de la double liaison est E et dans laquelle R représente ou bien un radical alcoyle linéaire, ramifié ou cyclique, saturé ou insaturé, renfermant de 1 à 8 atomes de carbone, éventuellement substitué par un ou plusieurs groupements fonctionnels identiques ou différents, ou bien un groupement aryle renfermant de 6 à 14 atomes de carbone, éventuellement substitué par un ou plusieurs groupements fonctionnels identiques ou différents, ou bien un radical hétérocyclique éventuellement substitué par ou plusieurs groupements fonctionnels identiques ou différents, X représente un atome d'halogène et B représente

soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxy et les atomes d'halogène,

soit un groupement

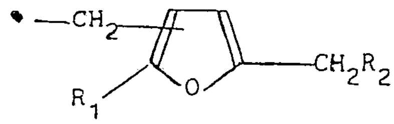

dans lequel le substituant $R_1$ représente un atome d'hydrogène ou un radical méthyle et le substituant $R_2$ un aryle monocyclique ou un groupement $-CH_2-C\equiv CH$ et notamment un groupement 5-benzyl 3-furyl méthyle,

soit un groupement

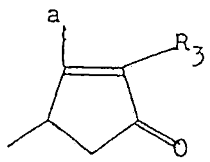

dans lequel a représente un atome d'hydrogène ou un radical méthyle et $R_3$ représente un radical organique aliphatique comportant de 2 à 6 atomes de carbones et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$,

soit un groupement

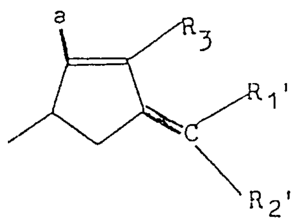

dans lequel a représente un atome d'hydrogène ou un radical méthyle, $R_3$ conserve la même signification que précédemment, $R_1'$ et $R_2'$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

soit un groupement

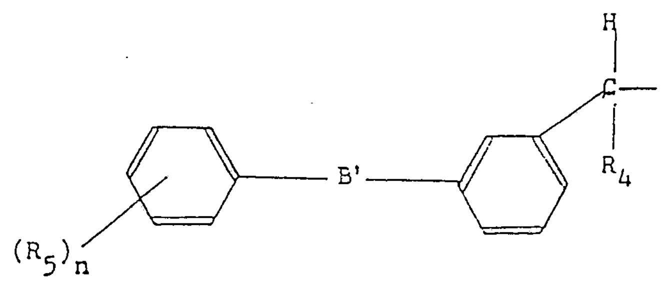

dans lequel B' représente un atome d'oxygène ou de soufre, un groupement

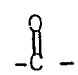

ou $-CH_2-$ ou un groupement sulfoxyde ou un groupement sulfone et $R_4$ représente un atome d'hydrogène, un radical $-C\equiv N$, un radical méthyle, un radical $-CONH_2$, un radical $-CSNH_2$ ou un radical $-C\equiv CH$, $R_5$ représente un atome d'halogène ou un radical méthyle et n représente un nombre égal à 0, 1 ou 2, et notamment le groupement 3-phénoxy benzyle, α-cyano 3-phénoxy benzyle, α-éthynyl 3-phénoxy benzyle, 3-benzoyl benzyle, 1-(3-phénoxy phényl) éthyle ou α-thioamido 3-phénoxy benzyle,

soit un groupement

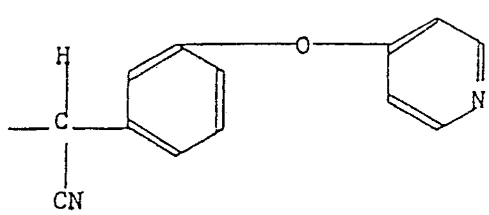

soit un groupement

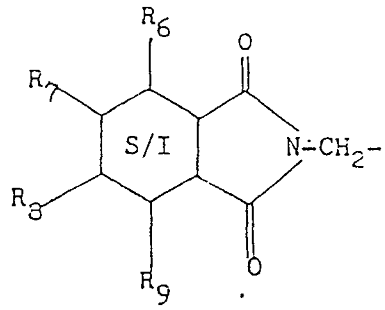

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

soit un groupement

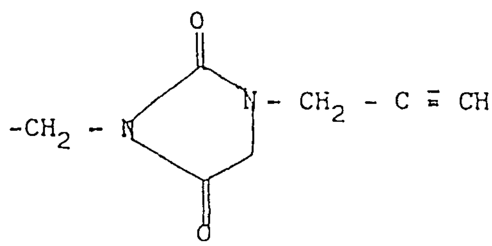

soit un groupement

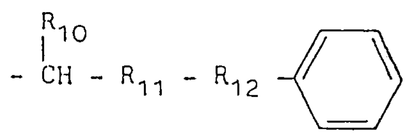

dans lequel $R_{10}$ représente un atome d'hydrogène ou un radical CN, $R_{12}$ représente un radical $—CH_2—$ ou un atome d'oxygène, $R_{11}$ représente un radical thiazolyle ou thiadiazolyle dont la liaison avec

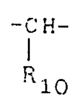

peut se trouver à l'une quelconque des positions disponibles, $R_{12}$ étant lié à $R_{11}$ par l'atome de carbone compris entre l'atome de soufre et un atome d'azote,

soit un groupement

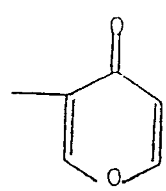

soit un groupement

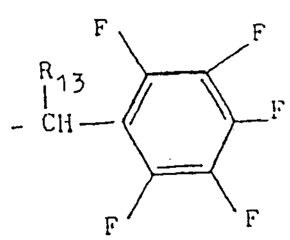

dans lequel $R_{13}$ représente un atome d'hydrogène ou un radical CN, soit un groupement

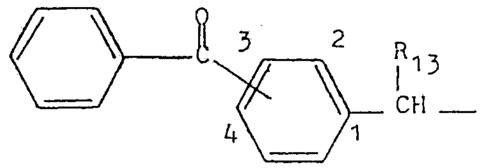

dans lequel $R_{13}$ est défini comme ci-dessus, et le radical benzoyle est en position 3 ou 4, soit un groupement

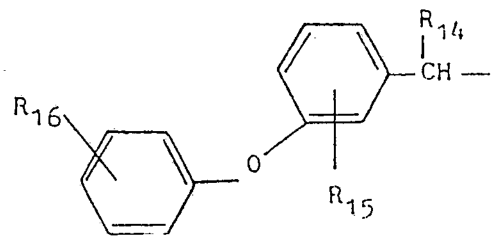

dans lequel $R_{14}$ représente un atome d'hydrogène, un radical méthyle, éthynyle ou cyano et $R_{15}$ et $R_{16}$, différents, représentent un atome d'hydrogène, de fluor ou de brome, soit un groupement

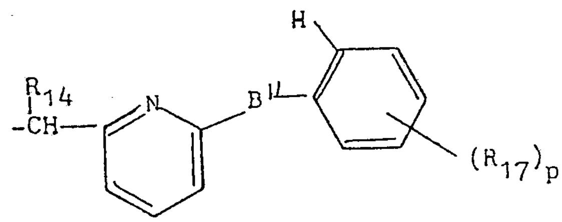

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoyl sulfonyl renfermant de 1 à 4 atomes de carbone, trifluorométhyl, 3,4-méthylène dioxy, chloro, fluoro ou bromo, p représente un nombre égal à 0, 1 ou 2 et B'' représente un atome d'oxygène ou un atome de soufre.

2. Les composés de formule I tels que définis à la revendication 1, pour lequels X représente un atome de fluor.

3. Les composés de formule I tels que définis à l'une quelconque des revendications 1 ou 2, pour lesquels R représente un radical alcoyle linéaire, ramifié ou cyclique renfermant de 1 à 8 atomes de carbone.

4. Les composés de formule I tels que définis à la revendication 3, pour lesquels R représente un radical éthyle.

5. Les composés de formule I tels que définis à la revendication 3, pour lesquels R représente un radical terbutyle.

6. Les composés de formule I tels que définis à la revendication 3, pour lesquels R représente un radical cyclopropyle ou cyclopropylméthyle.

7. Les composés de formule I tels que définis à l'une quelconque des revendications 1 ou 2, pour lesquels R représente un radical alcoyle linéaire ou ramifié, renfermant de 1 à 8 atomes de carbone, substitué par un ou plusieurs atomes d'halogène.

8. Les composés de formule I tels que définis à la revendication 7, pour lesquels R représente un radical alcoyle linéaire renfermant de 1 à 8 atomes de carbone substitué par un ou plusieurs atomes de fluor.

9. Les composés de formule I tels que définis à l'une quelconque des revendications 1 ou 2, pour lesquels R représente un radical $(CH_2)_mO(CH_2)_n{-}CH_3$ dans lequel m représente un nombre entier pouvant varier de 1 à 8 et n représente un nombre entier pouvant varier de 0 à 8, et notamment le radical $-CH_2OCH_3$.

10. Les composés de formule I tels que définis à l'une quelconque des revendications 1 à 9, répondant à la formule $I_A$.

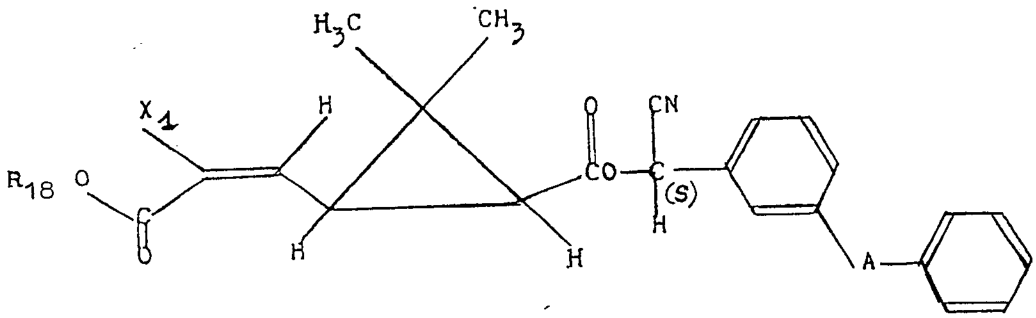

($I_A$)

dans laquelle la structure de la copule acide cyclopropanique est 1R cis et la géométrie de la double liaison est E et dans laquelle A représente un atome d'oxygène, un groupement méthylène, un groupement carbonyle, un atome de soufre, un groupement sulfoxyde ou un groupement sulfone, $R_{18}$ représente un radical alcoyle linéaire ou ramifié renfermant de 1 à 8 atomes de carbone, $X_1$ représente un atome de fluor, de chlore ou de brome.

11. Les composés de formule ($I_A$) tels que définis à la revendications 10, pour lesquels A représente un atome d'oxygène.

12. Le (1R,cis) 2,2-diméthyl 3(E)-[2-fluoro 2-(éthoxy carbonyl)éthényl] cyclopropane-1-carboxylate de (S) α-cyano 3-phénoxy benzyle.

13. Les composés de formule I tels que définis à l'une quelconque des revendications 1 à 9, pour lesquels B représente un radical

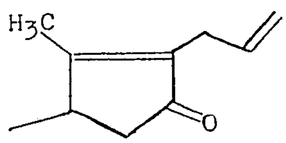

14. Les composés de formule I tels que définis à l'une quelconque des revendications 1 à 9, pour lesquels B représente un radical

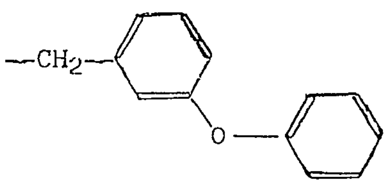

15. Les composés de formule I tels que définis à l'une quelconque des revendications 1 à 9, pour lesquels B représente un radical

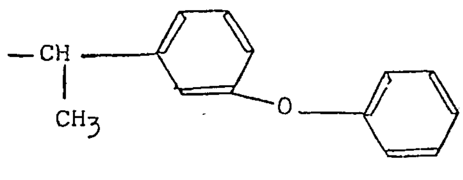

16. Les composés de formule I tels que définis à l'une quelconque des revendications 1 à 9, pour lesquels B représente un radical

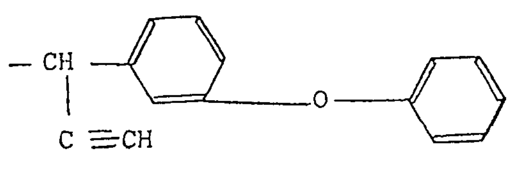

17. Les composés de formule I tels que définis à l'une quelconque des revendications 1 à 9, pour lesquels B représente un radical

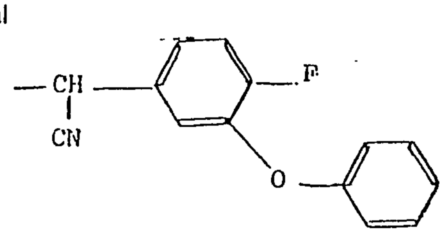

18. Application des composés de formule I tels que définis à l'une quelconque des revendications 1 à 9, 13 à 17, à la lutte contre les parasites des végétaux, les parasites des locaux.

19. Application des composés de formule $I_A$ tels que définis à l'une quelconque des revendications 10 à 12, à la lutte contre les parasites des végétaux, les parasites des locaux.

20. Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 9 et 13 à 17.

21. Les compositions destinées à la lutte contre les parasites des végétaux et les parasites des locaux et les parasites des animaux à sang chaud, charactérisées en ce qu'elles renferment comme principe actif au moins un composé défini à l'une quelconque des revendications 10 à 12.

22. Les compositions insecticides, caractérisées en ce qu'elles renferment comme principe actif au moins un composé défini à l'une quelconque des revendications 1 à 9 et 13 à 17.

23. Les compositions insecticides, caractérisées en ce qu'elles renferment comme principe actif au moins un composé défini à l'une quelconque des revendications 10 à 12.

24. Les compositions acaricides, caractérisées en ce qu'elles renferment comme principe actif au moins un composé défini à l'une quelconque des revendications 1 à 9 et 13 à 17.

25. Les compositions acaricides, caractérisées en ce qu'elles renferment comme principe actif au moins un composé défini à l'une quelconque des revendications 10 à 12.

26. Les compositions destinées à l'alimentation animale, caractérisées en ce qu'elles renferment comme principe actif un au moins des composés définis à l'une quelconque des revendications 1 à 9 et 13 à 17, associé à un aliment pour animal.

27. Les compositions destinées à l'alimentation animale, caractérisées en ce qu'elles renferment comme principe actif un au moins des composés définis à l'une quelconque des revendications 10 à 12, associé à un aliment pour animal.

28. Associations douées d'activité insecticide, acaricide ou nématicide, caractérisées en ce qu'elles contiennent comme matière active, d'une part, un au moins des composés de formule générale (I), et, d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane-1-carboxyliques, dans lesquels « halo » représente un atome de fluor, de chlore ou de brome, étant entendu que les composés I peuvent exister sous toutes leurs formes stéréoisomères possibles de même que les copules acides et alcools des esters pyréthrinoïdes ci-dessus.

29. A titre de médicaments, les composés de formule I tels que définis à l'une quelconque des revendications 1 à 17.

30. Les compositions pharmaceutiques renfermant, à titre de principe actif, l'un au moins des médicaments définis à la revendication 29.

31. Procédé de préparation des composés de formule I tels que définis à l'une quelconque des revendications 1 à 9 et 13 à 17, caractérisé en ce que l'on fait réagir un acide de formule (II) :

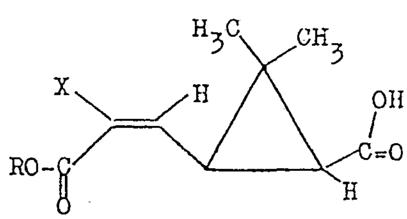

(II)

dans laquelle la structure de la copule cyclopropanique est 1R cis et la géométrie de la double liaison est E et dans laquelle X et R conservent la même signification que précédemment, ou un dérivé fonctionnel de cet acide avec un alcool de formule (III) :

B—OH (III)

B conservant la même signification que précédemment ou un dérivé fonctionnel de cet alcool, pour obtenir le composé de formule (I) correspondant, que l'on soumet, si désiré, à l'action d'un agent de clivage sélectif du groupement $CO_2R$ pour obtenir le composé de formule (IV) :

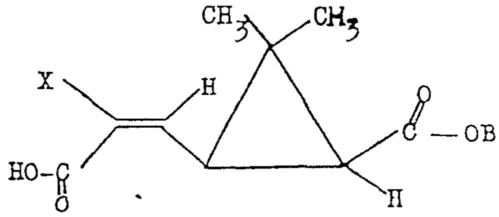

(IV)

dans laquelle la structure de la copule cyclopropanique est 1R cis et la géométrie de la double liaison est E et dans laquelle B conserve la même signification que précédemment, puis soumet, cet acide de formule (IV) ou un dérivé fonctionnel de cet acide, à l'action d'un alcool de formule R—OH dans laquelle R conserve sa signification précédente, pour obtenir le composé de formule (I) correspondant.

32. Procédé de préparation des composés de formule $I_A$, tels que définis à l'une quelconque des revendications 10 à 12, caractérisé en ce que l'on fait réagir un acide formule ($II_A$) :

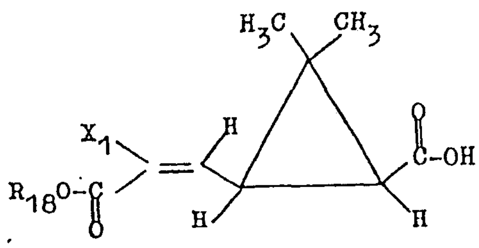

($II_A$)

dans laquelle la structure de la copule cyclopropanique est 1R cis et la géométrie de la double liaison est E et dans laquelle $X_1$ et $R_{18}$ conservent la même signification que dans la revendication 13, ou un dérivé fonctionnel de cet acide ($II_A$), avec un alcool de formule ($III_A$) :

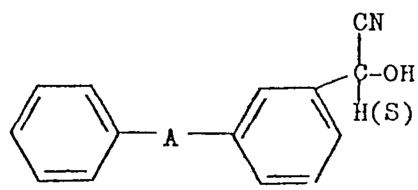

($III_A$)

dans laquelle A conserve la même signification que dans la revendication 10, ou un dérivé fonctionnel de l'alcool de formule ($III_A$), sépare pour obtenir le composé de formule ($I_A$) correspondant.

33. Procédé selon la revendication 31 ou 32, caractérisé en ce que les acides de formule (II) ou ($II_A$) dans laquelle R ou $R_{18}$ représente un radical alcoyle renfermant de 1 à 5 atomes de carbone, sont préparés en faisant réagir selon la réaction de Wittig, en présence d'une base forte, un aldéhyde cis sous forme de lactone de formule (V) :

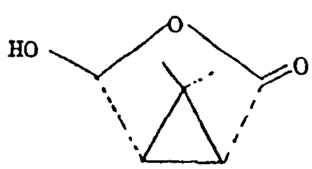

(V)

avec un phosphorane de formule (VII) :

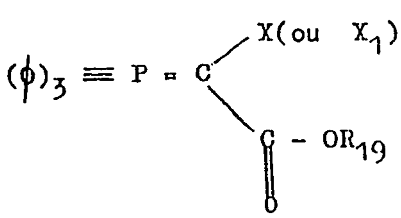

(VII)

formule dans laquelle $R_{19}$ représente un radical alcoyle comportant de 1 à 5 atomes de carbone et X et $X_1$ conservent leurs significations précédentes, ou avec un phosphonate de formule (VIII) :

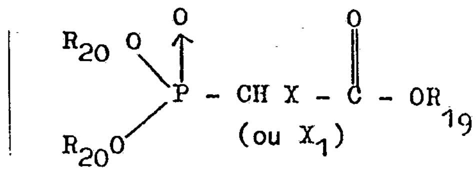

(VIII)

formule dans laquelle $R_{19}$, X et $X_1$ conservent les significations précitées et $R_{20}$ représente un radical alcoyle comportant de 1 à 6 atomes de carbone.

34. A titre de produits chimiques nouveaux, les acides de formules II et $II_A$ tels que définis aux revendications 31 et 32, ainsi que les dérivés fonctionnels de ces acides.

35. A titre de produits chimiques nouveaux, les acides de formule IV tels que définis à la revendication 31, ainsi que les dérivés fonctionnels de ces acides.

36. A titre de produits intermédiaires nécessaires à la mise en œuvre du procédé des revendications 31 et 32 les composés de formules II, $II_A$ et IV, tels que définis aux revendications 31 et 32.

**Revendications** (pour l'Etat contractant AT)

1. Procédé pour préparer sous toutes les formes isomères possibles, ou sous forme de mélanges d'isomères, les composés de formule I :

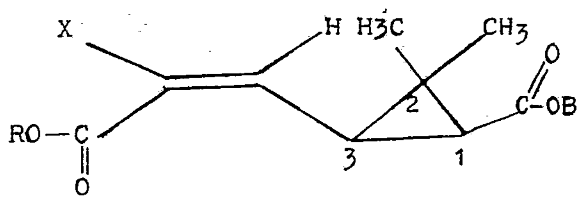

(I)

dans laquelle R représente ou bien un radical alcoyle linéaire, ramifié ou cyclique, saturé ou insaturé, renfermant de 1 à 8 atomes de carbone, éventuellement substitué par un ou plusieurs groupements fonctionnels identiques ou différents, ou bien un groupement aryle renfermant de 6 à 14 atomes de carbone, éventuellement substitué par un ou plusieurs groupements fonctionnels identiques ou différents, ou bien un radical hétérocyclique éventuellement substitué par un ou plusieurs groupements fonctionnels identiques ou différents, B représente ou bien un radical alcoyle linéaire, ramifié ou cyclique, saturé ou insaturé, renfermant de 1 à 18 atomes de carbone, ou bien le reste d'un alcool utilisé dans la synthèse des esters de la série pyréthrinoïde et X représente un atome d'halogène, la double liaison éthylénique ayant la géométrie Z ou E, caractérisé en ce que l'on fait réagir un acide de formule (II) :

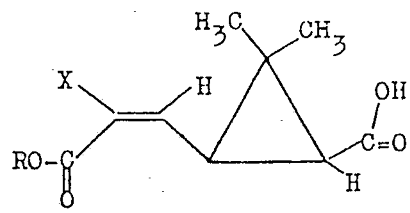

(II)

dans laquelle X et R conservent la même signification que précédemment, cet acide se trouvant sous forme de mélanges d'isomères E et Z ou sous forme d'isomère E ou Z, le cycle cyclopropanique substitué pouvant être sous toutes ses formes stéréoisomères, ou sous forme de mélange de stéréoisomères ou un dérivé fonctionnel de cet acide, avec un alcool de formule (III) :

B—OH (III)

B conservant la même signification que précédemment ou un dérivé fonctionnel de cet alcool, pour obtenir le composé de formule (I) correspondant, que l'on soumet, si désiré, à l'action d'un agent de clivage sélectif du groupement $CO_2R$ pour obtenir le composé de formule (IV) :

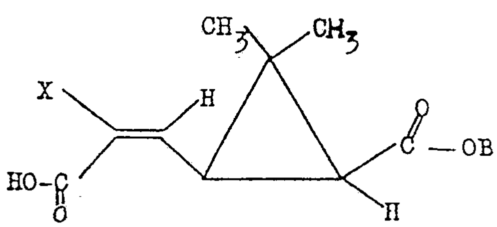

(IV)

dans laquelle B conserve la même signification que précédemment, puis soumet, cet acide de formule (IV) ou un dérivé fonctionnel de cet acide, à l'action d'un alcool de formule R—OH dans laquelle R conserve sa signification précédente, pour obtenir le composé de formule (I) correspondant.

2. Procédé selon la revendication 1, caractérisé en ce que l'on utilise au départ un acide de formule II de structure 1R, cis ou 1R, trans.

3. Procédé selon la revendication 1 ou 2, caractérisé en ce que l'on utilise au départ un acide de formule II, pour lequel la double liaison a la géométrie E.

4. Procédé selon l'une quelconque des revendications 1 à 3, caractérisé en ce que l'on utilise au départ un acide de formule II, pour lequel X représente un atome de fluor.

5. Procédé selon l'une quelconque des revendications 1 à 4, caractérisé en ce que l'on utilise au départ un acide de formule II, ou un alcool de formule R—OH, pour lesquels R représente un radical alcoyle, linéaire, ramifié ou cyclique renfermant de 1 à 8 atomes de carbone.

6. Procédé selon la revendication 5, caractérisé en ce que R est un radical éthyle ou terbutyle.

7. Procédé selon l'une quelconque des revendications 1 à 4, caractérisé en ce que l'on utilise au départ un acide de formule II ou un alcool de formule R—OH, pour lesquels R représente un radical cyclopropyle ou cyclopropylméthyle.

8. Procédé selon l'une quelconque des revendications 1 à 4, caractérisé en ce que l'on utilise au départ un acide de formule II ou un alcool de formule R—OH, pour lesquels R représente un radical alcoyle, linéaire ou ramifié, renfermant de 1 à 8 atomes de carbone, substitué par un ou plusieurs atomes d'halogène.

9. Procédé selon l'une quelconque des revendications 1 à 4, caractérisé en ce que l'on utilise au départ un acide de formule II ou un alcool de formule R—OH, pour lesquels R représente un radical alcoyle, linéaire renfermant de 1 à 8 atomes de carbone, substitué par un ou plusieurs atomes de fluor.

10. Procédé selon l'une quelconque des revendications 1 à 5, caractérisé en ce que l'on utilise au départ un acide de formule II, ou un alcool de formule R—OH, pour lesquels R représente un radical $(CH_2)_mO(CH_2)_n-CH_3$ dans lequel m représente un nombre entier pouvant varier de 1 à 8 et n représente un nombre entier pouvant varier de 0 à 8, et notamment le radical : $-CH_2OCH_3$.

11. Procédé selon l'une quelconque des revendications 1 à 10, caractérisé en ce que l'on utilise au départ un alcool de formule B—OH, pour lequel B représente :

soit un radical alcoyle renfermant de 1 à 18 atomes de carbone,

soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxy et les atomes d'halogène,

soit un groupement

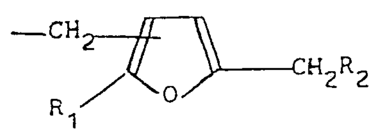

dans lequel le substituant $R_1$ représente un atome d'hydrogène ou un radical méthyle et le substituant $R_2$ un aryle monocyclique ou un groupement $-CH_2-C\equiv CH$ et notamment un groupement 5-benzyl 3-furyl méthyle,

soit un groupement

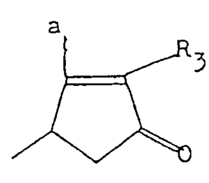

dans lequel a représente un atome d'hydrogène ou un radical méthyle et $R_3$ représente un radical organique aliphatique comportant de 2 à 6 atomes de carbone et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$,

soit un groupement

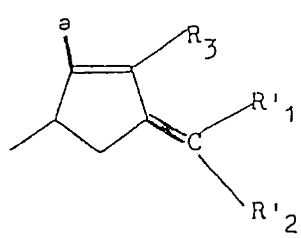

dans lequel a représente un atome d'hydrogène ou un radical méthyle, $R_3$ conserve la même signification que précédemment, $R_1'$ et $R_2'$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

soit un groupement

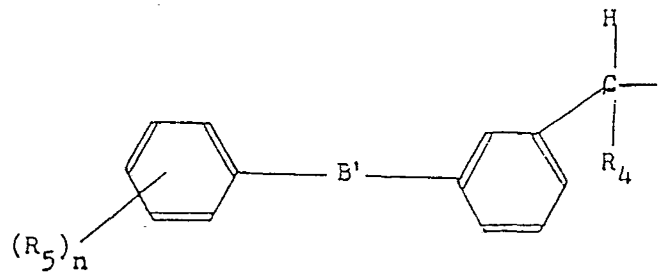

dans lequel B' représente un atome d'oxygène ou de soufre, un groupement

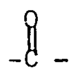

ou $—CH_2—$ ou un groupement sulfoxyde ou un groupement sulfone et $R_4$ représente un atome d'hydrogène, un radical —C≡N, un radical méthyle, un radical $—CONH_2$, un radical $—CSNH_2$ ou un radical —C≡CH, $R_5$ représente un atome d'halogène ou un radical méthyle et n représente un nombre égal à 0, 1 ou 2, et notamment le groupement 3-phénoxy benzyle, α-cyano 3-phénoxy benzyle, α-éthynyl 3-phénoxy benzyle, 3-benzoyl benzyle, 1-(3-phénoxy phényl) éthyle ou α-thioamido 3-phénoxy benzyle,

soit un groupement

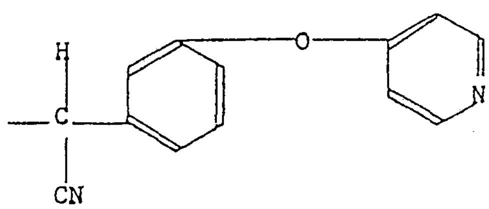

soit un groupement

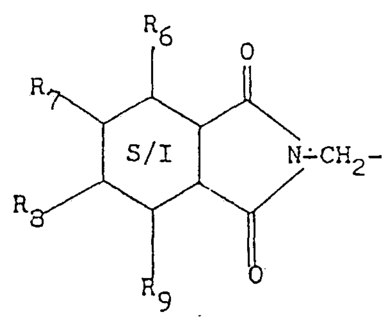

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

soit un groupement

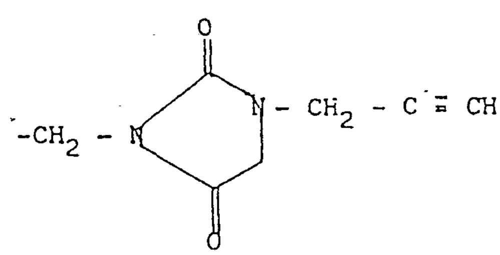

soit un groupement

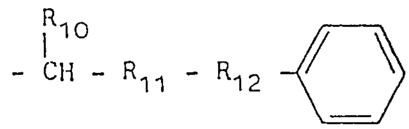

dans lequel $R_{10}$ représente un atome d'hydrogène ou un radical CN, $R_{12}$ représente un radical $-CH_2-$ ou un atome d'oxygène, $R_{11}$ représente un radical thiazolyle ou thiadiazolyle dont la liaison avec

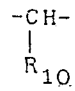

peut se trouver à l'une quelconque des positions disponibles, $R_{12}$ étant lié à $R_{11}$ par l'atome de carbone compris entre l'atome de soufre et un atome d'azote,

soit un groupement

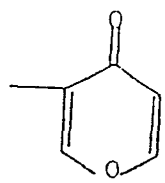

soit un groupement

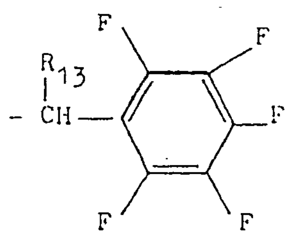

dans lequel $R_{13}$ représente un atome d'hydrogène ou un radical CN,

soit un groupement

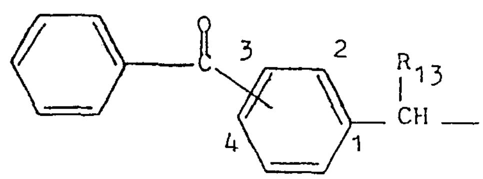

dans lequel $R_{13}$ est défini comme ci-dessus, et le radical benzoyle est en position 3 ou 4,

soit un groupement

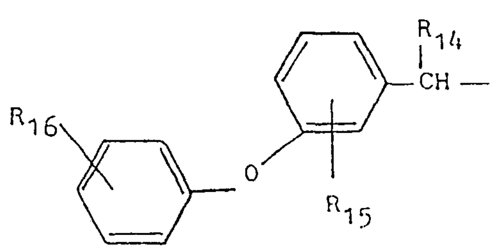

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

soit un groupement

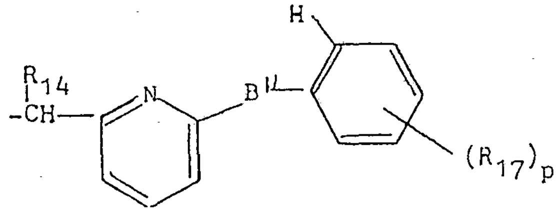

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoyl sulfonyl renfermant de 1 à 4 atomes de carbone, trifluorométhyl, 3,4-méthylène dioxy, chloro, fluoro ou bromo, p représente un nombre égal à 0, 1 ou 2 et B″ représente un atome d'oxygène ou un atome de soufre.

12. Procédé selon la revendication 11, caractérisé en ce que B est choisi dans le groupe formé par les radicaux suivants :

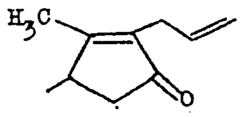 , 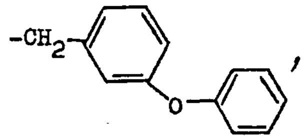 , 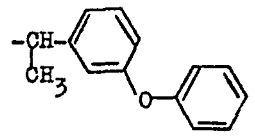 ,

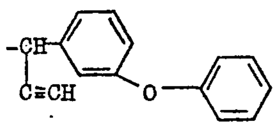 et 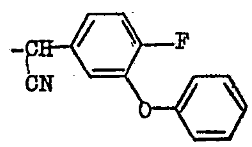

13. Procédé selon la revendication 1, pour préparer les composés répondant à la formule ($I_A$) :

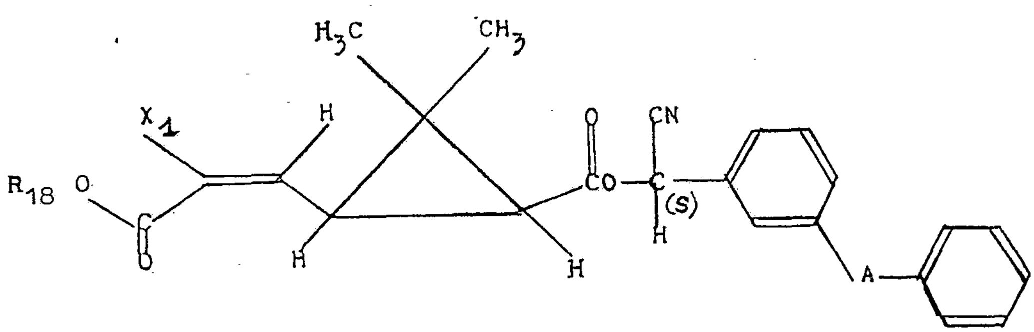

($I_A$)

dans laquelle A représente un atome d'oxygène, un groupement méthylène, un groupement carbonyle, un atome de soufre, un groupement sulfoxyde ou un groupement sulfone, $R_{18}$ représente un radical alcoyle linéaire ou ramifié renfermant de 1 à 8 atomes de carbone, $X_1$ représente un atome de fluor, de chlore ou de brome, la double liaison éthylénique ayant la géométrie (E) ou (Z) et le cycle cyclopropanique substitué pouvant être sous toutes ses formes stéréoisomères ou sous forme de mélange de ces stéréoisomères, caractérisé en ce que l'on fait réagir un acide de formule ($II_A$) :

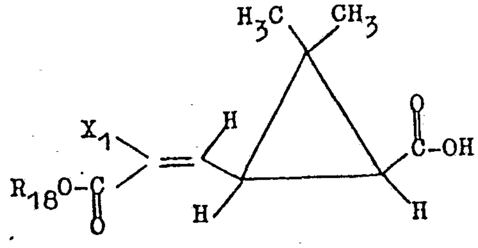

($II_A$)

dans laquelle $X_1$ et $R_{18}$ conservent la même signification que précédemment cet acide se trouvant sous forme de mélange d'isomères E + Z ou sous forme d'isomère E ou Z, le cycle cyclopropanique substitué

pouvant être sous toutes ses formes stéréoisomères ou sous forme de mélange de stéréoisomères, ou un dérivé fonctionnel de cet acide ($II_A$), avec un alcool de formule ($III_A$) :

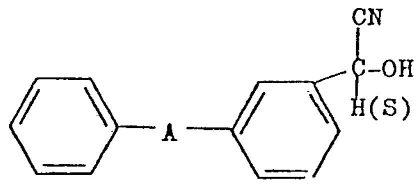

($III_A$)

dans laquelle A conserve la même signification que précédemment ou un dérivé fonctionnel de l'alcool de formule ($III_A$) et sépare éventuellement par des moyens physiques, les isomères E et Z au niveau de la double liaison.

14. Procédé selon la revendication 13, caractérisé en ce que A représente un atome d'oxygène.

15. Procédé selon la revendication 13 ou 14, caractérisé en ce que l'on prépare le (1R,cis)2,2-diméthyl 3-(E)/-2-fluoro 2-(éthoxycarbonyl)éthényl/cyclopropane-1-carboxylate de (S) α-cyano 3-phénoxy benzyle.

16. Application des composés de formule I tels que définis à la revendication 1, à la lutte contre les parasites des végétaux, les parasites des locaux et les parasites des animaux à sang chaud.

17. Application des composés de formule $I_A$ tels que définis à la revendication 13, à la lutte contre les parasites des végétaux, les parasites des locaux et les parasites des animaux à sang chaud.

18. Les compositions destinées à la lutte contre les parasites des végétaux, les parasites des locaux et les parasites des animaux à sang chaud, caractérisées en ce qu'elles renferment comme principe actif au moins un composé défini à la revendication 1.

19. Les compositions insecticides, caractérisées en ce qu'elles renferment comme principe actif au moins un composé défini à la revendication 1.

20. Les compositions acaricides, caractérisées en ce qu'elles renferment comme principe actif au moins un composé défini à la revendication 1.

21. Les compositions destinées à l'alimentation animale, caractérisées en ce qu'elles renferment comme principe actif un au moins des composés définis à la revendication 1, associé à un aliment pour animal.

22. Compositions douées d'activité insecticide, acaricide ou nématicide, caractérisées en ce qu'elles contiennent comme matière active, d'une part, un au moins des composés de formule générale (I), et, d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyanc 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane-1-carboxyliques, dans lesquels « halo » représente un atome de fluor, de chlore ou de brome, étant entendu que les composés I peuvent exister sous toutes leurs formes stéréoisomères possibles de même que les copules acides et alcools des esters pyréthrinoïdes ci-dessus.

23. Compositions selon l'une quelconque des revendications 18 à 21, caractérisées en ce que le principe actif répond à la formule ($I_A$), telle que définie à la revendication 13.

**Claims** (for the Contracting States : BE, CH, DE, FR, GB, IT, LI, LU, NL, SE)

1. In all possible isomeric forms, or in the form of a mixture of isomers, the compounds with the formula I

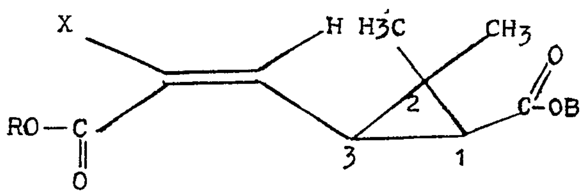

(I)

in which the cyclopropane acid copula is of 1R cis structure and the geometry of the double bond is E and in which R represents either a linear, branched or cyclic alkyl radical, saturated or unsaturated, containing from 1 to 8 carbon atoms, possibly substituted by one or more functional groups, identical or different, or an aryl group containing from 6 to 14 carbon atoms, possibly substituted by one or more functional groups, identical or different, or a heterocyclic radical possibly substituted by one or more functional groups, identical or different, X represents a halogen atom and B represents

either a benzyl radical possibly substituted by one or more radicals chosen from the group composed of alkyl radicals including from 1 to 4 carbon atoms, alkenyl radicals including from 2 to 6 carbon atoms, alkenyloxy radicals including from 2 to 6 carbon atoms, alkadienyl radicals including from 4 to 8 carbon atoms, the methylene dioxy radical and halogen atoms,

or a

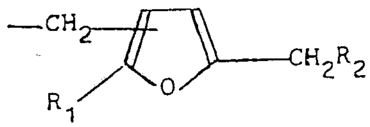

group in which the substituent $R_1$ represents a hydrogen atom or a methyl radical and the substituent $R_2$ represents a monocyclic aryl or a $-CH_2-C\equiv CH$ group and in particular a 5-benzyl-3-furyl methyl group,

or a

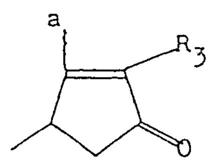

group in which a represents a hydrogen atom or a methyl radical and $R_3$ represents an organic aliphatic radical including from 2 to 6 carbon atoms and one or more carbon-carbon unsaturations and in particular the $-CH_2-CH=CH_2$, $-CH_2-CH=CH-CH_3$, $-CH_2-CH=CH-CH=CH_2$, $-CH_2-CH=CH-CH_2-CH_3$ radical,

or a

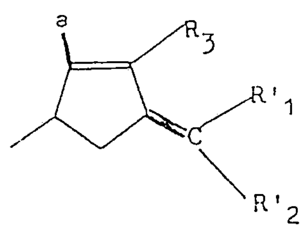

group, in which a represents a hydrogen atom or a methyl radical, $R_3$ retains the same significance as before, each of $R'_1$ and $R'_2$, identical or different, represents a hydrogen atom, a halogen atom, an alkyl radical including from 1 to 6 carbon atoms, an aryl radical including from 6 to 10 carbon atoms, an alkyloxycarbonyl group including from 2 to 5 carbon atoms, or a cyano group,

or a

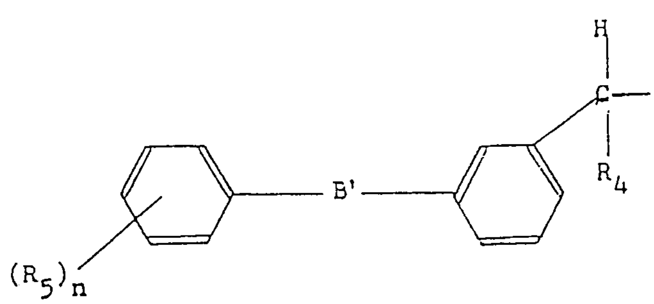

group, in which B′ represents an oxygen or sulphur atom, a

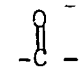

or —$CH_2$— group or a sulphoxide group or a sulphone group and $R_4$ represents a hydrogen atom, a —C≡CN radical, a methyl radical, a —$CONH_2$ radical, a —$CSNH_2$ radical or a —C≡CH radical, $R_5$ represents a halogen atom or a methyl radical and n represents a numeral, 0, 1 or 2, and in particular the 3-phenoxybenzyl, α-cyano-3-phenoxybenzyl, α-ethynyl-3-phenoxybenzyl, 3-benzoylbenzyl, 1-(3-phenoxyphenyl) ethyl or α-thioamido-3-phenoxybenzyl group,

or a

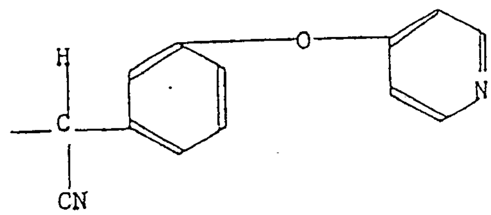

group,

or a

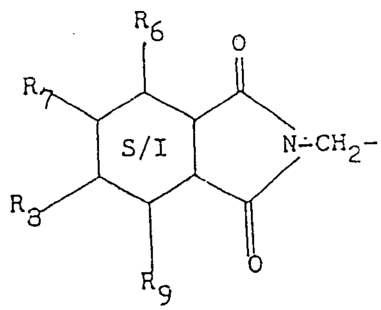

group, in which the substituents $R_6$, $R_7$, $R_8$, $R_9$ represent a hydrogen atom, a chlorine atom, or a methyl radical and in which S/I symbolises an aromatic ring or an analogous dihydro or tetrahydro ring,

or a

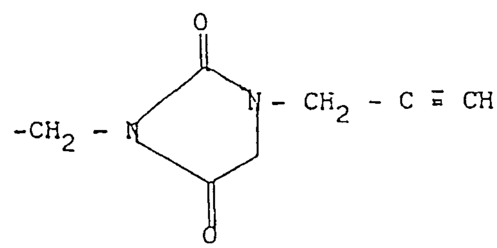

group

or a

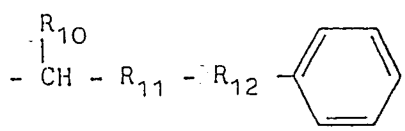

group, in which $R_{10}$ represents a hydrogen atom or a CN radical, $R_{12}$ represents a —$CH_2$— radical or an oxygen atom, $R_{11}$ represents a thiazolyl or a thiadiazolyl radical, whose bond with

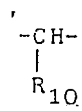

can be found at any one of the available positions, $R_{12}$ being linked to $R_{11}$ by the carbon atom contained between the sulphur atom and a nitrogen atom,

or a

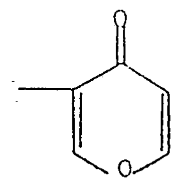

group

or a

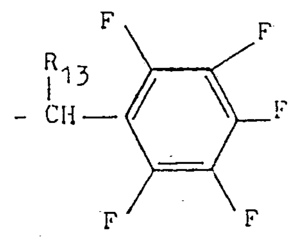

group in which $R_{13}$ represents a hydrogen atom or a CN radical,

or a

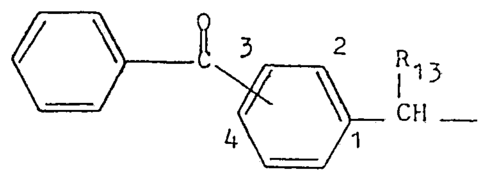

group in which $R_{13}$ is defined as above, and the benzoyl radical is at position 3 or 4,

or a

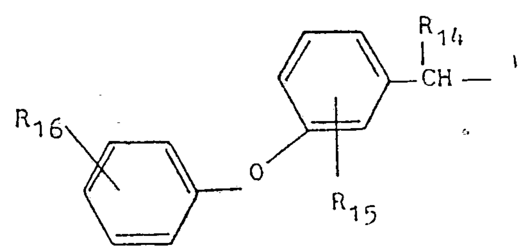

group in which $R_{14}$ represents a hydrogen atom, a methyl, ethynyl or cyano radical and each of $R_{15}$ and $R_{16}$ being different, represents a hydrogen, fluorine or bromine atom,

or a

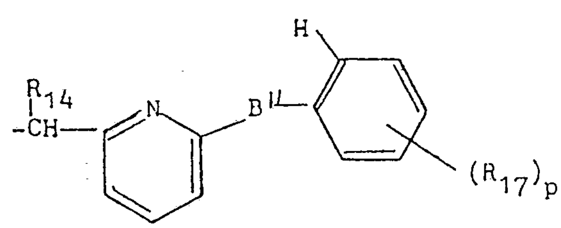

group in which $R_{14}$ is defined as above, each of the $R_{17}$'S represents independently an alkyl group containing from 1 to 4 carbon atoms, an alkoxy group containing from 1 to 4 carbon atoms, an alkylthio group containing from 1 to 4 carbon atoms, an alkyl sulphonyl group containing from 1 to 4 carbon atoms, a trifluoromethyl group, a 3,4-methylene dioxy group, or a chloro, fluoro or bromo group, p represents a numeral 0, 1 or 2 and B″ represents an oxygen atom or a sulphur atom.

2. Compounds with the formula I as defined in Claim 1 for which X represents a fluorine atom.

3. Compounds with the formula I as defined in any one of the Claims 1 or 2, for which R represents an alkyl radical, linear, branched or cyclic, containing from 1 to 8 carbon atoms.

4. Compounds with the formula I as defined in Claim 3, for which R represents an ethyl radical.

5. Compounds with the formula I as defined in Claim 3, for which R represents a tert-butyl radical.

6. Compounds with the formula I as defined in Claim 3, for which R represents a cyclopropyl or cyclopropylmethyl radical.

7. Compounds with the formula I as defined in any one of the Claims 1 or 2, for which R represents an alkyl radical, linear or branched, containing from 1 to 8 carbon atoms, substituted by one or more halogen atoms.

8. Compounds with the formula I as defined in Claim 7, for which R represents a linear alkyl radical, containing from 1 to 8 carbon atoms substituted by one or more fluorine atoms.

9. Compounds with the formula I as defined in either of the Claims 1 or 2, for which R represents a $(CH_2)_mO(CH_2)_n—CH_3$ radical in which m represents a whole number from 1 to 8 and n represents a whole number from 0 to 8, and in particular the radical $—CH_2OCH_3$.

10. Compounds with the formula I as defined in any one of the Claims 1 to 9, responding to the formula $I_A$

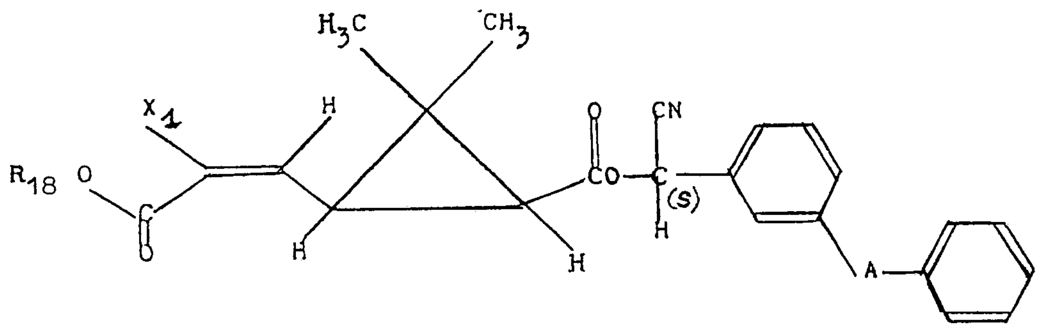

($I_A$)

in which the cyclopropane acid copula is of 1R cis structure and the geometry of the double bond is E and in which A represents an oxygen atom, a methylene group, a carbonyl group, a sulphur atom, a sulphoxide group or aa sulphone group, $R_{18}$ represents an alkyl radical, linear or branched, containing from 1 to 8 carbon atoms, $X_1$ represents a fluorine, chlorine or bromine atom.

11. Compounds with the formula $I_A$, as defined in Claim 10, for which A represents an oxygen atom.

12. (1R,cis) 2,2-dimethyl-3(E)-[2-fluoro-2-(ethoxycarbonyl)ethenyl] cyclopropane-1-carboxylate of (S) α-cyano-3-phenoxybenzyl.

13. Compounds with the formula I as defined in any one of the Claims 1 to 9, for which B represents a radical,

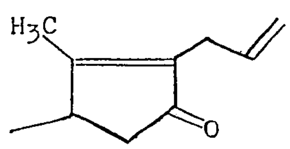

14. Compounds with the formula I as defined in any one of the Claims 1 to 9, for which B represents a radical,

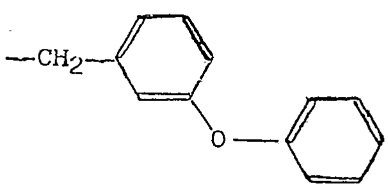

15. Compounds with the formula I as defined in any one of the Claims 1 to 9 for which B represents a radical,

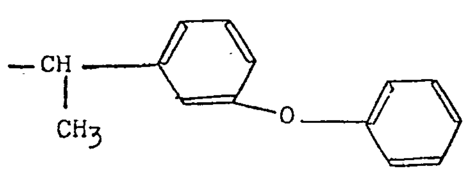

16. Compounds with the formula I as defined in any one of the Claims 1 to 9 for which B represents a radical,

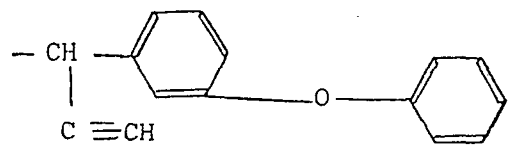

17. Compounds with the formula I as defined in any one of the Claims 1 to 9 for which B represents a radical,

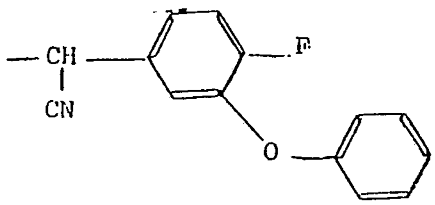

18. Use of compounds with the formula I as defined in any one of the Claims 1 to 9, 13 to 17, in combatting parasites of vegetation, parasites of premises.

19. Use of compounds with the formula I as defined in any one of the Claims 10 to 12, in combatting parasites of vegetation, parasites of premises.

20. Compositions intended for combatting parasites of vegetation, parasites of premises and parasites of warmblooded animals characterized in that they contain as active principle at least one compound defined in any one of the Claims 1 to 9 and 13 to 17.

21. Compositions intended for combatting parasites of vegetation and parasites of premises and parasites of warmblooded animals, characterized in that they contain as active principle at least one compound defined in any one of the Claims 10 to 12.

22. Insecticidal compositions characterized in that they contain as active principle at least one compound defined in any one of the Claims 1 to 9 and 13 to 17.

23. Insecticidal compositions characterized in that they contain as active principle at least one compound defined in any one of the Claims 10 to 12.

24. Acaricidal compositions, characterized in that they contain as active principle at least one compound defined in any one of the Claims 1 to 9 and 13 to 17.

25. Acaricidal compositions, characterized in that they contain as active principle at least one compound defined in any one of the Claims 10 to 12.

26. Compositions intended for animal feeding, characterized in that they contain as active principle at least one compound defined in any one of the Claims 1 to 9 and 13 to 17, combined with an animal foodstuff.

27. Compositions intended for animal feeding, characterized in that they contain as active principle at least one compound defined in any one of the Claims 10 to 12, combined with an animal foodstuff.

28. Combinations endowed with insecticide, acaricide or nematocide activity, characterized in that they contain as active material, on the one hand one or more of the compounds with the general formula I, and on the other hand, one at least of the pyrethrinoid esters chosen from the group constituted by the esters of allethrolones, of 3,4,5,6-tetrahydrophthalimidomethyl alcohol, of 5-benzyl-3-furylmethyl alcohol, of 3-phenoxybenzyl alcohol and of α-cyano-3-phenoxybenzyl alcohols with chrysanthemic acids, by the esters of 5-benzyl-3-furylmethyl alcohol with 2,2-dimethyl-3-(2-oxo-3-tetrahydrothiophenylidenemethyl)c yclopropane-1-carboxylic acids, by the esters of 3-phenoxybenzyl alcohol and α-cyano-3-phenoxybenzyl alcohols with 2,2-dimethol-3-(2,2-dichlorovinyl)cyclopropane-1-carboxylic acids, by the esters of α-cyano-3-phenoxybenzyl alcohols with 2,2-dimethyl-3-(2,2-dibromovinyl)cyclopropane-1-carboxylic acids, by the esters of 3-phenoxybenzyl alcohol with 2-parachlorophenyl-2-isopropylacetic acids, by the esters of allethrolones, of 3,4,5,6-tetrahydrophthalimidomethyl alcohol, of 5-benzyl-3-furylmethyl alcohol, of 3-phenoxybenzyl alcohol and α-cyano-3-phenoxybenzyl alcohols with 2,2-dimethyl-3-(1,2,2,2-tetrahaloethyl)cyclopropane-1-carboxylic acids, in which « halo » represents a fluorine, chlorine or bromine atom, it being understood that the compounds I can exist in all their possible stereoisomeric forms as can also the acid and alcohol copulas of the above pyrethrinoid esters.

29. As medicaments, the compounds with the formula I, as defined in any one of the Claims 1 to 17.

30. Pharmaceutical compositions containing as active principle, at least one of the medicaments defined in Claim 29.

31. Preparation process for the compounds with the formula I as defined in any one of the Claims 1 to 9 and 13 to 17, characterized in that an acid with the formula II :

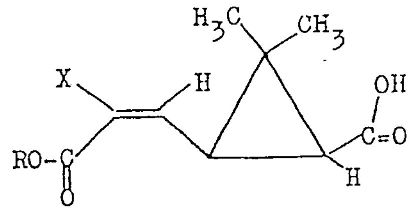

(II)

in which the cyclopropane copula is of 1R cis structure and the geometry of the double bond is E and in which X and R retain the same significance as previously, or a functional derivative of this acid, is made to react with an alcohol with the formula III :

B—OH (III)

where B retains the same significance as previously, or with a functional derivative of this alcohol, so as to obtain a corresponding compound with the formula I, which is submitted, if desired, to the action of a selective cleavage agent of the $CO_2R$ group so as to obtain a compound with the formula IV :

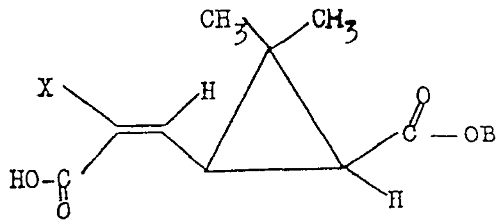

(IV)

in which the cyclopropane copula is of 1R cis structure and the geometry of the double bond is E and in which B retains the same significance as previously, then this acid with the formula IV or a functional derivative of this acid, is submitted to the action of an alcohol with the formula R—OH in which R retains its previous significance, so as to obtain the corresponding compound with the formula I.

32. Preparation process of compounds with the formula $I_A$, as defined in any one of the Claims 10 to 12, characterized in that an acid, with the formula $II_A$

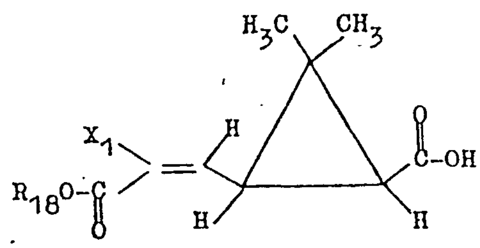

($II_A$)

in which the cyclopropane copula is of 1R cis structure and the geometry of the double bond is E and in which $X_1$ and $R_{18}$ retain the same significance as in Claim 13, or a functional derivative of this acid $II_A$, is made to react with an alcohol with the formula $III_A$ :

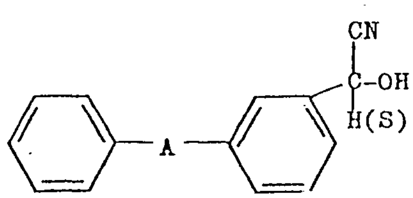

($III_A$)

in which A retains the same significance as in Claim 10, or with a functional derivative of an alcohol with the formula $III_A$, which is separated so as to obtain a corresponding compound with the formula $I_A$.

33. Process according to Claim 31 or 32, characterized in that the acids with the formula II or $II_A$ in which R or $R_{18}$ represents an alkyl radical containing from 1 to 5 carbon atoms, are prepared by making a cis aldehyde in the form of a lactone with the formula V :

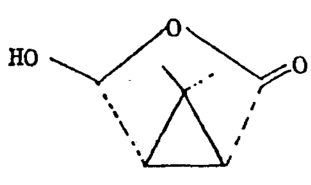

(V)

react according to Wittig's reaction with a phosphorane with the formula VII :

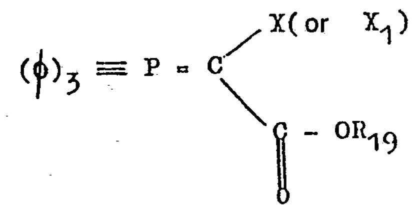

(VII)

in which formula $R_{19}$ represents an alkyl radical including from 1 to 5 carbon atoms and X and $X_1$ retain their previous significances, or with a phosphonate with the formula VIII :

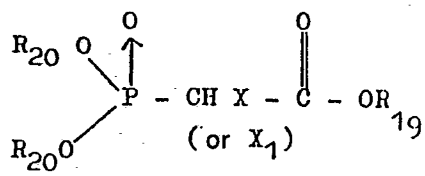

(VIII)

in which formula $R_{19}$, X and $X_1$ retain the significances cited previously and $R_{20}$ represents an alkyl radical including from 1 to 6 carbon atoms.

34. As new chemical products, acids with the formulas II and $II_A$ as defined in the Claims 31 and 32, as well as the functional derivatives of these acids.

35. As new chemical products, acids with the formula IV as defined in Claim 31, as well as the functional derivatives of these acids.

36. As intermediate products necessary for carrying out the process of Claims 31 and 32, the compounds with the formulas II, $II_A$ and IV, as defined in Claims 31 and 32.

**Claims** (for the Contracting State AT)

1. Process for preparing, in all possible isomeric forms, or in the form of a mixture of isomers, the compounds with the formula I :

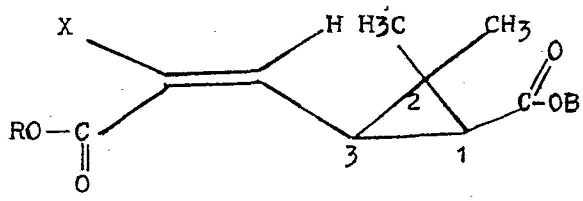

(I)

in which R represents either a linear, branched or cyclic alkyl radical, saturated or unsaturated, containing from 1 to 8 carbon atoms, possibly substituted by one or more functional groups, identical or different, or an aryl group containing from 6 to 14 carbon atoms, possibly substituted by one or more functional groups, identical or different, or a heterocyclic radical possibly substituted by one or more functional groups, identical or different, B represents either a linear, branched or cyclic alkyl radical, saturated or unsaturated, containing from 1 to 18 carbon atoms, or the residue of an alcohol used in the synthesis of esters of the pyrethrinoid series and X represents a halogen atom, the ethylene double bond having the geometry Z or E, characterized in that an acid with the formula II :

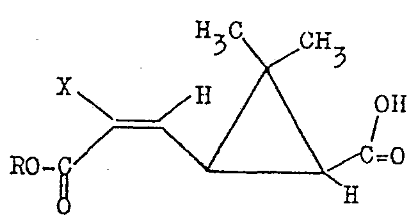

(II)

in which X and R retain the same significance as previously, this acid being in the form of mixtures of E or Z isomers or in the form of an E or Z isomer, the substituted cyclopropane ring being able to be in all its possible stereo-isomeric forms, or in the form of a mixture of stereo-isomers or a functional derivative of this acid, is made to react with an alcohol with the formula III :

B—OH (III)

where B retains the same significance as previously or with a functional derivative of this alcohol, so as to obtain a corresponding compound with the formula I, which if desired, is submitted to the the action of a selective cleavage agent of the $CO_2R$ group so as to obtain a compound with the formula IV :

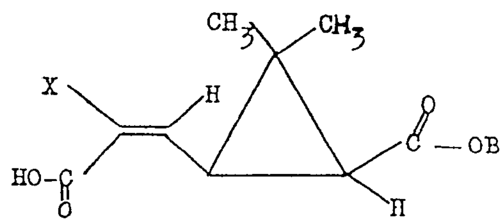

(IV)

in which B retains the same significance as previously, then, this acid with the formula IV or a functional derivative of this acid, is submitted to the action of an alcohol with the formula R—OH in which R retains its previous significance, so as to obtain a corresponding compound with the formula I.

2. Process according to Claim 1, characterized in that, at the start, an acid with the formula II and of 1R cis or 1R trans structure is used.

3. Process according to Claim 1 or 2, characterized in that at the start, an acid with the formula II, for which the double bond has the geometry E, is used.

4. Process according to any one of the Claims 1 to 3, characterized in that at the start, an acid with the formula II, for which X represents a fluorine atom, is used.

5. Process according to any one of the Claims 1 to 4, characterized in that, at the start, an acid with the formula II, or an alcohol with the formula R—OH, for which R represents a linear, branched or cyclic alkyl radical, containing from 1 to 8 carbon atoms, is used.

6. Process according to Claim 5, characterized in that R is an ethyl or tert-butyl radical.

7. Process according to any one of the Claims 1 to 4, characterized in that, at the start, an acid with the formula II or an alcohol with the formula R—OH, for which R represents a cyclopropyle or cyclopropylmethyl radical, is used.

8. Process according to any one of the Claims 1 to 4, characterized in that at the start, an acid with the formula II or an alcohol with the formul R—OH, for which R represents an alkyl radical, linear or branched, containing from 1 to 8 carbon atoms, substituted by one or more halogen atoms, is used.

9. Process according to any one of the Claims 1 to 4, characterized in that, at the start, an acid with the formula II and an alcohol with the formula R—OH, for which R represents a linear alkyl radical, containing from 1 to 8 carbon atoms, substituted by one or more fluorine atoms, are used.

10. Process according to any one of the Claims 1 to 5, characterized in that at the start, an acid with the formula II and an alcohol with the formula R—OH, for which R represents a $(CH_2)_mO(CH_2)_n—CH_3$ radical in which m represents a whole number from 1 to 8 and n represents a whole number from 1 to 8 and n represents a whole number from 0 to 8, and in particular the radical $—CH_2OCH_3$, are used.

11. Process according to any one of the Claims 1 to 10, characterized in that at the start, an alcohol with the formula B—OH is used, for which B represents :

either an alkyl radical containing from 1 to 18 carbon atoms,

or a benzyl radical possibly substituted by one or more radicals chosen from the group composed of the alkyl radicals including from 1 to 4 carbon atoms, the alkenyl radicals including from 2 to 6 carbon atoms, the alkenyloxy radicals including from 2 to 6 carbon atoms, the alkadienyl radical including from 4 to 8 carbon atoms, the methylene dioxy radical and the halogen atoms,

or a

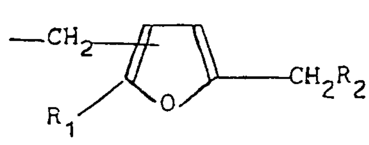

group, in which the substituent $R_1$ represents a hydrogen atom or a methyl radical and the substituent $R_2$ represents a monocyclic aryl or a $—CH_2—C{=}CH$ group, and in particular a 5-benzyl-3-furyl methyl group,

or a

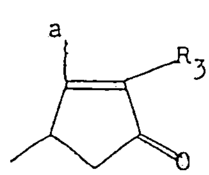

group, in which a represents a hydrogen atom or a methyl radical and $R_3$ represents an organic aliphatic radical including from 2 to 6 carbon atoms and one or more carbon-carbon unsaturations and in particular the $-CH_2-CH=CH_2$, $-CH_2-CH=CH-CH_3$, $-CH_2CH=CH-CH=CH_2$, $-CH_2-CH=CH-CH_2-CH_3$,

or a

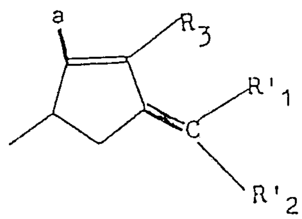

group, in which a represents a hydrogen atom or a methyl radical, $R_3$ retains the same significance as before, each of $R_1'$ and $R_2'$, identical or different, represents a hydrogen atom, a halogen atom, an alkyl radical containing from 1 to 6 carbon atoms, an aryl radical containing from 6 to 10 carbon atoms, an alkyloxycarbonyl group containing from 2 to 5 carbon atoms, or a cyano group,

or a

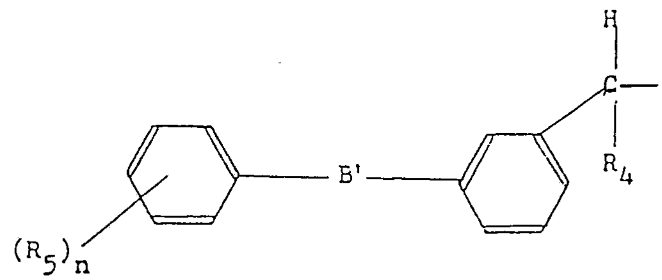

group, in which B′ represents an oxygen or sulphur atom, a

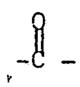

or $-CH_2-$ group or sulphoxide group or a sulphone group and $R_4$ represents a hydrogen atom, a $-C\equiv N$ radical, a methyl radical, a $-CONH_2$, radical, a $-CSNH_2$ radical or a $-C\equiv CH$ radical, $R_5$ represents a halogen atom or a methyl radical and n represents a numeral, 0, 1 or 2, and in particular the 3-phenoxybenzyl, α-cyano-3-phenoxybenzyl, α-ethynyl-3-phenoxybenzyl, 3-benzoylbenzyle, 1-(3-phenoxy phenyl) ethyl or α-thioamido-3-phenoxybenzyl group,

or a

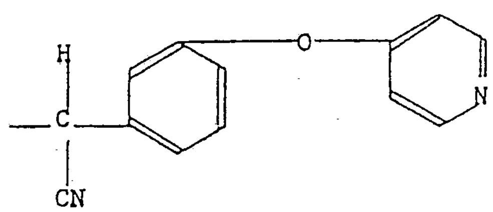

group,

or a

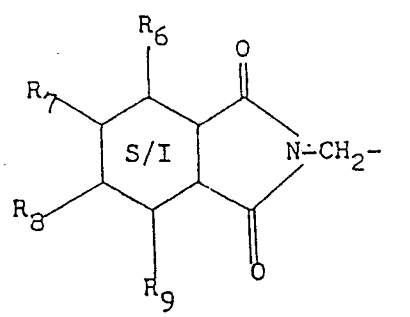

group, in which the substituents $R_6$, $R_7$, $R_8$, $R_9$ represent a hydrogen atom, a chlorine atom, or a methyl radical and in which S/I symbolises an aromatic ring or an analogous dihydro or tetrahydro ring,
or a

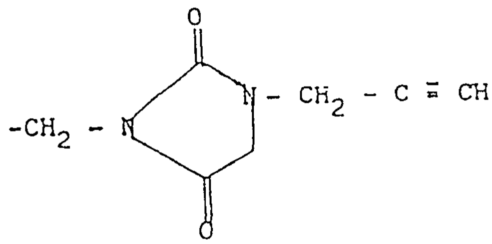

group,
or a

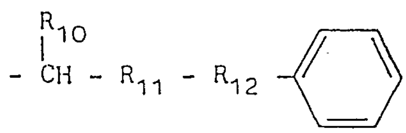

group, in which $R_{10}$ represents a hydrogen atom or a CN radical, $R_{12}$ represents a $—CH_2—$ radical or an oxygen atom, $R_{11}$ represents a thiazolyl or a thiadiazolyl radical, whose bond with

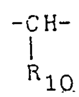

can be found at any one of the available positions, $R_{12}$ being linked to $R_{11}$ by the carbon atom contained between the sulphur atom and a nitrogen atom,
or a

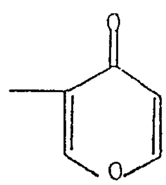

group,
or a

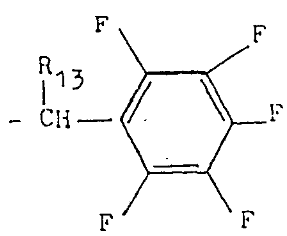

group, in which $R_{13}$ represents a hydrogen atom or a CN radical,
or a

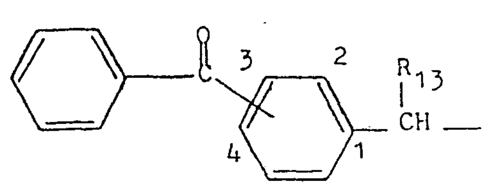

group, in which $R_{13}$ is defined as above, and the benzoyl radical is at position 3 or 4,
or a

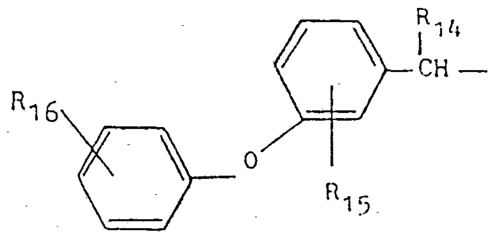

group, in which $R_{14}$ represents a hydrogen atom, a methyl, ethynyl or cyano radical and each of $R_{15}$ and $R_{16}$ being different, represents a hydrogen, fluorine or bromine atom,

or a

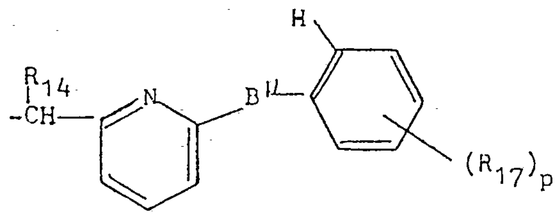

group in which $R_{14}$ is defined as above, each of the $R_{17}$'s represents independently an alkyl group containing from 1 to 4 carbon atoms, an alkoxy group containing from 1 to 4 carbon atoms, an alkylthio group containing from 1 to 4 carbon atoms, an alkyl sulphonyl group containing from 1 to 4 carbon atoms, a trifluoromethyl group, a 3,4-methylene dioxy group, or a chloro, fluoro or bromo group, p represents a numeral 0, 1 or 2 B" represents an oxygen atom or a sulphur atom.

12. Process according to Claim 11, characterized in that B is chosen from the group formed by the following radicals :

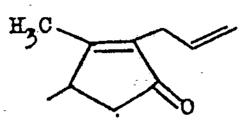 , 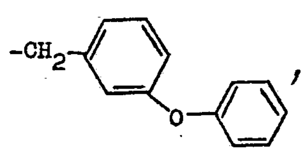 , 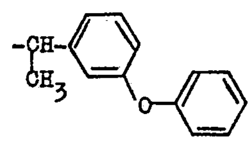

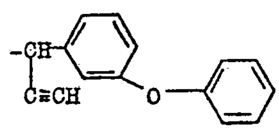 and 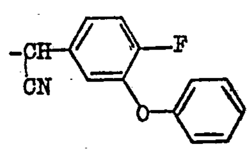

13. Process according to Claim 1, for the preparation of the compounds responding to the formula $I_A$

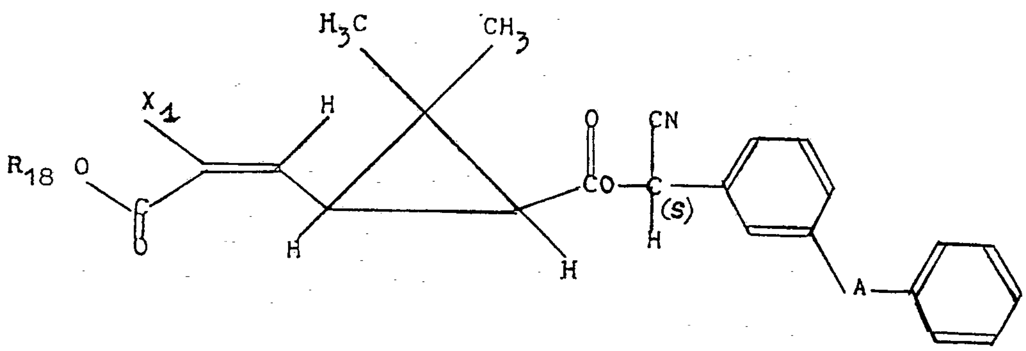

$(I_A)$

in which A represents an oxygen atom, a methylene group, a carbonyl group, a sulphur atom, a

sulphoxide group or a sulphone group, $R_{18}$ represents a linear or branched alkyl radical containing from 1 to 8 carbon atoms, $X_1$ represents a fluorine, chlorine or bromine atom, the ethylene double bond has the geometry E or Z and the substituted cyclopropane cycle can be in all its stereo-isomeric forms or in the form of a mixture of these stereo-isomers, characterized in that an acid with the formula $II_A$

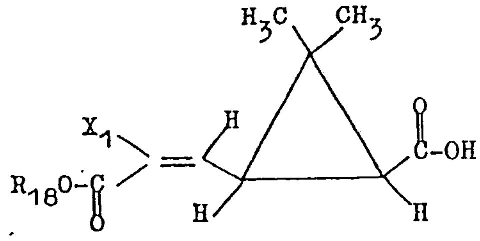

($II_A$)

in which $X_1$ and $R_{18}$ retain the same significance as before, this acid being in the form of a mixture of isomers E + Z or in the form of an isomer E or Z, the substituted cyclopropane cycle being able to be in all of its stereo-isomeric forms or in the form of a mixture of stereo-isomers, or a functional derivative of this acid $II_A$, is made to react with an alcohol with the formula $III_A$ :

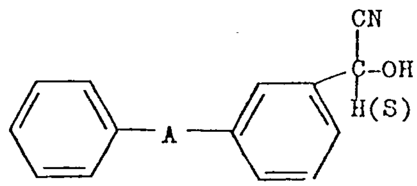

($III_A$)

in which A retains the same significance as before or with a functional derivative of the alcohol with the formula $III_A$ and possibly by physical means, the isomers E and Z are separated at the double bond.

14. Process according to Claim 13, characterized in that A represents an oxygen atom.

15. Process according to Claims 13 or 14, characterized in that (1R,cis) 2,2-dimethyl-3(E) [-2-fluoro-2-(ethoxy carbonyl) ethenyl] cyclopropane-1-carboxylate of (S) α-cyano-3-phenoxybenzyl is prepared.

16. Use of compounds with the formula I as defined in Claim 1, in combatting parasites of vegetation, parasites of premises and parasites of warm-blooded animals.

17. Use of compounds with the formula $I_A$ as defined in Claim 13, in combatting parasites of vegetation, parasites of premises and parasites of warm-blooded animals.

18. Compositions intended for combatting parasites of vegetation, parasites of premises and parasites of warm-blooded animals, characterized in that they contain as active principle at least one compound defined in Claim 1.

19. Insecticidal compositions, characterized in that they contain as active principle at least one compound defined in Claim 1.

20. Acaricidal compositions, characterized in that they contain as active principle at least one compound defined in Claim 1.

21. Compositions intended for animal feeding, characterized in that they contain as active principle at least one of the compounds defined in Claim 1, combined with an animal foodstuff.

22. Compositions endowed with insecticide, acaricide or nematocide activity, characterized in that they contain as active material, on the one hand one at least of the compounds with the general formula I, and on the other hand, one at least og the pyrethrinoid esters chosen from the group constituted by the esters of allethrolones, of 3,4,5,6-tetrahydrophthalimidomethyl alcohol, of 5-benzyl-3-furyl-methyl alcohol, of 3-phenoxybenzyl alcohol and of α-cyano-3-phenoxybenzyl alcohols with chrysanthemic acids, by the esters of 5-benzyl-3-furylmethyl alcohol with 2,2-dimethyl-3-(2-oxo-tetrtahydrothiophenylidenemethyl) cyclopropane-1-carboxylic acids, by the esters of 3-phenoxybenzyl alcohol and α-cyan-3-phenoxybenzyl alcohols with 2,2-dimethyl-3-(2,2-dichlorovinyl) cyclopropane-1-carboxylic acids, by the esters of α-cyano-3-phenoxybenzyl alcohols with 2,2-dimethyl-3-(2,2-dibromovinyl) cyclopropane-1-carboxylic acids, by the esters of 3-phenoxybenzyl alcohol with 2-parachlorophenyl-2-isopropylacetic acids, by the esters of allethrolones, of 3,4,5,6-tetrahydrophthalimidomethyl alcohol, of 5-benzyl-3-furylmethyl alcohol, of 3-phenoxybenzyl alcohol and α-cyano-3-phenoxybenzyl alcohols with 2,2-dimethyl-3-(1,2,2,2-tetrahaloethyl) cyclopropane-1-carboxylic acids, in which « halo » represents a fluorine, chlorine or bromine atom, it being understood that the compounds I can exist in all their possible stereo-isomeric forms as can also acid and alcohol copulas of the above pyrethrinoid esters.

23. Compositions according to any one of Claims 18 to 21, characterized in that the active principle corresponds to the formula ($I_A$), as defined in Claim 1.

**Ansprüche** (für die Vertragsstaaten : BE, CH, DE, FR, GB, IT, LI, LU, NL, SE)

1. In sämtlichen ihrer möglichen isomeren Formen oder in Form eines Gemisches der Isomeren die Verbindungen der Formel I

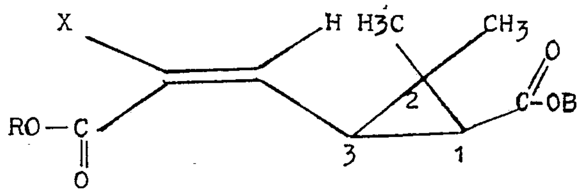

(I)

worin die Struktur der Cyclopropansäure-Verknüpfung die 1R,cis-Struktur und die Geometrie der Doppelbindung die E-Geometrie ist und worin R entweder einen gesättigten oder ungesättigten, linearen, verzweigten oder cyclischen Alkylrest mit 1 bis 8 Kohlenstoffatomen, der gegebenenfalls durch eine oder mehrere identische oder verschiedene funktionelle Gruppen substituiert ist, oder eine Arylgruppe mit 6 bis 14 Kohlenstoffatomen, die gegebenenfalls durch eine oder mehrere identische oder verschiedene funktionelle Gruppen substituiert ist, oder einen heterocyclischen Rest, der gegebenenfalls durch eine oder mehrere identisch oder verschiedene funktionnelle Gruppen substituiert ist, bedeutet, X ein Halogenatom bedeutet und B bedeutet:

entweder einen Benzylrest, der durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen, substituiert ist,

oder eine Gruppe

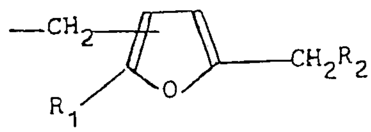

worin der Substituent $R_1$ ein Wasserstoffatom oder einen Methylrest bedeutet und der Substituent $R_2$ einen monocyclischen Arylrest oder eine Gruppe $—CH_2—C{\equiv}CH$ bedeutet, und insbesondere eine 5-Benzyl-3-furyl-methylgruppe,

oder eine Gruppe

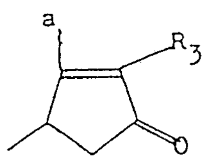

worin a ein Wasserstoffatom oder einen Methylrest bedeutet und $R_3$ einen aliphatischen, organischen Rest mit 2 bis 6 Kohlenstoffatomen und einer oder mehreren Kohlenstoff-Kohlenstoff-Unsättigungen und insbesondere den Rest $—CH_2—CH{=}CH_2$, $—CH_2—CH{=}CH—CH_3$, $—CH_2—CH{=}CH—CH{=}CH_2$, $—CH_2—CH{=}CH—CH_2—CH_3$ bedeutet,

oder eine Gruppe

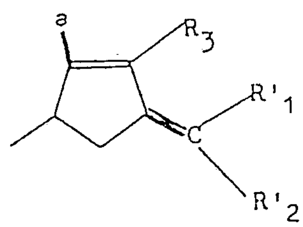

worin a ein Wasserstoffatom oder einen Methylrest bedeutet, $R_3$ die vorstehend angegebene Bedeutung besitzt, $R_1'$ und $R_2'$, die gleich oder verschiedenen sein können, ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeuten,

oder eine Gruppe

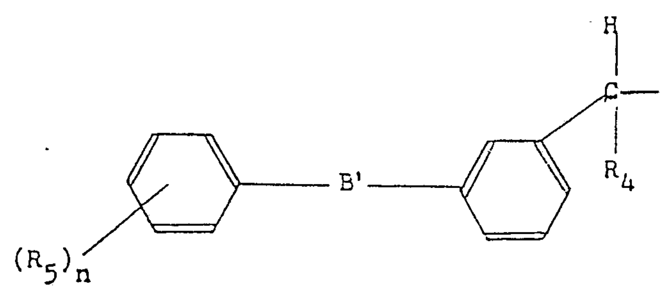

worin B' ein Sauerstoff- oder Schwefelatom, eine Gruppe

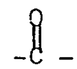

oder $—CH_2—$ oder eine Sulfoxidgruppe oder eine Sulfongruppe bedeutet und $R_4$ ein Wasserstoffatom, einen Rest —C≡N, einen Methylrest, einen Rest $—CONH_2$, einen Rest $—CSNH_2$ oder einen Rest —C≡CH bedeutet, $R_5$ ein Halogenatom oder einen Methylrest bedeutet und n eine Zahl entsprechend 0, 1 oder 2 bedeutet, und insbesondere die 3-Phenoxybenzyl-, α-Cyano-3-phenoxybenzyl-, α-Ethinyl-3-phenoxybenzyl-, 3-Benzoylbenzyl-, 1-(3-Phenoxyphenyl)-ethyl- oder α-Thioamido-3-phenoxybenzyl-Gruppe,

oder eine Gruppe

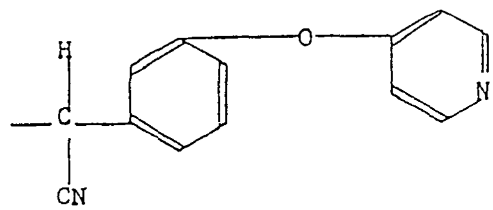

oder eine Gruppe

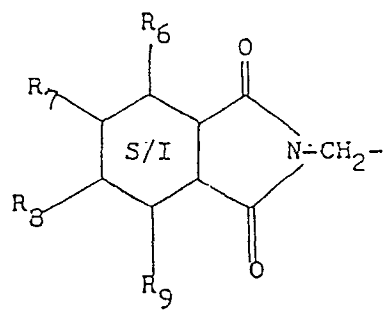

worin die Substituenten $R_6$, $R_7$, $R_8$ und $R_9$ ein Wasserstoffatom, ein Chloratom oder einen Methylrest darstellen und worin S/I einen aromatischen Ring oder einen analogen Dihydro- oder Tetrahydroring symbolisiert,

oder eine Gruppe

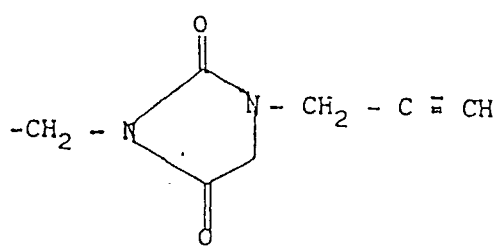

oder eine Gruppe

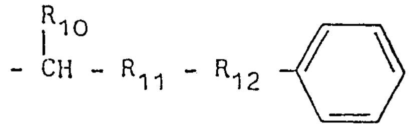

worin $R_{10}$ ein Wasserstoffatom oder einen CN-Rest darstellt, $R_{12}$ einen Rest $—CH_2—$ oder ein Sauerstoffatom bedeutet, $R_{11}$ einen Thiazolyl- oder Thiadiazolylrest darstellt, dessen Bindung mit

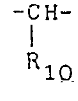

sich in irgendeiner der verfügbaren Positionen befinden kann, wobei $R_{12}$ an $R_{11}$ über ein Kohlenstoffatom gebunden ist, das zwischen dem Schwefel- und einem Stickstoffatom liegt,

oder eine Gruppe

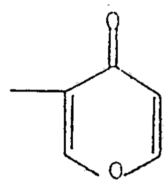

oder eine Gruppe

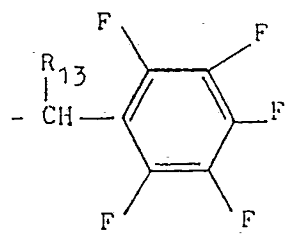

worin $R_{13}$ ein Wasserstoffatom oder einen CN-Rest bedeutet,

oder eine Gruppe

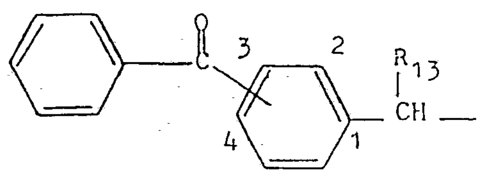

worin $R_{13}$ wie vorstehend definiert ist und der Benzoylrest sich in 3- oder 4-Stellung befindet,

oder eine Gruppe

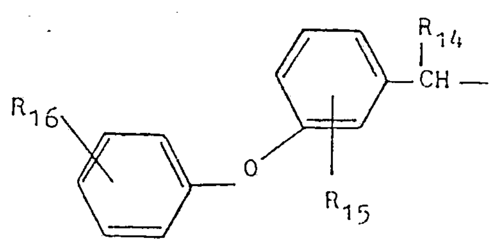

worin $R_{14}$ ein Wasserstoffatom, einen Methyl-, Ethinyl- oder Cyanorest bedeutet und $R_{15}$ und $R_{16}$, die voneinander verschieden sind, ein Wasserstoff-, Fluor- oder Bromatom bedeuten,

oder eine Gruppe

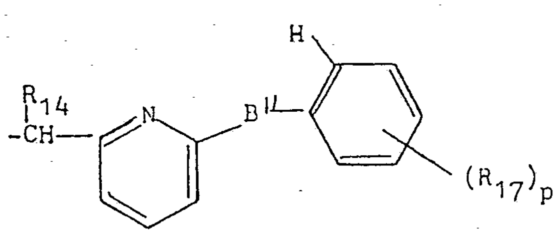

worin $R_{14}$ wie vorstehend definiert ist, ein jeder der $R_{17}$-Reste unabhängig eine Alkylgruppe mit 1 bis 4 Kohlenstoffatomen, eine Alkoxygruppe mit 1 bis 4 Kohlenstoffatomen, eine Alkylthiogruppe mit 1 bis 4 Kohlenstoffatomen, eine Alkylsulfonylgruppe mit 1 bis 4 Kohlenstoffatomen, eine Trifluormethyl-, 3,4-Methylendioxy-Gruppe, Chlor, Fluor oder Brom bedeutet, p eine Zahl entsprechend 0, 1 oder 2 darstellt und B″ ein Sauerstoff- oder ein Schwefelatom bedeutet.

2. Verbindungen der Formel I gemäß Anspruch 1, worin X ein Fluoratom bedeutet.

3. Verbindungen der Formel I gemäß einem der Ansprüsche 1 oder 2, worin R einen linearen, verzweigten oder cyclischen Alkylrest mit 1 bis 8 Kohlenstoffatomen darstellt.

4. Verbindungen der Formel I gemäß Anspruch 3, worin R einen Ethylrest bedeutet.

5. Verbindungen der Formel I gemäß Anspruch 3, worin R einen tert.-Butylrest bedeutet.

6. Verbindungen der Formel I gemäß Anspruch 3, worin R einen Cyclopropyl- oder Cyclopropylmethylrest bedeutet.

7. Verbindungen der Formel I gemäß einem der Ansprüche 1 oder 2, worin R einen linearen oder verzweigten Alkylrest mit 1 bis 8 Kohlenstoffatomen darstellt, der durch ein oder mehrere Halogenatome substituiert ist.

8. Verbindungen der Formel I gemäß Anspruch 7, worin R einen linearen Alkylrest mit 1 bis 8 Kohlenstoffatomen, der durch ein oder mehrere Fluoratome substituiert ist, bedeutet.

9. Verbindungen der Formel I gemäß einem der Ansprüche 1 oder 2, worin R einen Rest $(CH_2)_mO(CH_2)_n—CH_3$, worin m eine ganze Zahl von 1 bis 8 darstellt und n eine ganze Zahl von 0 bis 8 darstellt, und insbesondere den Rest $—CH_2OCH_3$ bedeutet.

10. Verbindungen der Formel I gemäß einem der Ansprüche 1 bis 9 der Formel $I_A$

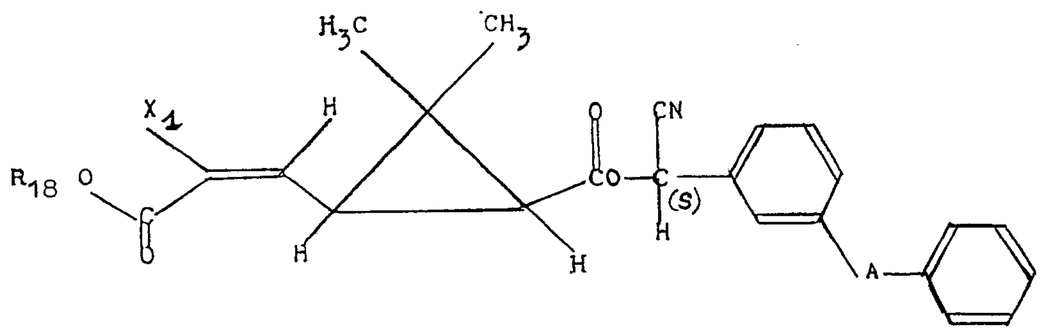

$(I_A)$

worin die Struktur der Cyclopropansäure-Verknüpfung die 1R,cis-Struktur ist und die Geometrie der Doppelbindung die E-Geometrie ist und worin A ein Sauerstoffatom, eine Methylengruppe, eine Carbonylgruppe, ein schwefelatom, eine Sulfoxidgruppe oder eine Sulfongruppe bedeutet, $R_{18}$ einen linearen oder verzweigten Alkylrest mit 1 bis 8 Kohlenstoffatomen darstellt, $X_1$ ein Fluor-, Chlor- oder Bromatom bedeutet.

11. Verbindungen der Formel $(I_A)$ gemäß Anspruch 10, worin A ein Sauerstoffatom bedeutet.

12. (S)-α-Cyano-3-phenoxybenzyl-(1R,cis)-2,2-dimethyl-3(E)-[2-fluor-2-(ethoxycarbonyl)-ethenyl]-cyclopropan-1-carboxylat.

13. Verbindungen der Formel I gemäß einem der Ansprüche 1 bis 9, worin B einen Rest

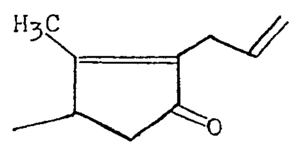

bedeutet.

14. Verbindungen der Formel I gemäß einem der Ansprüche 1 bis 9, worin B einen Rest der Formel

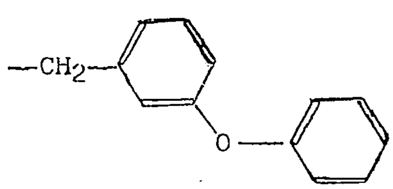

bedeutet.

15. Verbindungen der Formel I gemäß einem der Ansprüche 1 bis 9, worin B einen Rest der Formel

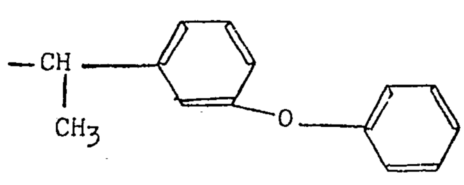

bedeutet.

16. Verbindungen der Formel I gemäß einem der Ansprüche 1 bis 9, worin B einen Rest der Formel

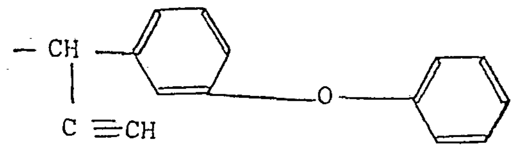

bedeutet.

17. Verbindungen der Formel I gemäß einem der Ansprüche 1 bis 9, worin B einen Rest der Formel

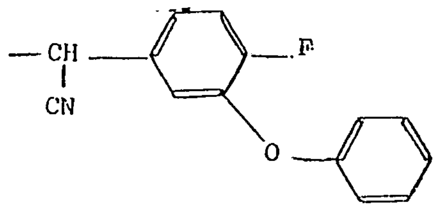

bedeutet.

18. Verwendung der Verbindungen der Formel I gemäß einem der Ansprüche 1 bis 9 und 13 bis 17 zur Bekämpfung von Parasiten der Pflanzen und Parasiten von Örtlichkeiten.

19. Verwendung der Verbindungen der Formel $I_A$ gemäß einem der Ansprüche 10 bis 12 zur Bekämpfung von Parasiten der Pflanzen und Parasiten von Örtlichkeiten.

20. Zusammensetzungen für die Bekämpfung von Parasiten der Pflanzen, Parasiten von Örtlichkeiten und Parasiten warmblütiger Tiere, dadurch gekennzeichnet, daß sie als Wirkstoff zumindest eine Verbindung gemäß einem der Ansprüche 1 bis 9 und 13 bis 17 enthalten.

21. Zusammensetzungen für die Bekämpfung von Parasiten der Pflanzen und Parasiten von Örtlichkeiten sowie Parasiten warmblütiger Tiere, dadurch gekennzeichnet, daß sie als Wirkstoff zumindest eine Verbindung gemäß einem der Ansprüche 10 bis 12 enthalten.

22. Insektizide Zusammensetzungen, dadurch gekennzeichnet, daß sie als Wirkstoff zumindest eine Verbindung gemäß einem der Ansprüche 1 bis 9 und 13 bis 17 enthalten.

23. Insektizide Zusammensetzungen, dadurch gekennzeichnet, daß sie als Wirkstoff zumindest eine Verbindung gemäß einem der Ansprüche 10 bis 12 enthalten.

24. Akarizide Zusammensetzungen, dadurch gekennzeichnet, daß sie als Wirkstoff zumindest eine Verbindung gemäß einem der Ansprüche 1 bis 9 und 13 bis 17 enthalten.

25. Akarizide Zusammensetzungen, dadurch gekennzeichnet, daß sie als Wirkstoff zumindest eine Verbindung gemäß einem der Ansprüche 10 bis 12 enthalten.

26. Zusammensetzungen für die tierische Ernährung, dadurch gekennzeichnet, daß sie als Wirkstoff zumindest eine Verbindung gemäß einem der Ansprüche 1 bis 9 und 13 bis 17 zusammen mit einem tierischen Ernährungsmittel enthalten.

27. Zusammensetzungen für die tierische Ernährung, dadurch gekennzeichnet, daß sie als Wirkstoff zumindest eine der Verbindungen gemäß einem der Ansprüche 10 bis 12 zusammen mit einem tierischen Ernährungsmittel enthalten.

28. Mit insektizider, akarizider oder nematizider Aktivität ausgestattete Assoziationen, dadurch gekennzeichnet, daß sie als Wirkstoff einesteils zumindest eine Verbindung der allgemeinen Formel (I) und anderenteils zumindest einen der Pyrethrinoidester enthalten, ausgewählt unter den Estern der Allethrolone, des 3,4,5,6-Tetrahydrophthalimido-methylalkohols, des 5-Benzyl-3-furyl-methylalkohols, des 3-Phenoxybenzylalkohols und der α-Cyano-3-phenoxy-benzylalkohole der Chrysanthemumsäuren, unter den Estern des 5-Benzyl-3-furyl-methylalkohols der 2,2-Dimethyl-3-(2-oxo-3-tetrahydrothiophenyliden-methyl)-cyclopropan-1-carbonsäuren, unter den Estern des 3-Phenoxybenzylalkohols und der α-Cyano-3-phenoxy-benzylalkohole der 2,2-Dimethyl-3-(2,2-dichlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropylessigsäuren, unter den Estern der Allethrolone, des 3,4,5,6-Tetrahydrophthalimido-methylalkohols, des 5-Benzyl-3-furyl-methylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, worin « halo » ein Chlor-, Fluor- oder Bromatom bedeutet, wobei die Verbindungen I in sämtlichen ihrer möglichen stereoisomeren Formen ebenso wie die Säure- und Alkohol-Verknüpfungen der vorstehenden Pyrethrinoidester vorliegen können.

29. Als Arzneimittel die Verbindungen der Formel I gemäß einem der Ansprüche 1 bis 17.

30. Pharmazeutische Zusammensetzungen, enthaltend als Wirkstoff zumindest eines der Arzneimittel gemäß Anspruch 29.

31. Verfahren zur Herstellung der Verbindungen der Formel I gemäß einem der Ansprüche 1 bis 9 und 13 bis 17, dadurch gekennzeichnet, daß man eine Säure der Formel (II)

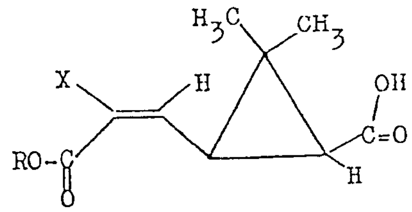

(II)

worin die Struktur der Cyclopropansäure-Verknüpfung die 1R,cis-Struktur ist und die Geometrie der Doppelbindung die E-Geometrie ist und worin X und R die vorstehend angegebene Bedeutung besitzen, oder ein funktionnelles Derivat dieser Säure mit einem Alkohol der Formel (III)

B—OH (III)

worin B die vorstehend angegebene Bedeutung besitzt, oder mit einem funktionellen Derivat dieses Alkohols umsetzt, um die entsprechende Verbindung der Formel (I) zu erhalten, die man gewünschtenfalls der Einwirkung eines selektiven Mittels zur Spaltung der Gruppe $CO_2R$ unterzieht, um die Verbindung der Formel (IV)

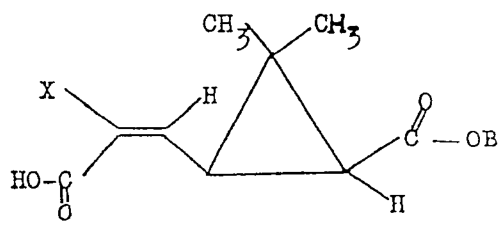

(IV)

zu erhalten, worin die Struktur der Cyclopropansäure-Verknüpfung die 1R,cis-Struktur ist und die Geometrie der Doppelbindung die E-Geometrie ist und worin B die vorstehend angegebene Bedeutung besitzt, danach diese Säure der Formel (IV) oder ein funktionelles Derivat dieser Säure der Einwirkung eines Alkohols der Formel R—OH unterzieht, worin R die vorstehend angegebene Bedeutung besitzt, um die entsprechende Verbindung der Formel (I) zu erhalten.

32. Verfahren zur Herstellung der Verbindungen der Formel $I_A$ gemäß einem der Ansprüche 10 bis 12, dadurch gekennzeichnet, daß man eine Säure der Formel ($II_A$)

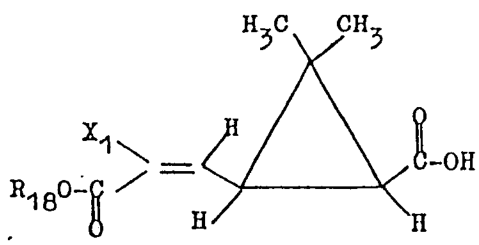

($II_A$)

worin die Struktur der Cyclopropansäure-Verknüpfung die 1R,cis-Struktur ist und die Geometrie der Doppelbindung die E-Geometrie ist und worin $X_1$ und $R_{18}$ die in Anspruch 13 angegebene Bedeutung besitzen, oder ein funktionelles Derivat dieser Säure ($II_A$) mit einem Alkohol der Formel ($III_A$)

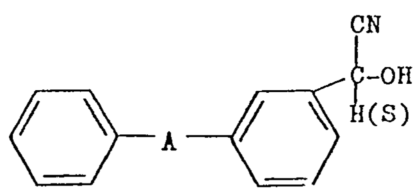

($III_A$)

worin A die Anspruch 10 angegebene Bedeutung besitzt, oder einem funktionellen Derivat des Alkohols der Formel ($III_A$) umsetzt und trennt, um die entsprechende Verbindung der Formel ($I_A$) zu erhalten.

33. Verfahren gemäß Anspruch 31 oder 32, dadurch gekennzeichnet, daß die Säuren der Formel (II) oder ($II_A$), worin R oder $R_{18}$ einen Alkylrest mit 1 bis 5 Kohlenstoffatomen bedeuten, hergestellt werden, indem man nach der Wittig-Reaktion in Gegenwart einer starken Base einen cis-Aldehyd in Form des Lactons der Formel (V)

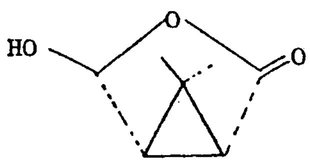

(V)

mit einem Phosphoran der Formel (VII)

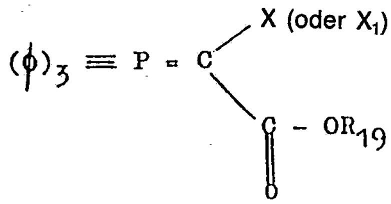

(VII)

worin $R_{19}$ einen Alkylrest mit 1 bis 5 Kohlenstoffatomen bedeutet und X und $X_1$ die vorstehend angegebenen Bedeutungen besitzen, oder mit einem Phosphonat der Formel (VIII)

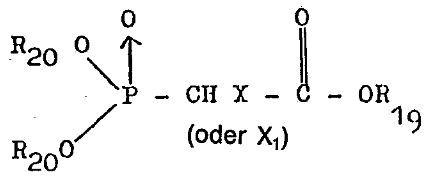

(VIII)

worin $R_{19}$, X und $X_1$ die vorstehend angegebenen Bedeutungen besitzen und $R_{20}$ einen Alkylrest mit 1 bis 6 Kohlenstoffatomen darstellt, umsetzt.

34. Als neue chemische Produkte die Säuren der Formeln II und $III_A$ gemäß den Ansprüchen 31 und 32 sowie die funktionellen Derivate dieser Säuren.

35. Als neue chemische Produkte die Säuren der Formel IV gemäß Anspruch 31 sowie die funktionellen Derivate dieser Säuren.

36. Als Zwischenprodukte für die Durchführung des Verfahrens der Ansprüche 31 und 32 die Verbindungen der Formel II, $II_A$ und IV gemäß den Ansprüchen 31 und 32.

**Ansprüche** (für den Vertragsstaat AT)

1. Verfahren zur Herstellung in sämtlichen ihrer möglichen isomeren Formen oder in Form von Gemischen der Isomeren der Verbindungen der Formel (I)

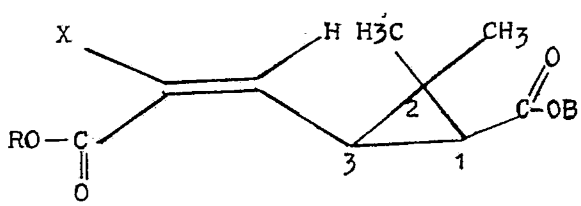

(I)

worin R entweder einen gesättigten oder ungesättigten, linearen, verzweigten oder cyclischen Alkylrest mit 1 bis 8 Kohlenstoffatomen, der gegebenenfalls durch ein oder mehrere gleiche oder verschiedene funktionelle Gruppen substituiert ist, oder eine Arylgruppe mit 6 bis 14 Kohlenstoffatomen, die gegebenenfalls durch eine oder mehrere gleiche oder verschiedene funktionelle Gruppen substituiert ist, oder einen gegebenenfalls durch eine oder mehrere gleiche oder verschiedene funktionelle Gruppen substituierten, heterocyclischen Rest bedeutet, B entweder einen gesätigten oder ungesättigten, linearen, verzweigten oder cyclischen Alkylrest mit 1 bis 18 Kohlenstoffatomen oder den Rest eines bei der Synthese der Ester der Pyrethrinoid-Reihe verwendeten Alkohols bedeutet und X ein Halogenatom ist, wobei die ethylenische Doppelbindung die Z- oder E-Geometrie besitzt, dadurch gekennzeichnet, daß man eine Säure der Formel (II)

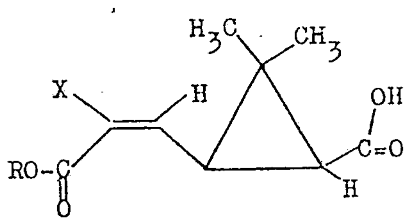

(II)

worin X und R die vorstehend angegebene Bedeutung besitzen und wobei die Säure sich in Form von Gemischen der E- und Z-Isomeren oder in Form des E- oder Z-Isomeren befindet und der substituierte Cyclopropan-Ring sich in sämtlichen seiner stereoisomeren Formen oder in Form eines Gemisches der Stereoisomeren befinden kann, oder ein funktionelles Derivat dieser Säure mit einem Alkohol der Formel (III)

B—OH (III)

wobei B die vorstehend angegebene Bedeutung besitzt, oder einem funktionellen Derivat dieses Alkohols umsetzt, um die entsprechende Verbindung der Formel (I) zu erhalten, die man gewünschtenfalls der Einwirkung eines selektiven Spaltungsmittels für die Gruppe $CO_2R$ unterzieht, um die Verbindung der Formel (IV)

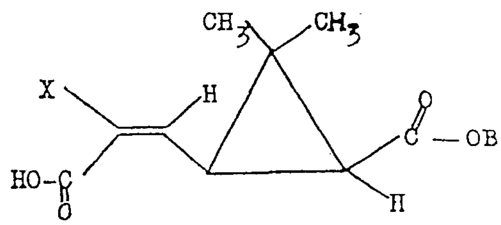

(IV)

zu erhalten, worin B die vorstehend angegebene Bedeutung besitzt, danach diese Säure der Formel (IV) oder ein funktionelles Derivat dieser Säure der Einwirkung eines Alkohols der Formel R—OH unterzieht, worin R die vorstehend angegebene Bedeutung besitzt, um die entsprechende Verbindung der Formel (I) zu erhalten.

2. Verfahren gemäß Anspruch 1, dadurch gekennzeichnet, daß man von einer Säure der Formel II mit 1R,cis-Struktur oder 1R,trans-Struktur ausgeht.

3. Verfahren gemäß Anspruch 1 oder 2, dadurch gekennzeichnet, daß man von einer Säure der Formel II ausgeht, in der die Doppelbindung die E-Geometrie besitzt.

4. Verfahren gemäß einem der Ansprüche 1 bis 3, dadurch gekennzeichnet, daß man von einer Säure der Formel II ausgeht, in der X ein Fluoratom bedeutet.

5. Verfahren gemäß einem der Ansprüche 1 bis 4, dadurch gekennzeichnet, daß man von einer Säure der Formel II oder einem Alkohol der Formel R—OH ausgeht, worin R einen linearen, verzweigten oder cyclischen Alkylrest mit 1 bis 8 Kohlenstoffatomen bedeutet.

6. Verfahren gemäß Anspruch 5, dadurch gekennzeichnet, daß R einen Ethyl- oder tert.-Butylrest bedeutet.

7. Verfahren gemäß einem der Ansprüche 1 bis 4, dadurch gekennzeichnet, daß man von einer Säure der Formel II oder einem Alkohol der Formel R—OH ausgeht, worin R einen Cyclopropyl- oder Cyclopropylmethylrest bedeutet.

8. Verfahren gemäß einem der Ansprüche 1 bis 4, dadurch gekennzeichnet, daß man von einer Säure der Formel II oder einem Alkohol der Formel R—OH ausgeht, worin R einen linearen oder verzweigten Alkylrest mit 1 bis 8 Kohlenstoffatomen, der durch ein oder mehrere Halogen-atome substituiert ist, bedeutet.

9. Verfahren gemäß einem der Ansprüche 1 bis 4, dadurch gekennzeichnet, daß man von einer Säure der Formel II und einem Alkohol der Formel R—OH ausgeht, worin R einen linearen Alkylrest mit 1 bis 8 Kohlenstoffatomen bedeutet, der durch ein oder mehrere Fluoratome substituiert ist.

10. Verfahren gemäß einem der Ansprüche 1 bis 5, dadurch gekennzeichnet, daß man von einer Säure der Formel II und einem Alkohol der Formel R—OH ausgeht, worin R einen Rest $(CH_2)_mO(CH_2)_n—CH_3$, worin m eine ganze Zahl von 1 bis 8 bedeutet und n eine ganze Zahl von 0 bis 8 bedeutet, und insbesondere den Rest $—CH_2OCH_3$ darstellt.

11. Verfahren gemäß einem der Ansprüche 1 bis 10, dadurch gekennzeichnet, daß man von einem Alkohol der Formel B—OH ausgeht, worin B bedeutet:

entweder einen Alkylrest mit 1 bis 18 Kohlenstoffatomen,

oder einen Benzylrest, der gegebenenfalls durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen substituiert ist,

oder eine Gruppe

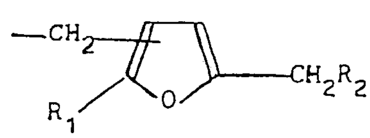

worin der Substituent $R_1$ ein Wasserstoffatom oder einen Methylrest bedeutet und der Substituent $R_2$

einen monocyclischen Arylrest oder eine Gruppe —$CH_2$—C≡CH bedeutet und insbesondere eine 5-Benzyl-3-furylmethyl-Gruppe,

oder eine Gruppe

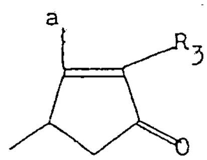

worin a ein Wasserstoffatom oder einen Methylrest bedeutet und $R_3$ einen aliphatischen, organischen Rest mit 2 bis 6 Kohlenstoffatomen und einer oder mehreren Kohlenstoff-Kohlenstoff-Unsättigungen und insbesondere den Rest —$CH_2$—CH=$CH_2$, —$CH_2$—CH=CH—$CH_3$, —$CH_2$—CH=CH—CH=$CH_2$, —$CH_2$—CH=CH—$CH_2$—$CH_3$ bedeutet,

oder eine Gruppe

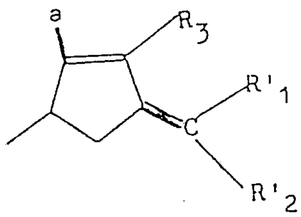

worin a ein Wasserstoffatom oder einen Methylrest bedeutet und $R_3$ die gleiche Bedeutung wie vorstehend angegeben besitzt, $R_1'$ und $R_2'$, die gleich oder verschieden sein können, ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonyl-gruppe mit 2 bis 5 Kohlenstoffatomen oder eine Cyanogruppe bedeuten,

oder eine Gruppe

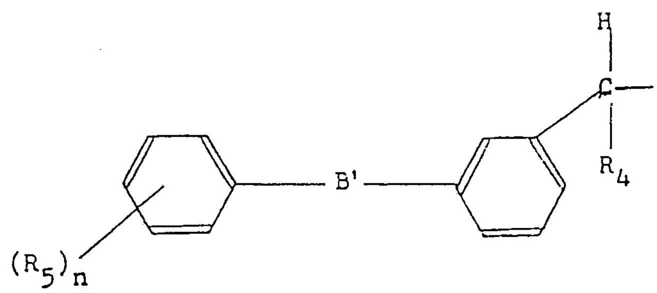

worin B' ein Sauerstoff- oder Schwefelatom, eine Gruppe

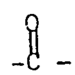

oder —$CH_2$— oder eine Sulfoxidgruppe oder eine Sulfongruppe bedeutet und $R_4$ ein Wasserstoffatom, einen Rest —C≡N, einen Methylrest, einen Rest —$CONH_2$, einen Rest —$CSNH_2$ oder einen Rest —C≡CH darstellt, $R_5$ ein Halogenatom oder einen Methylrest bedeutet und n eine Zahl entsprechend 0, 1 oder 2 darstellt, und insbesondere die 3-Phenoxybenzyl-, die α-Cyano-3-phenoxybenzyl-, die α-Ethinyl-3-phenoxybenzyl-, die 3-Benzoylbenzyl-, die 1-(3-Phenoxyphenyl)-ethyl-oder die α-Thioamido-3-phenoxybenzyl-gruppe,

oder eine Gruppe

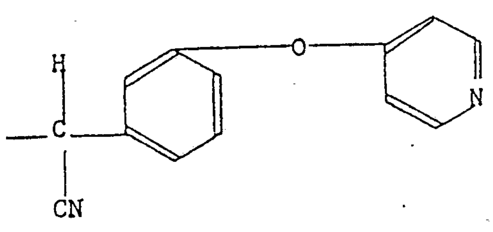

oder eine Gruppe

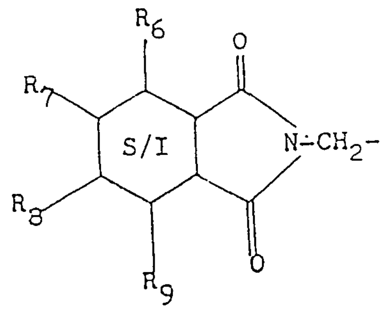

worin die Substituenten $R_6$, $R_7$, $R_8$ und $R_9$ ein Wasserstoffatom, ein Chloratom oder einen Methylrest bedeuten und worin S/I einen aromatischen Ring oder einen analogen Dihydro- oder Tetrahydro-Ring symbolisiert,

oder eine Gruppe

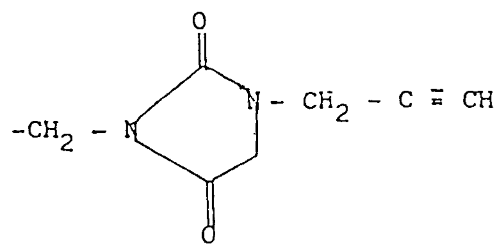

oder eine Gruppe

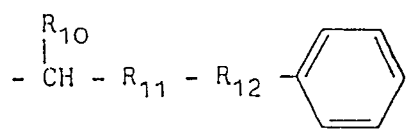

worin $R_{10}$ ein Wasserstoffatom oder einen Rest CN bedeutet, $R_{12}$ einen Rest $-CH_2-$ oder ein Sauerstoffatom bedeutet, $R_{11}$ einen Thiazolyl- oder Thiadiazolylrest darstellt, dessen Bindung mit

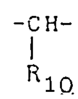

sich in irgendeiner der verfügbaren Positionen befinden kann, wobei $R_{12}$ an $R_{11}$ über ein Kohlenstoffatom gebunden ist, das zwischen dem Schwefelatom und einem Stickstoffatom liegt,

oder eine Gruppe

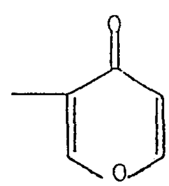

oder eine Gruppe

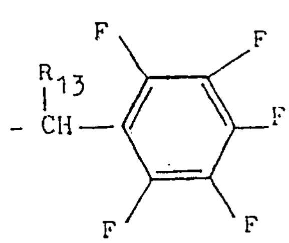

worin $R_{13}$ ein Wasserstoffatom oder einen CN-Rest bedeutet, oder eine Gruppe

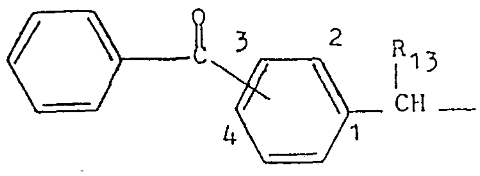

worin $R_{13}$ wie vorstehend definiert ist und der Benzoylrest sich in 3- oder 4-Stellung befindet, oder eine Gruppe

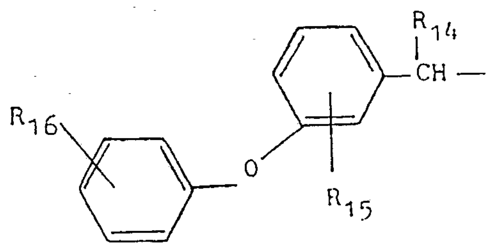

worin $R_{14}$ ein Wasserstoffatom, einen Methyl-, Ethinyl- oder Cyanorest bedeutet und $R_{15}$ und $R_{16}$, die voneinander verschieden sind, ein Wasserstoff-, Fluor- oder Bromatom bedeuten, oder eine Gruppe

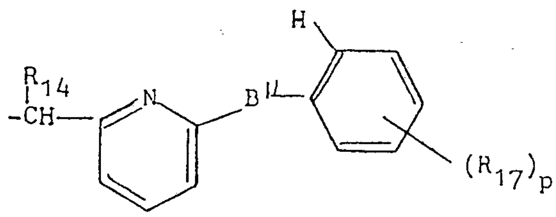

worin $R_{14}$ wie vorstehend definiert ist, ein jeder der $R_{17}$-Reste unabhängig eine Alkylgruppe mit 1 bis 4 Kohlenstoffatomen, eine Alkoxygruppe mit 1 bis 4 Kohlenstoffatomen, eine Alkylthiogruppe mit 1 bis 4 Kohlenstoffatomen, eine Alkylsulfonylgruppe mit 1 bis 4 Kohlenstoffatomen, eine Trifluormethyl-Gruppe, eine 3,4-Methylendioxy-Gruppe, Chlor, Fluor oder Brom bedeutet, p eine Zahl entsprechend 0,1 oder 2 darstellt und B" ein Sauerstoff- oder ein Schwefelatom bedeutet.

12. Verfahren gemäß Anspruch 11, dadurch gekennzeichnet, daß B ausgewählt wird unter den folgenden Resten :

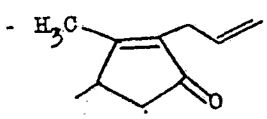 , 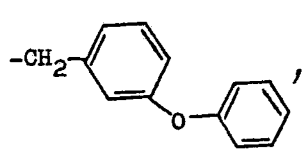 , 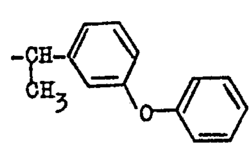

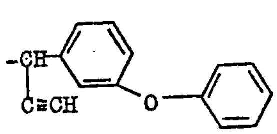 und 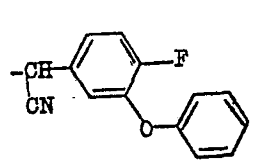

13. Verfahren gemäß Anspruch 1 zur Herstellung von Verbindungen der Formel ($I_A$)

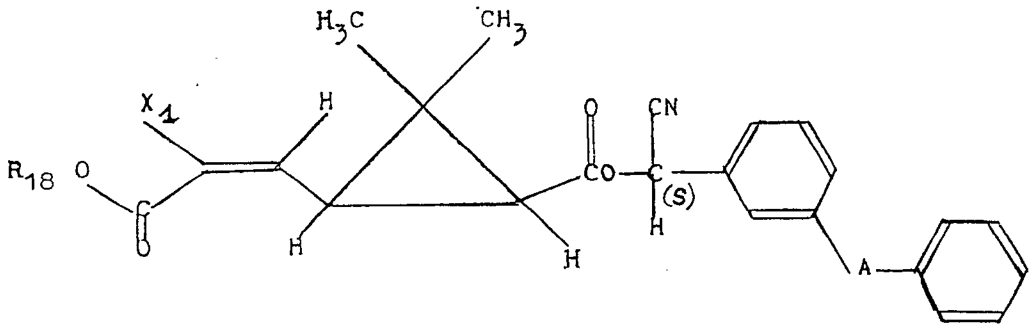

($I_A$)

worin A ein Sauerstoffatom, eine Methylengruppe, eine Carbonylgruppe, ein Schwefelatom, eine Sulfoxidgruppe oder eine Sulfongruppe bedeutet, $R_{18}$ einen linearen oder verzweigten Alkylrest mit 1 bis 8 Kohlenstoffatomen darstellt, $X_1$ ein Fluor-, Chlor- oder Bromatom bedeutet, wobei die ethylenische Doppelbindung die (E)- oder (Z)-Geometrie besitzt und der substituierte Cyclopropanring sich in jeder seinersteroisomeren Formen oder in Form eines Gemisches dieser Stereoisomeren befinden kann, dadurch gekennzeichnet, daß man eine Säure der Formel ($II_A$)

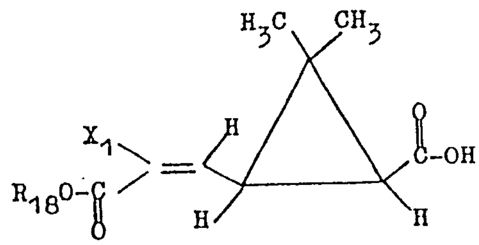

($II_A$)

worin $X_1$ und $R_{18}$ die vorstehend angegebene Bedeutung besitzen, wobei diese Säure sich in Form eines Gemisches der E + Z-Isomeren oder in Form des E- oder Z-Isomeren befinden kann, wobei der substituierte Cyclopropanring sich in jeder seinerstereoisomeren Formen oder in Form des Gemisches der Stereoisomeren befinden kann, oder ein funktionelles Derivat dieser Säure ($II_A$) mit einem Alkohol der Formel ($III_A$)

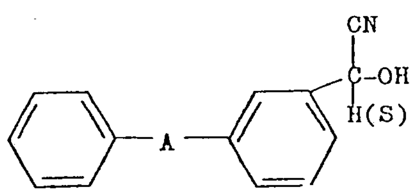

($III_A$)

worin A die vorstehend angegebene Bedeutung besitzt, oder mit einem Funktionellen Derivat des Alkohols der Formel ($III_A$) umsetzt und gegebenenfalls nach physikalischen Methoden die E- und Z-Isomeren im Hinblick auf die Doppelbindung trennt.

14. Verfahren gemäß Anspruch 13, dadurch gekennzeichnet, daß A ein Sauerstoffatom bedeutet.

15. Verfahren gemäß Anspruch 13 oder 14, dadurch gekennzeichnet, daß man das (S)-α-Cyano-3-phenoxybenzyl-(1R,cis)-2,2-dimethyl-3(E)-[2-fluor-2-(ethoxycarbonyl)-ethenyl]-cyclopropan-1-carboxylat herstellt.

16. Verwendung der Verbindungen der Formel I gemäß Anspruch 1 zur Bekämpfung von Parasiten der Pflanzen, Parasiten von Örtlichkeiten und Parasiten warmblütiger Tiere.

17. Verwendung der Verbindungen der Formel $I_A$ gemäß Anspruch 13 zur Bekämpfung von Parasiten der Pflanzen, Parasiten von Örtlichkeiten und Parasiten warmblütiger Tiere.

18. Zusammensetzungen für die Bekämpfung von Parasiten der Pflanzen, Parasiten von Örtlichkeiten und Parasiten warmblütiger tiere, dadurch gekennzeichnet, daß sie als Wirkstoff zumindest eine Verbindung gemäß Anspruch 1 enthalten.

19. Insektizide Zusammensetzungen, dadurch gekennzeichnet, daß sie als Wirkstoff zumindest eine Verbindung gemäß Anspruch 1 enthalten.

20. Akarizide Zusammensetzungen, dadurch gekennzeichnet, daß sie als Wirkstoff zumindest eine Verbindung gemäß Anspruch 1 enthalten.

21. Zusammensetzungen für die tierische Ernährung, dadurch gekennzeichnet, daß sie als Wirkstoff zumindest eine der Verbindungen gemäß Anspruch 1 zusammen mit einem tierischen Nahrungsmittel enthalten.

22. Mit insektizider, akarizider oder nematizider Aktivität ausgestattete Zusammensetzungen, dadurch gekennzeichnet, daß sie als Wirkstoff einesteils zumindest eine der Verbindungen der

allgemeinen Formel (I) und anderenteils zumindest einen der Pyrethrinoidester enthalten, ausgewählt unter den Estern der Allethrolone, des 3,4,5,6-Tetrahydrophthalimido-methylalkohols, des 5-Benzyl-3-furyl-methylalkohols, des 3-Phenoxybenzylalkohols und der α-Cyano-3-phenoxybenzylalkohole der Chrysanthemumsäuren, unter den Estern des 5-Benzyl-3-furyl-methylalkohols der 2,2-Dimethyl-3-(2-oxo-3-tetrahydrothiophenyliden-methyl)-cyclopropan-1-carbonsäuren, unter den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropylessigsäuren, unter den Estern der Allethrolone, des 3,4,5,6-Tetrahydrophthalimido-methylalkohols, des 5-Benzyl-3-furyl-methylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, worin « halo » ein Fluor-, Chlor- oder Bromatom bedeutet, wobei die Verbindungen I in sämtlichen ihrer möglichen stereoisomeren Formen ebenso wie die Säure- und Alkohol-Verknüpfungskomponenten der vorstehenden Pyrethrinoidester vorliegen können.

23. Zusammensetzungen gemäß einem der Ansprüche 18 bis 21, dadurch gekennzeichnet, daß der Wirkstoff der Formel ($I_A$), wie in Anspruch 13 definiert, entspricht.